# EUROPEAN PATENT APPLICATION

(11) **EP 2 105 740 A1**
(43) Date of publication of application: **30.09.2009**
(21) Application number: 08075242.1
(22) Date of filing: 28.03.2008
(51) Int. Cl.: G01N 33/574, G01N 33/68, G01N 33/50, A01K 67/027

(54) **Biomarkers for monitoring or predicting the treatment of cancer**

(71) Applicant: Fraunhofer-Gesellschaft zur Förderung der Angewandten Forschung e.V., 80686 München (DE)
(72) Inventor: Borlak, Jürgen, Prof. Dr., 31275 Lehrte/OT Immensen (DE); Chatterji, Bijon-Gopal, 30655 Hannover (DE)
(74) Representative: Baumbach, Friedrich

(57) **Abstract**

The invention is directed to biomarkers for determining the c-myc activity in a subject, and the use thereof for predicting and monitoring therapeutic intervention in cancer patients. According to the invention at least one biomarker selected from the group consisting of APOE, APOC3, A1AT6, A2MG, PROP, TTHY, A1AG8, APOA1, APOH, GPX3, MUP8, RETBP, SAMP, VTDB, S6A11, EGFR, and particular peptide sequences derived thereof, is used in the diagnosis, prognosis and/or treatment monitoring of cancer, in particular of lung cancer or the level of at least one of said biomarkers is measured in a body fluid sample, in particular in a serum sample, of a patient suffering from or being susceptible to cancer.

More particular, within the context of said biomarkers, the invention concerns a composition for qualifying the c-myc activity in a patient suffering or being susceptible to cancer or for classifying a patient suffering from or being susceptible to lung cancer, in particular by an *in vitro* body fluid analysis, and a procedure to screen for and to identify drugs against cancer associated with an increased c-myc activity is provided, wherein in a body fluid sample of a transgenic cancer mouse being treated with a compound to be tested, in particular of a mouse whose genome comprises a non natural c-myc sequence, at least one of said biomarkers is determined.

## Description

The invention is directed to biomarkers for determining the c-myc activity in a subject, and the use thereof for predicting and monitoring therapeutic intervention in cancer patients. Areas of application are the life sciences: biology, biochemistry, biotechnology, medicine and medical technology.

Despite the fact that many cancers are considered to be sporadic diseases, changes in c-myc activity are frequently associated with a variety of human malignancies, e.g. cancer of the lung, breast, liver, colon, as well as ovarian cancer and lymphomas.

Lung cancer, for instance, remains the leading cause of cancer death worldwide. In 2007, approximately 160.000 people died from lung cancer in the United States alone (American Cancer Society, 2007). In Germany, lung cancer is the fourth-most frequent disease. More than 40,000 humans die at lung cancer annually (Federal Statistical Office, 2007) country wide and therefore it is one of the most frequent cancer disease at all. Smoking is considered the primary cause of lung cancer (relative risk > 30-fold) and accounts for > 80% of all diagnosed cases [2]. Among other things, further risk factors are an exposition to radioactive compounds and the inhalation of heavy metals, asbestos and exhaust gases. In general, lung tumors are classified as small cell (SCLC) or non-small cell lung carcinomas (NSCLC). NSCLC are further divided into adenocarcinoma, large and squamous cell carcinoma. Subclasses of adenocarcinomas may be derived from Clara and alveolar epithelium [3]. Primarily, lung cancer develops from the respiratory epithelium, whereby alveolar epithelial adenocarcinomas of humans are rather rare. Newer data point to a significant rise of alveolar and Clara cell tumors. Note, alveolar epithelial carcinomas may account for up to one third of all adenocarcinomas [4]. The chances of survival of a patient with a lung adenocarcinoma are not even with 10% within 5 years.
The molecular mechanisms in the emergence of lung tumors are unclear and arise sporadically. Only 12 to 15 % of the cancer patients can be healed by present methods. The method spectrum covers the operational removal of the tumor tissue as well as radiating and chemotherapy. In most cases, tumor metastasis is progressed so far, that healing chances are small. Indeed, after operational interference at stages of disease, chemotherapy leads to a longer survival rate, but the present medicamentous and radiological treatments are not sufficient to destroy the cancer tissues lastingly and to finally prevent the patients from death.
Transgenic animal models deliver crucial beginnings for an improved understanding of the molecular causes. Due to their genetic similarity to humans of more than 99%, mice are routinely used for biomedical research. Genetically modified mice enable a better understanding for the investigation and/or the relevance of overexpressed oncogenes or for a switching off of tumor supressor genes.
Since each cell type must be able to react to extracellular signals, cells use defined signal transduction ways to achieve a cell answer, for example an altered gene expression, mostly by activation of receptors and phosphorylation cascades. Thereby, mitogen activated protein kinase (MAPK)- signal transduction belongs to the best examined signal transduction ways. Here, the transcription factor c-myc is of outmost importance for the regulation of the cell cycle and apoptosis.
C-myc is modulated in nearly all epithelial tumors. Therefore the identification and description of disease regulated serum proteins for both, the diagnostic and therapeutic monitoring, are of great importance. Especially the use of c-myc transgenic mice for the identification of those new diagnostic serum proteins appears promising.
In transgenic mice, which overexpress c-myc, morphological changes can be observed at early stages of tumorigenesis and typical characteristics of the bronchiolo-alveolar carcinoma are shown in the entire lung after 8 to 10 months. By means of "disease proteomics" regulated proteins can now be searched and characterized better, which leads to new information about the molecular pathogenesis of lung tumors.
In this context, diagnostic and prognostic disease biomarkers are searched. Their quantification is substantial to predict and to detect biological processes.

Taken collectively, a world-wide search for diagnostic biomarkers is on the rise for the early detection and monitoring of cancer. The benefits of biomarkers for the early detection of lung adenocarcinomas was already discussed in the literature in detail. Thus, proteomic research is useful to identify novel biomarkers, for instance, by comparative studies of the serum proteome of tumor bearing and healthy animals, in order to identify tumor-specific proteins and to evaluate both, their differential expression and their transportation in blood for diagnostic purposes.
The tumor proteome of c-myc induced adenocarcinomas of the lung is unknown. Integrated methods of proteomic research enable detection and characterization of specifically regulated proteins.

Specifically, the c-myc proto-oncogene encodes a 49 kDa nuclear phosphoprotein which is a basic helix-loop-helix/leucine zipper-type (bHLH/LZ) transcription factor and a major modulator of cell proliferation [7]. The c-myc protein forms a heterodimer with max, an 18 kDa bHLH/LZ protein. The c-myc/max complex recognizes a cognate DNA sequence (CACGTG) known as the E-box motif [8]. This motif is located in the promoter region of genes targeted by c-myc. Genome wide screening by DNA microarray, SAGE and chromatin immunoprecipitation (ChIP) identified more than 1.000 c-myc target genes involved in various metabolic processes, in signal transduction, DNA synthesis and repair, apoptosis, cell adhesion, cytoskeleton dynamics and ion channels [9].

Indeed, expression of c-myc is increased in many human malignant tumors resulting from an amplification of the reference allele or the mutated c-myc gene [10]. Noteworthy, in the United States more than 70.000 cancer deaths per year are estimated to be related to c-myc abnormalities [11].

Since c-myc plays a key role in malignant tumor diseases, inhibition of c-myc expression may be sufficient to stop tumor growth permanently and to induce regression of tumors. Furthermore, tumor cells overexpressing c-myc are sensitized to treatment with DNA-damaging drugs. In fact, molecules that counter an increased activity of c-myc, termed as "c-myc activity modulators" herein, in particular antisense oligonucleotides to inhibit c-myc, inhibitors of c-myc/max dimerization, and chemotherapeutical drugs have shown promising results. Moreover, since expression of the c-myc gene is related to chemotherapeutical response, it might be a useful prognostic factor in cancer patients (Iba et al. Cancer Sci. 2004 May;95(5):418-23).

However, as of today, disease regulated proteins in body fluids of patients suffering from c-myc-induced cancer, such as SCLC and NSCLC may be, are unknown.

Thus, there is a need for novel prognostic and predictive biomarkers and methods for easily identifying the indication "c-myc hyperactivity" or "c-myc overexpression", respectively, in a subject suffering from or being susceptile to cancer, for predicting and monitoring the response of a subject to a treatment with c-myc activity modulators, thereby enabeling an individualized cancer treatment of the subject for enhancing its chances of survival.

Therefore, the aim of the present invention is to provide biomarkers, compositions and a kit, as well as a method for a fast, easy and efficient qualification or quantification of the c-myc activity status of a subject suffering from or being susceptible to cancer, in particular for predicting and monitoring the response of a cancer patient to the treatment with a c-myc activity modulator.

Finally, the implementation of the embodiments and actions as described in the claims provide appropriate means to fulfill these demands in a satisfying manner.

The basic finding, relevant to this invention, is the unexpected regulation of certain proteins in c-myc induced lung tumors.
By using two pH ranges (4-7 and 3-10) a mapping for the characterization of the serum proteome of the SP-C/c-myc mouse model is accomplished, which covers 46 identified proteins in total, 3 of which are unknown, yet. Moreover, 13 significantly disease regulated proteins are discovered, e.g. orosomucoid-8, alpha-2-macroglobulin, glutathione peroxidase 3, properdin, plasma retinol binding protein, transthyretin as well as numerous apolipoproteins, like apolipoprotein H, for which regularization in connection with lung cancer has not been reported, yet.
Furthermore, exclusively in tumor bearing mice a component of the serum amyloid P protein has been identified.
These investigations provide new information about the mouse serum proteome and the role of c-myc in lung tumorigenesis as well. Therefore, serum proteomics leads to a routinely use of proteomic methods in clinical laboratories for the prognostic and diagnostic classification of several stages of the disease as well as for monitoring the evaluation of therapy processes.

### Detailed description

The invention is based on the surprising finding that biomarkers selected from a first group consisting of Alpha-1-antitrypsin 1-1 (A1AT1), Alpha-1-antitrypsin 1-6 (A1AT6), Alpha-2-macroglobulin (A2MG), Properdin (PROP), Transthyretin (TTHY), Orosomucoid-8 (A1AG8), Apolipoprotein A-I (APOA1), Apolipoprotein C-III (APOC3), Apolipoprotein H (APOH), Glutathione peroxidase 3 (GPX3), Plasma retinol-binding protein (RETBP), Serum amyloid P-component (SAMP), Vitamin D binding protein (VTDB), Sodium- and chloride-dependent GABA transporter 4 (S6A11), epidermal growth factor receptor (EGFR) or from a second group consisting of Apolipoprotein E (APOE) and fragments thereof are regulated by c-myc overexpression in subjects suffering from or being susceptile to cancer.

The biomarkers according to the invention concern gene products of mammalia, preferably gene products of the genome of *mus musculus* or *homo sapiens,* in particular the respective gene products of *homo sapiens* are preferred, or, respectively, sequence fragments of said gene products as described herein.

Within the context of the invention, the term "subject", and "patient", respectively, is directed to a mammal, in particular to a mouse or a human being suffering from or being susceptible to cancer, more particular to a human cancer patient or a transgenic cancer mouse, such as a SCLC or NSCLC patient or a c-myc-transgenic mouse may be.

In one aspect, the invention is directed to the use of at least one biomarker selected from the group consisting of APOE, APOC3, A1AT6, A2MG, PROP, TTHY, A1AG8, APOA1, APOH, GPX3, MUP8, RETBP, SAMP, VTDB, S6A11, EGFR in the diagnosis of cancer and/or the prognosis of cancer and/or the treatment monitoring of cancer, in particular of lung cancer such as lung adenocarcinoma(s) may be. Preferentially, a combination of APOE and at least one further biomarker selected from the group of APOC3, A1AT6, A2MG, PROP, TTHY, A1AG8, APOA1, APOH, GPX3, MUP8, RETBP, SAMP, VTDB, S6A11, EGFR is used as the at least one biomarker. Preferably, an appropriate amount of the at least one biomarker is used, in particular an amount for manufacturing a reference, more particular for manufacturing a reference comprising a reference level of said at least one biomarker, such as the level of said at least one biomarker in a sample of a normal healthy individual or the level of a said at least one biomarker in a sample of a patient suffering from cancer may be.

In particular, at least one biomarker selected from the group consisting of APOE, APOC3, A1AT6, A2MG, PROP, TTHY, A1AG8, APOA1, APOH, GPX3, MUP8, RETBP, SAMP, VTDB, S6A11, EGFR is used for monitoring the therapeutic treatment of a patient, in particular a human patient, suffering from lung cancer, in particular for monitoring the treatment of said patient with irinotecan, paclitaxel and/or 5-fluorouracil.

More preferably, the at least one biomarker used is selected from the group consisting of APOE, APOC3, A1AG8, APOA1, APOH, GPX3, RETBP, SAMP, in particular for the diagnosis, prognosis and/or treatment monitoring of BAC.

Also, it may be preferred to use at least one biomarker selected from the group consisting of MUP8, VTDB, S6A11, EGFR, in particular for the diagnosis, prognosis and/or treatment monitoring of AAH.

Preferentially, a combination of at least one biomarker selected from the group consisting of APOE, APOC3, A1AG8, APOA1, APOH, GPX3, RETBP, SAMP and at least one biomarker selected from the group consisiting of MUP8, VTDB, S6A11, EGFR is used.

Another aspect of the invention is directed to a method for diagnosing cancer and/or prognosing cancer and/or staging cancer and/or monitoring the treatment of cancer, in particular lung cancer, such as lung adenocarcinoma(s) may be, comprising the steps of
(a) measuring the level of at least one biomarker selected from the group consisting of APOE, APOC3, A1AT6, A2MG, PROP, TTHY, A1AG8, APOA1, APOH, GPX3, MUP8, RETBP, SAMP, VTDB, S6A11, EGFR in a body fluid sample, in particular in a serum sample, of a patient suffering from or being susceptible to cancer, and
(b) comparing the level of said at least one biomarker in said sample to a reference level of said at least one biomarker, in particular by the use according to one of the claims 1-8.

Preferably, the method for diagnosing, prognosing and/or staging cancer and/or monitoring the treatment of cancer, is implemented for monitoring the therapeutic treatment of a patient suffering from lung cancer, in particular the treatment with irinotecan, paclitaxel and/or 5-fluorouracil.

In one preferred embodiment, at least one biomarker is selected from the group consisting of APOE, APOC3, A1AG8, APOA1, APOH, GPX3, RETBP, SAMP, in particular for the diagnosis, prognosis, staging and/or treatment monitoring of BAC.

In another preferred embodiment at least one biomarker is selected from the group consisting of MUP8, VTDB, S6A11, EGFR, in particular for the diagnosis, prognosis, staging and/or treatment monitoring of AAH.

In particular, the method is preferably implemented to distinguish between different subtypes of lung cancer, such as (but not limited to) lung adenocarcinomas as defined by AAH or BAC, wherein at least one biomarker selected from the group consiting of APOE, APOC3, A1AG8, APOA1, APOH, GPX3, RETBP, SAMP and at least one biomarker selected from the group consisiting of MUP8, VTDB, S6A11, EGFR are measured, and, more particularly, wherein a significantly altered level of APOE, APOC3, A1AG8, APOA1, APOH, GPX3, RETBP and/or SAMP in comparison with the respective level of a normal individual is indicative of BAC and wherein a significantly altered level of MUP8, VTDB, S6A11 and/or EGFR in comparison with the respective level of a normal individual is indicative of AAH.

In another aspect, the invention further concerns a composition for qualifying the c-myc activity in a subject suffering from or being susceptible to cancer, in particular by an *in vitro* body fluid analysis, wherein the composition comprises an effective amount of at least one biomarker selected from the first group of said biomarkers or an effective amount of at least one biomarker selected from the second group of said biomarkers.

In one embodiment of the invention, the biomarker is preferably selected from a first group consisting of A1AG8, APOA1, APOC3, APOH, GPX3, RETBP, SAMP or from a second group consisting of APOE and fragments thereof

In another preferred embodiment, a biomarker is provided for qualifying the c-myc activity in a patient suffering or being susceptible to cancer or for classifying a patient suffering from or being susceptible to lung cancer, wherein the biomarker is selected from a first group consisting of sequence fragments of the first group of said biomarkers or is selected from a second group consisting of sequence fragments of the second group of said biomarkers, and wherein the sequence fragments are 6-24 amino acid residues in length and are preferably synthetic peptides.

Preferably, the biomarker is selected from a first group consisting of
LAQIHFPR,
TLMSPLGITR,
RLAQIHFPR,
MQHLEQTLSK,
ELISKFLLNR,
IFNNGADLSGITEENAPLK,
NHYQAEVFSVNFAESEEAK,
DQSPASHEIATNLGDFAISLYR,
KPFDPENTEEAEFHVDESTTVK,
FDHPFLFIIFEEHTQSPLFVGK,

APFALQVNTLPLNFDK,

TCDHPAPR,
QRLCTPLLPK,
HGGPFCAGDATR,
MSINCEGTPGQQSR,
LRMSINCEGTPGQQSR,
HGGPFCAGDATRNQMCNK,
CGGHCPGEAQQSQACDTQK,
SCSAPAPSHQPPGKPCSGPAYEHK,

FVEGVYR,
TSEGSWEPFASGK,
TAESGELHGLTTDEK,
TLGISPFHEFADVVFTANDSGHR,
HYTIAALLSPYSYSTTAVVSNPQN,

YEGGVETFAHLIVLR,
LQELQGR,
EDVELYR,
ARPALEDLR,
LSPVAEEFR,
QKLQELQGR,
WKEDVELYR,
VQPYLDEFQK,
TQLAPHSEQMR,
LSPVAEEFRDR,
SNPTLNEYHTR,
VAPLGAELQESAR,
QEMNKDLEEVK,
VKDFANVYVDAVK,
LQELQGRLSPVAEEFR,

TVQDALSSVOESDIAVVAR,

IHFYCK,
ATVLYQGMR,
ITCPPPPVPK,
WSPDIPACAR,
DGTIEIPSCFK,
CSYTVEAHCR,
TGTWSFLPTCR,
VCPFAGILENGIVR,
IQEQFKNGMMHGDK,
FTCPLTGMWPINTLR,
ICPKPDDLPFATVVPLK,
TSYDPGEQIVYSCKPGYVSR,
CPFPPRPENGYVNYPAKPVLLYK,

YVRPGGGFVPNFQLFEK,

DPNGLSPETR,
YWGVASFLQR,
QRQEELCLER,
LQNLDGTCADSYSFVFSR,
KDPEGLFLQDNIIAEFSVDEK,

APPSIVLGQEQDNYGGGFQR,
or from a second group consisting of
EVQAAQAR,
FWDYLR,
DRLEEVR,
EHMEEVR,
LGPLVEQGR,
LEEVGNQAR,
QWANLMEK,
DRAQAFGDR,
LQAEIFQAR,
MEEQTQQIR,
GRLEEVGNQAR,
TANLGAGAAQPLR,
SKMEEQTQQIR,
GWFEPIVEDMHR,
ELEEQLGPVAEETR,
NEVHTMLGQSTEEIR.

More preferably, the biomarker is selected from a first group consisting of
YEGGVETFAHLIVLR,

LQELQGR,
EDVELYR,
ARPALEDLR,
LSPVAEEFR,
QKLQELQGR,
WKEDVELYR,
VQPYLDEFQK,
TQLAPHSEQMR,
LSPVAEEFRDR,
SNPTLNEYHTR,
VAPLGAELQESAR,
QEMNKDLEEVK,
VKDFANVYVDAVK,
LQELQGRLSPVAEEFR,

TVQDALSSVQESDIAVVAR,

IHFYCK,
ATVLYQGMR,
ITCPPPPVPK,
WSPDIPACAR,
DGTIEIPSCFK,
CSYTVEAHCR,
TGTWSFLPTCR,
VCPFAGILENGIVR,
IQEQFKNGMMHGDK,
FTCPLTGMWPINTLR,
ICPKPDDLPFATVVPLK,
TSYDPGEQIVYSCKPGYVSR,
CPFPPRPENGYVNYPAKPVLLYK,

YVRPGGGFVPNFQLFEK,

IEDNGNFR,
EKIEDNGNFR,
ENIIDLSNANR,
FAQLCEEHGILR,
DGETFQLMGLYGR,
INGEWHTIILASDKR,
TDYDNFLMAHLINEK,
LFLEQIHVLENSLVLK,
AGEYSVTYDGFNTFTIPK,

DPNGLSPETR,
YWGVASFLQR,
QRQEELCLER,
LQNLDGTCADSYSFVFSR,

KDPEGLFLQDNIIAEFSVDEK,

APPSIVLGQEQDNYGGGFQR,
or from a second group consisting of
EVQAAQAR,
FWDYLR,
DRLEEVR,
EHMEEVR,
LGPLVEQGR,
LEEVGNQAR,
QWANLMEK,
DRAQAFGDR,
LQAEIFQAR,
MEEQTQQIR,
GRLEEVGNQAR,
TANLGAGAAQPLR,
SKMEEQTQQIR,
GWFEPIVEDMHR,
ELEEQLGPVAEETR,
NEVHTMLGQSTEEIR.

In particular, it is preferred, if the composition according to the invention comprises an effective amount of at least one biomarker selected from the first group of said biomarkers and an effective amount of at least one biomarker selected from the second group of said biomarkers, wherein the combination
(a) biomarker selected from the group consisting of A1AT1, A1AT6, A2MG, PROP, TTHY, A1AG8, APOA1, APOC3, APOH, GPX3, RETBP, SAMP or selected from the group consisting of the sequence fragments thereof, as described herein, and biomarker selected from the group consisting of APOE or selected from the group of the sequence fragments thereof, as described herein, or the combination
(b) biomarker selected from the group consisting of A1AG8, APOA1, APOC3, APOH, GPX3, RETBP, SAMP, AFP, ApoE, ApoM or selected from the group consisting of the sequence fragments thereof, as described herein, and biomarker selected from the group consisting of APOE or selected from the group consisting of the sequence fragments thereof, as described herein, is particularly preferred.

In another preferred embodiment the composition further comprises an effective amount of a biomarker selected from the group of c-myc, thus allowing an easy calibration of the system.

In yet another preferred embodiment, the composition according to the invention further comprises an effective amount of a protease, in particular of trypsin, thus enabling a further enhancement of the system sensitivity.

The composition according to the invention, in particular the protease digest thereof, may be preferably used for producing a vaccine for the immunization of an animal in order to produce polyclonal antibodies specific for the at least one biomarker.

Another aspect of the invention concerns the use of the composition according to the invention for the production of a diagnostic agent, in particular of a diagnostic standard for *in vitro* body fluid analyses.

The term "body fluid" according to the invention is directed to any body fluid of a subject, in particular to blood, plasma, serum or urine, whereas serum is the preferred body fluid within the context of the invention. The term "diagnostic agent" as used herein relates to any solution, suspension or solid formulation, containing said composition in an acceptable amount for diagnostic purposes.

In particular, the composition is used for the production of a diagnostic agent for qualifying the c-myc activity in a subject suffering from or being susceptible to cancer, preferably cancer of the lung, breast, liver, colon, as well as ovarian cancer and lymphomas, in particular in a subject suffering from or being susceptible to SCLC or NSCLC.

In a further preferred embodiment, the composition according to the invention is used for the production of a diagnostic agent for predicting or monitoring the response of a cancer patient to a method of treating cancer comprising administering a c-myc activity modulator to the patient.

In yet another aspect, the invention provides a kit for qualifying the c-myc activity in a subject suffering from or being susceptible to cancer, in particular for predicting or monitoring the response of a cancer patient to a method of treating cancer comprising administering an c-myc activity modulator, wherein the kit comprises at least one standard (1) indicative of the body fluid level of a biomarker selected from the first group of said biomarkers in normal individuals or individuals having cancer associated with increased c-myc activity and/or at least one standard (2) indicative of the body fluid level of a biomarker selected from the second group of said biomarkers in normal individuals or individuals having cancer associated with increased c-myc activity, and instructions for the use of the kit.

In a preferred embodiment of the kit, the standard (1) comprises an indicative amount of at least one biomarker selected from the first group of said biomarkers and/or the at least one standard (2) comprises an indicative amount of at least one biomarker selected from the second group of said biomarkers.

In another preferred embodiment, the kit comprises a mixture of the at least one standard (1) and the at least one standard (2), in particular a composition according to the invention comprising an effective amount of at least one biomarker selected from the first group of said biomarkers and an effective amount of at least one biomarker selected from the second group of said biomarkers, wherein the set of biomarkers according to combination (a) or combination (b), as described herein, is particularly preferred.

In yet another preferred embodiment, the kit according to the invention further comprises a lysis buffer, wherein the lysis buffer comprises (a) at least one buffer component, (b) at least one chaotrope, (c) at least one detergens, (d) at least one reducing agent (e) at least one carrier ampholyte, and (f) at least one ribonuclease, Preferably, the lysis buffer is an aqueous solution of (a) at least one buffer compound selected from the group consisting of Tris and HEPES, (b) at least one chaotrope selected from the group consisting of urea and thiourea, (c) at least one detergens selected from the group consisting of CHAPS and SDS, (d) at least one reducing agent selected from the group consisting of DTT and TCEP, (e) at least one carrier ampholyte selected from the group consisting of biolyte 5-7 and biolyte 3-10, and (f) at least one ribonuclease selected from the group consisting of endonuclease and exonuclease, wherein an aqueous solution of (a) Tris; (b) urea and thiourea, (c) CHAPS, (d) DTT, (e) biolyte 3-10, and (f) endonuclease, is particularly preferred.

In one preferred embodiment, the kit according to the invention further comprises at least one antibody specific for a biomarker selected from the first group of said biomarkers and/or at least one antibody specific for a biomarker selected from the second group of said biomarkers, and reagents effective to detect said biomarker(s) in a serum sample, such as buffers for dissolving or equilibrating the standard (1) and/or the standard (2), or an enzyme substrate for imaging enzyme labels may be.
In particular, a kit is preferred, comprising at least one antibody specific for a biomarker selected from the group consisting of A1AT1, A1AT6, A2MG, PROP, TTHY, A1AG8, APOA1, APOC3, APOH, GPX3, RETBP, SAMP or selected from the group consisting of the sequence fragments thereof, as described herein, and/or at least one antibody specific for a biomarker selected from the group consisting of APOE or selected from the group consisting of the sequence fragments thereof, as described herein, or a peptide fragment thereof, as described herein.

More particular, it is preferred, if the at least one antibody is polyclonal, thus allowing a further enhancement of the system sensitivity.
Advantageously, the kit further comprises at least one labelled secondary antibody specific for the at least one antibody, thus allowing a fast screening of the binding of the at least one antibody to the at least one biomarker, in particular if the at least one biomarker or the digest thereof is immobilized to a solid phase support, such as nitrocellulose may be.

In a further aspect, the invention provides a method of qualifying the c-myc activity in a subject, comprising determining in a body fluid sample of a subject suffering from or being susceptible to cancer at least one biomarker selected from the first group of said biomarkers and/or at least one biomarker selected from the second group of said biomarkers, wherein the body fluid level of the at least one biomarker of said first group being significantly higher and/or the body fluid level of the at least one biomarker of said second group being significantly lower than the level of said biomarker(s) in the body fluid of subjects without cancer, in particular without cancer associated with increased activity of c-myc, is indicative of induced c-myc activity in the subject.

In particular, it is preferred, if the method comprises determining at least one biomarker selected from the first group of said biomarkers and at least one biomarker selected from the second group of said biomarkers, wherein the body fluid level of the at least one biomarker of said first group being significantly higher and the body fluid level of the at least one biomarker of said second group being significantly lower than the level of said biomarkers in the body fluid of subjects without cancer, in particular without cancer associated with increased activity of c-myc, is indicative of induced c-myc activity in the subject, preferably if a combination of a biomarker selected from the group consisting of A1AT1, A1AT6, A2MG, PROP, TTHY, A1AG8, APOA1, APOC3, APOH, GPX3, RETBP, SAMP or selected from the group consisting of the sequence fragments thereof, as described herein, and a biomarker selected from the group consisting of APOE or selected from the group of the sequence fragments thereof, as described herein, is determined.

Preferably, the method according to the invention is carried out for predicting the response of a cancer patient to a method of treating cancer comprising administering an c-myc activity modulator, wherein the body fluid level of the at least one biomarker of said first group being significantly higher and/or the body fluid level of the at least one biomarker of said second group being significantly lower than the level of said biomarker(s) in the body fluid of subjects without cancer, in particular without cancer associated with increased activity of c-myc, is indicative that the subject will respond therapeutically to a method of treating cancer comprising administering a c-myc activity modulator.

In one embodiment, the method is implemented for monitoring the therapeutically response of a cancer patient to a method of treating cancer comprising administering a c-myc activity , wherein the body fluid level of the at least one biomarker of said first group before and after the treatment and/or the body fluid level of the at least one biomarker of said second group before and after the treatment is determined, and a significant decrease of said body fluid level(s) of the at least one biomarker of said first group and/or a significant increase of said body fluid level(s) of the at least one biomarker of said second group after the treatment is indicative that the cancer patient therapeutically responds to the administration of the c-myc activity modulator.

In a preferred embodiment, the method is implemented by performing an immunoassay, such as an enzyme immunoassay (EIA), a radio immunoassay (RIA) or a fluorescence immunoassay (FIA) may be, in particular by using the kit according to the invention and/or by performing a western blot. Preferably, at least one antibody specific for a biomarker selected from the group consisting of A1AT1, A1AT6, A2MG, PROP, TTHY, A1AG8, APOA1, APOC3, APOH, GPX3, RETBP, SAMP or selected from the group consisting of the sequence fragments thereof, as described herein, and/or at least one antibody specific for a biomarker selected from the group consisting of APOE or selected from the group consisting of the sequence fragments thereof, as described herein, is used for the immunoassay and/or reagents effective to detect said biomarker(s) in a serum sample, such as a blocking buffer for reducing secondary antibodies unspecific antibody binding or an enzyme substrate for imaging enzyme labelled antibodies may be, is used for the immunoassay.

Within the context of immunoassys, the method preferably comprises the steps of
- isolating a serum sample from a blood sample of a subject suffering from or being susceptible to cancer,
- adding lysis buffer to the serum sample;
- separating the proteins of the lysed serum sample by 2-D gel electrophoresis;
- excising from the gel at least one sample containing a protein of interest;
- adding digesting buffer to the at least one excised sample, and
- determining the amount of the at least one protein of interest by analyzing the at least one digest mixture by an immunoassay, in particular by a Western blot.

In another preferred embodiment, the method is implemented by performing a peptide mass fingerprinting, in particular by using the kit described herein.

Within the context of peptide mass fingerprinting, the method preferably comprises the steps of
- isolating a serum sample from a blood sample of a subject suffering from or being susceptible to cancer,
- adding lysis buffer to the serum sample;
- separating the proteins of the lysed serum sample by 2-D gel electrophoresis;
- excising from the gel at least one sample containing a protein of interest;
- adding digesting buffer to the at least one excised sample, and
- determining the amount of the at least one protein of interest by analyzing the at least one digest mixture by mass spectrometry.

In one embodiment of the method, the subject is a human patient or a non-human transgenic animal, in particular suffering from or being susceptible to cancer, more particular suffering from or being susceptible to cancer of the lung, breast, liver, colon, as well as ovarian cancer and lymphomas, such as a transgenic mouse, in particular a mouse whose genome comprises a non natural c-myc sequence, may be.

In another embodiment of the method, the serum sample is isolated by centrifuging the blood sample.

In yet another embodiment of the method, the 2-DE is performed by using two different pH gradients, preferably by using the pH gradients 3-10 and 4-7.

In a further embodiment of the method, the lysis buffer comprises (a) at least one buffer component, (b) at least one chaotrope, (c) at least one detergens, (d) at least one reducing agent (e) at least one carrier ampholyte, and (f) at least one ribonuclease. Preferably, the lysis buffer used is an aqueous solution of (a) at least one buffer compound selected from the group consisting of Tris and HEPES, (b) at least one chaotrope selected from the group consisting of urea and thiourea, (c) at least one detergens selected from the group consisting of CHAPS and SDS, (d) at least one reducing agent selected from the group consisting of DTT and TCEP, (e) at least one carrier ampholyte selected from the group consisting of biolyte 5-7 and biolyte 3-10, and (f) at least one ribonuclease selected from the group consisting of endonuclease and exonuclease, wherein an aqueous solution of (a) Tris; (b) urea and thiourea, (c) CHAPS, (d) DTT, (e) biolyte 3-10, and (f) endonuclease, is particularly preferred. The lysis buffer comprises (a) at least one buffer component, (b) at least one chaotrope, (c) at least one detergens, (d) at least one reducing agent (e) at least one carrier ampholyte, (f) at least one ribonuclease is particularly preferred.

In yet a further embodiment of the method, the protein of interest is a biomarker selected from the first group of said biomarkers or is a biomarker selected from the second group of said biomarkers, in particular is selected from the first group consisting of A1AT1, A1AT6, A2MG, PROP, TTHY, A1AG8, APOA1, APOC3, APOH, GPX3, RETBP, SAMP, or more preferably, is selected from the first group consisting of A1AG8, APOA1, APOC3, APOH, GPX3, RETBP, SAMP, or is selected from the second group consisting of APOE.

Within the context of peptide mass fingerprinting, it is particularly preferred if the amount of A1AT1, A1AT6, A2MG, PROP, TTHY, A1AG8, APOA1, APOC3, APOH, GPX3, RETBP, SAMP and/or APOE is determined by determining the amount of the peptide fragments thereof, as described herein, in the digest mixture.

In another embodiment of the method the digesting buffer comprises a bicarbonate compound and a protease, wherein the digesting buffer preferably is an aqueous solution of at least one bicarbonate compound selected from the group consisting of ammonium bicarbonate and sodium bicarbonate and of at least one serine protease, in particular selected from the group consisting of trypsin, chymotrypsin and elastase, or, in particular preferred, the digesting buffer is an aqueous solution of ammonium bicarbonate and trypsin.

In yet another embodiment of the method, the mass spectrometry is selected from the group consisting of MALDI-TOF and ESI-TOF, preferably the mass spectrometry is performed by MALDI-TOF.

In a further embodiment of the method, a tandem mass spectrometer is used for the peptide mass fingerprinting, wherein a MALDI-TOF/TOF spectrometry is particularly preferred for putting the method into practice.

In yet a further embodiment of the method, a matrix is used for the mass spectrometry selected from the group consisting of 3,5-dimethoxy-4-hydroxycinnamic acid, α-cyano-4-hydroxycinnamic acid and 2,5-dihydroxybenzoic acid, wherein α-cyano-4-hydroxycinnamic acid is particularly preferred as the matrix.

In another preferred embodiment of the method, the serum sample is calibrated or the serum samples are equilibrated to a predefined protein concentration by adding the lysis buffer, thus allowing an easy adaption of the system to different purposes.

In particular, it is preferred, if the method further comprises the steps of
- determining the protein concentration of the serum sample, in particular by the Bradford method; or
- freezing and thawing the serum sample before the lysis buffer is added; and/or
- staining the gel after the 2-DE, in particular by using coomassie blue; and/or
- destaining the exised sample; or
- shrinking, in particular by adding acetonitrile, and drying of the excised sample before the digesting buffer is added; or
- using a peptide calibration standard for the mass spectrometry,
wherein preferably a combination of said steps, in particular two of said steps, more preferably three of said steps, in particular four or five of said steps, most preferably all of said steps are implemented.

Yet another aspect of the invention concerns a procedure to screen for and to identify drugs against cancer associated with an increased c-myc activity,
wherein the procedure comprises determining in a body fluid sample of a transgenic cancer mouse being treated with a compound to be tested, in particular of a mouse whose genome comprises a non natural c-myc sequence, at least one biomarker selected from the first group of said biomarkers and/or at least one biomarker selected from the second group of said biomarkers, and
wherein the body fluid level of the at least one biomarker of said first group being significantly lower and/or the body fluid level of the at least one biomarker of said second group being significantly higher than the level of said biomarker(s) in the body fluid of an untreated transgenic cancer mouse is indicative of the therapeutic effect of said compound as an c-myc activity modulator.

In a preferred embodiment, the procedure is implemented by using the method according the invention, in particular by using the method comprising an immunoassay or a peptide mass fingerprinting as described herein.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Examples

### Overview

Samples of transgenic lung tumor bearing mice and samples of healthy mice are used.
The protein concentration of samples which are examined is determined by means of Bradford protein assay. The serum proteins of transgenic and healthy control animals are extracted in a lysis buffer containing thiourea and isolated by 2-D gel electrophoresis according to their individual iso-electrical point as well as to their molecular weight.
After colloidal Coomassie blue staining of the 2-D gels, the coloured gel spots are quantified by means of software and significant differences between healthy and ill mice are determined. Subsequently, the differing spots are excised, destained and digested with trypsin. The peptides received are characterized by matrix-assisted laser desorption/ionization mass spectrometry (MALDI-TOF/TOF).

### Histopathology

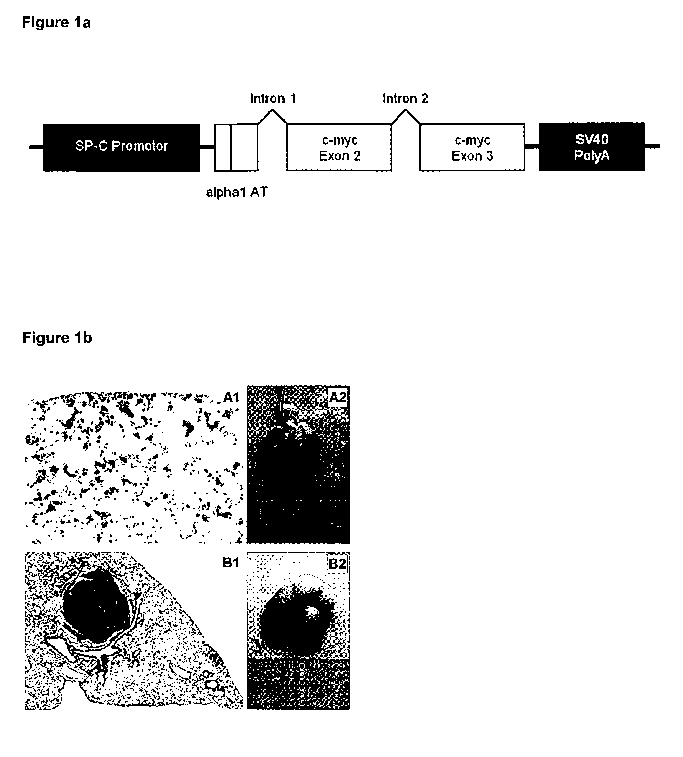

Transgenic lung tumor bearing mice are bred and kept as described previously [1]. Fig. 1 a depicts the gene construct while Fig. 1 b (B1, B2) displays typical features of the lung tumors. Tissues are fixed in 4% buffered paraformaldehyde for approximately 20 h, dehydrated and embedded in paraffin (Roti-Plast ^{™}, Roth). Tissue captions are stained with hematoxylin and eosin according to standard protocols. The mouse tumors are classified according to the IARC - WHO (2004).

### Sample preparation

Blood serum of lung tumor bearing SP-C/c-myc mice (n = 6, aged 14 months) and blood serum of healthy non-transgenic mice (n = 6, aged 14 months) that served as controls, are studied. Blood from healthy and tumor bearing mice is withdrawn from the vena cava. After clotting for 2 h at room temperature, the blood is centrifuged at 3500 rpm for 15 min. The resultant supernatants are removed and frozen immediately in liquid nitrogen and stored at -80°C until further analysis. The protein concentration in serum is determined by the Bradford protein assay (BioRad).

### Two-dimensional gel-electrophoresis

Each sample is analyzed in triplicate. Serum proteins are separated by isoelectric focusing (IEF) with precast IPG strips (pH 3-10, non-linear gradient and pH 4-7, linear gradient; both 170x3x0.5 mm, BioRad). 800 µg is diluted in a lysis buffer containing 2 mol/L thiourea, 5 mol/L urea, 40 mmol/L Tris, 4% CHAPS, 0.5% BioLyte 3-10 (BioRad), 100 mmol/L DTT resulting in a total volume of 350 µL per strip. Focused IPG strips are rehydrated at 50 V for 12 h. IEF is performed at 20°C with a maximum voltage of 10 kV and a maximum current of 50 µA per strip.
After IEF, IPG strips are equilibrated in 10 mL reducing buffer (2% DTT in 10 mL equilibration buffer containing 6 mol/L urea, 30% glycerin, 2% SDS, 0.05 mol/L Tris-HCl, pH 8.8 and 0.5% bromphenol blue) for 15 min, followed by equilibration in 10 mL alkylation buffer (4% iodoacetamide and 0.5% bromphenol blue in 10 mL equilibration buffer) for 15 min [13].
SDS-PAGE is performed in a Protean-plus Dodeca ^{™} Cell (BioRad) using self-cast polyacrylamide gels (200 x 205 x 1.5 mm; 12% T). Gels are run in parallel in 0.025 mol/L Tris/ 0.192 mol/L glycine/ 0.1% SDS at 10°C with a constant voltage of 70 V overnight. Precision Plus Protein Unstained Standard ^{™} (BioRad) is used for calibration of Mᵣ and pl.

### Staining and imaging

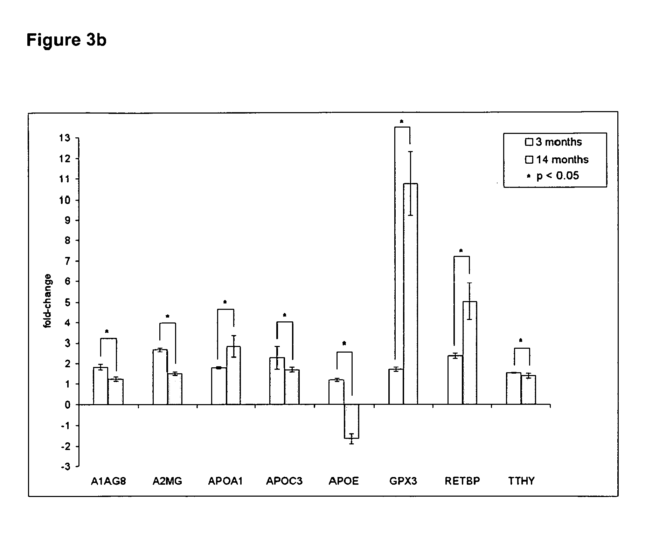

2-D Gels are fixed overnight in 500 mL 30% ethanol/ 2% phosphoric acid and washed three times for 20 min each in 500 mL 2% phosphoric acid. Equilibration is done with 500 mL 2% phosphoric acid/ 18% ethanol/ 15% ammonium sulfate thereafter. Colloidal Coomassie Brilliant Blue (CBB) staining of proteins is started by addition of 5 mL staining solution (2% CBB G250, Roth) to 500 mL of equilibration solution. Staining is carried out for 48 h and thereafter washed once with 500 mL water for 10 min. Gels are scanned with the Perfection 4990 Photo ^{™} densitometer (Epson). Detection of spots, quantification and comparison of 2-D protein profiles is done with the PDQuest 8.0 software (BioRad). After removal of background and vertical streaks from each gel image spots are digitized by Gaussian fit. To quantify protein spots, a matchset of all gels is made and the absorbance of individual protein spots from 2-D gels is measured. The raw quantity of each spot in a member gel is divided by the total intensity value of all the pixels in the image, for instance total density in gel image. This normalization procedure of the software assumed that the total density of an image, consisting of background and spot density will be relatively consistent from gel to gel.
The expression of serum proteins is analyzed by the Student's t-test. A probability of p < 0.05 is considered statistically significant (Tab. 2). Graphical evaluation is performed with the SigmaPlot (SPSS) software (Fig. 3b). Spots are excised and transferred to 96-well microtiter plates (ABgene) by the EXQuest ^{™} spot cutter (BioRad) as described previously [14].

### Mass spectrometry

### In-gel digestion

Each of the CBB-stained gel plugs is washed twice with 15 µL ammonium hydrogencarbonate solution (100 mmol/L) and then dehydrated twice with 15 µL acetonitrile. Proteins are digested with a total of 160 µg trypsin (13 ng/µL, sequencing grade, Promega) per gel plug at 37°C for 4 h. Resulting peptides are extracted with 8 µL n-Octyl-β-D-glucopyranoside (5 mmol/L, Applichem)/1% trifluoroacetic acid in an ultrasonic bath (Sonorex, Super RK 514 BH, Bandelin) for 5 min.

### MALDI-TOF/TOF

The HCCA matrix is prepared with the thin layer method. 1 µL of the peptide extracts were manually spotted onto a 600 µm/384 well AnchorChip ^{™} sample target (Bruker Daltonics) and dried at ambient temperature. Recristallization was performed with 1 µL of 60% ethanol/ 30% acetone/ 10% of 1% trifluoroacetic acid thereafter. MALDI mass spectra are recorded using a Ultraflex II TOF/TOF mass spectrometer (Bruker Daltonics) equipped with a 384-sample scout source. A peptide calibration standard (Bruker Daltonics) is used for external calibration. MS and MS/MS data are recorded automatically on the MALDI-TOF/TOF instrument using the three most abundant peptide signals of the corresponding peptide mass fingerprint (PMF) spectrum. The Swiss-Prot database employing the Mascot program (version 2.0, Matrix Science, in-house server) is used for the search of peptide masses to identify proteins [15]. Database searches are performed taking into account carbamidomethyl modification of cysteines and possible oxidation of methionine. One missed cleavage was allowed. A mass accuracy of ≤ 100 ppm is requested for PMF. For MS/MS searches, a mass accuracy of ≤ 70 ppm is requested for peptide masses and their fragments, respectively. Identified proteins are sent to the Proteinscape ^{™} database (Protagen) and checked individually for further consideration.

### Results and discussion

### Separation of serum proteins by 2-DE

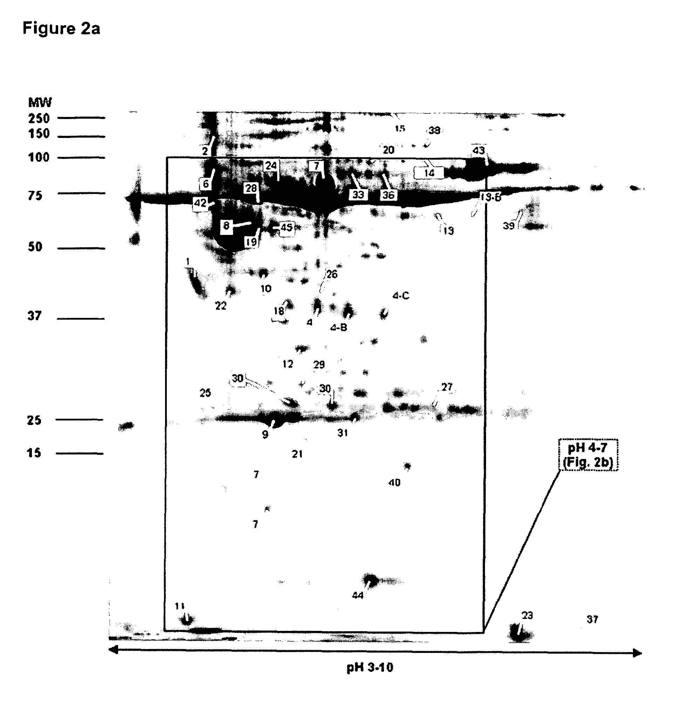

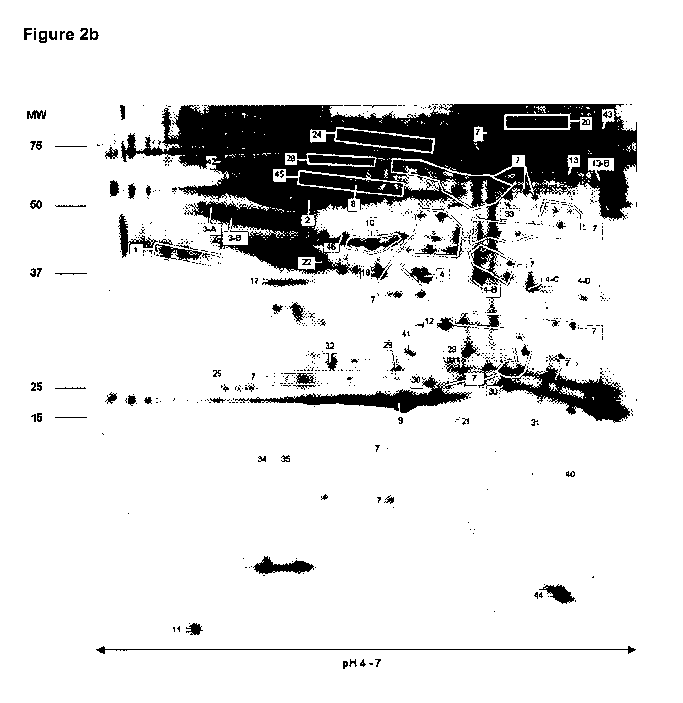

A thiourea-containing lysis buffer is used [16, 17] to extract proteins from serum. Proteins are separated within pH ranges of 3-10 and 4-7. Proteins are visualized with the colloidal CBB (CCB) stain. Approximately 400 (pH 3-10) and 200 (pH 4-7) spots per gel are detected. Fig. 2a and Fig. 2b depict examples of serum proteome maps (pH 3-10 and 4-7) of c-myc tumor bearing mice.

### Protein identification

72 2-D gels are stained with the CCB method. About 350 (pH 3-10) and 170 (pH 4-7) spots per gel are excised, resulting in 12.500 spots in total. Protein spots are analyzed by MALDI-MS and -MS/MS after tryptic in-gel digest. Identification is based on Swiss-Prot database entries with the Mascot search engine. In tumor bearing and healthy non-transgenic mice, 46 common (Tab. 1) serum proteins are identified. In Supplementary Table 1 the Mascot score, sequence coverage and the most informative peptide sequences identified by MS is listed. Within 2-DE mouse serum proteome mapping, three novel serum proteins, not reported so far, are identified (Tab. 1). These include orosomucoid-8 **(spot no. 1),** C-reactive protein **(spot no. 18)** and cytokeratin-8 **(spot no. 26).**

### Regulation of serum proteins in lung cancer

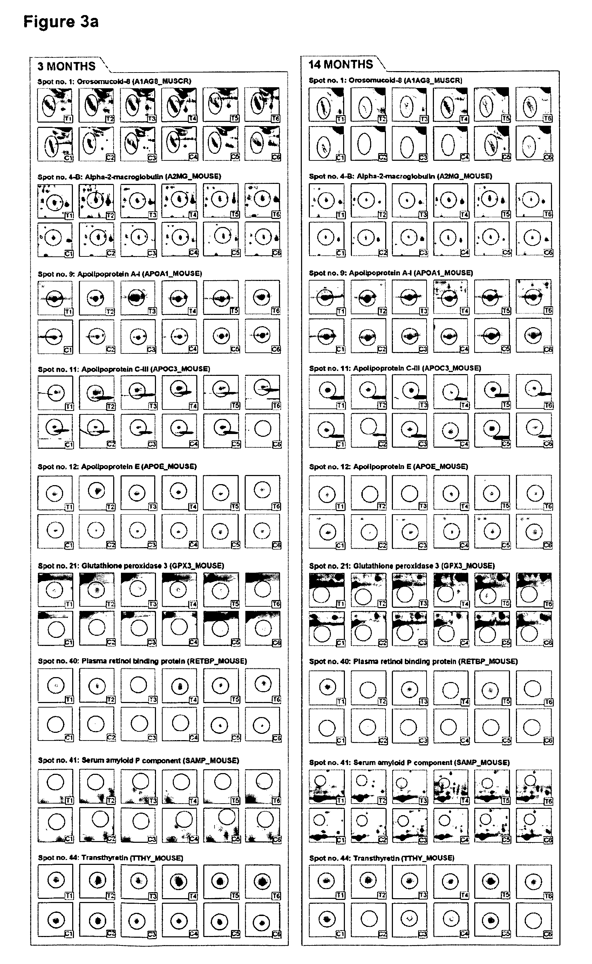

Tab. 2 and Fig. 3b show expression profiles of 13 proteins (p < 0.05) when extracts of healthy and lung tumor serum proteomes are compared, whereas Fig. 3a depicts prominent examples of regulated proteins in tumor bearing mice. Differentially expressed proteins are discussed below for their disease association. Their relationship to the c-myc proto-oncogene is determined by comparison of database entries from http://www.myccancergene.org [47]. Furthermore, results are compared with another study on the serum proteome of c-raf transgenic mice which developed lung tumors as well.

### Acute phase proteins

As observed in serum of healthy and tumor bearing mice of our present study, eight acute phase proteins are regulated. The positive acute phase proteins, e.g. alpha-1 acid glycoprotein-8, also named orosomucoid-8 (**spot no. 1**), alpha-1 antitrypsin (**spot no. 2** and **spot no. 3**), alpha-2 macroglobulin (**spot no. 4**) and serum amyloid P component (**spot no. 41**) are found to be increased or exclusively expressed in tumor bearing mice. In contrast, the negative acute phase proteins (n-APP) plasma retinol-binding protein (**spot no. 40**), transthyretin (**spot no. 44**) and serum albumin (**spot no. 7**) are either up- or downregulated.
In particular, alpha-2 macroglobulin (A2M, **spot no. 4**) is mainly produced in the liver, but in the lung as well. With a molecular weight of 165 kDa, A2M represents a large plasma protein that consists of four identical subunits that are linked together by disulfide bonds. These subunits are visible at 37 kDa in our 2-D gel (Fig. 2b). Specifically, A2M acts as a proteinase inhibitor and targets serine-, cysteine-, aspartic- and metalloproteinases. The A2M structure includes a "decoy" region where such proteinases are bound and cleaved. Macrophage receptors recognize and eliminate the A2M-proteinase complex.

Serum A2M levels are useful for diagnosis and therapeutic monitoring in lung cancer and in bone metastases of prostate cancer as reported elsewhere [19].

Here a significant (p < 0.05), > 6-fold (pH 4-7) and 1.5-fold (pH 3-10) increase of alpha-2 macroglobulin levels in serum of tumor bearing mice is demonstrated.
Serum amyloid P component (SAMP, **spot no. 41**) is a member of the pentraxins, produced in the liver. SAMP has a sequence homology of 51% with the C-reactive protein **(spot no. 18**), a classical plasma APP as well. Likewise, SAMP is exclusively expressed in tumor bearing mice.
Transthyretin, also named prealbumin **(spot no. 44**) is a common blood protein. It is a carrier for thyroid hormones from bloodstream to tissues. Transthyretin interacts with the retinol binding protein (RBP, **spot no. 40**), thus enabling retinol transportation. If transthyretin is not expressed, lower levels of retinol and RBP are observed
Since a decrease of transthyretin levels in serum is linked to a negative acute phase reaction during inflammation as well, an increase of this protein might be a significant biomarker for cancers.

Likewise, upregulation of transthyretin by 1.4-fold in serum of lung tumor bearing mice alongwith expression of RBP by 2.5-fold (pH 4-7) and 5-fold (pH 3-10) is demonstrated.

### Apolipoproteins

Several apolipoproteins regulated in tumor bearing mice are found. Here the differential expression of apolipoproteins A, C, E and H in c-myc transgenic mice is reported.
Apolipoprotein A-I (ApoA1, spot no. 9) belongs to the ApoA1/A4/E protein family and is primarily produced in the liver and the intestine. ApoA1 is found in the extracellular space and, being a structural component of high density lipid proteins (HDL), takes part in cholesterol absorption.

The ApoA1 expression is found to be significantly increased by 1.4-fold (pH 4-7) and 2.8-fold (pH 3-10) in tumor bearing mice as well.
**Spot no. 11** is identified as apolipoprotein C3 (ApoC3) which is produced in the liver, inhibits the lipoprotein and hepatic lipase and represses the uptake of lymph chylomicrons by hepatic cells. Thus, ApoC3 may repress the catabolism of triglyceride-rich particles. Upregulation of ApoC3 is demonstrated in a chronic renal failure model and in diabetes. A regulation of ApoC3 in lung cancer is not reported so far. Serum levels of ApoC3 are increased by 2.7-fold (pH 4-7) and 1.7-fold (pH 3-10) in lung tumor bearing mice. Apolipoprotein E (ApoE, **spot no. 12**) is a mediator for binding, internalizing and metabolism of lipoprotein particles. It serves as a ligand for the low density lipoprotein (LDL) receptor and for the ApoE receptor (chylomicron remnant) of hepatic tissues. ApoE expression is reduced in serum of tumor bearing mice by 1.6-fold (pH 4-7) and 1.2-fold (pH 3-10).
Apolipoprotein H, also named as beta-2 glycoprotein-1 (ApoH, **spot no. 13**) binds to various kinds of negatively charged substances such as heparin, phospholipids and dextran sulfate. Through binding to phospholipids on the surface of damaged cells, ApoH inhibits activation of the intrinsic blood coagulation cascade. ApoH is synthesized in the liver and secreted into plasma.

ApoH expression is induced in sera of lung tumor bearing mice by 1.4-fold (pH 4-7) and 1.7-fold (pH 3-10) and is a novel finding for its regulation in lung cancer.

### Oxidative defense and complement activation

Glutathione peroxidase 3 (Gpx3, **spot no. 21**) functions in response to oxidative damage by catalyzing the reduction of hydrogen peroxide, lipid peroxides and organic hydroperoxide

An upregulation of Gpx3 in serum of lung tumor bearing mice by > 10-fold (pH 3-10) is observed.

Properdin (**spot no. 39**), also known as factor P, is a serum glycoprotein and positive regulator of the alternate pathway for complement activation. It binds to and stabilizes the C3- and C5-convertase enzyme complexes. Complement C3 (**spot no. 17**) is not regulated in tumor bearing mice whereas properdin expression is increased by 1.5-fold. Properdin plays a role in some specific immune responses and in tissue inflammation.

### Comparison with previous results

In our present study on c-myc transgenic mice, regulation of various serum proteins is unique to bronchiolo-alveolar carcinomas (BAC). Compared with our serum proteomic study on c-raf transgenic mice a large number of common proteins between these two mouse models were identified (Suppl. Tab. 2). Strikingly, the data of regulated proteins are highly suggestive for candidate serum biomarkers to differentiate between AAH and BAC. Table 3 gives an overview of those findings. For instance, alpha-1 antitrypsin, alpha-2 macroglobulin, transthyretin and properdin are found in common in terms of their regulation in serum of tumor bearing mice. Notably, several proteins described in the previous sections, such as apolipoproteins, plasma retinol binding protein or serum amyloid P component are exclusively regulated in serum of BAC. In contrast, regulation of major urinary proteins, vitamin D-binding protein or a soluble form of the epidermal growth factor receptor (EGFR) in serum is unique to AAH.

### Conclusions

Disease regulated serum proteins at advanced stages of lung cancer are reported. Serum protein expression of tumor bearing mice is compared with those of healthy animals. Separation of serum proteins at two different pH ranges yielded a total of 46 distinct proteins, some of which are direct gene targets of the c-myc transcription factor, e.g. alpha-2 macroglobulin (A2M), actin, albumin, complement factor B, fetuin A and hemoglobin subunit alpha. Some of the identified proteins are known as acute phase proteins (APP) and were regulated or exclusively expressed, notably orosomucoid-8, alpha-1 antitrypsin (A1AT), A2M, serum amyloid P component (SAMP), plasma retinol binding protein (RBP), transthyretin and serum albumin.

Glutathione peroxidase 3 (Gpx3), properdin and transthyretin are found to be upregulated in tumor bearing mice. Gpx3 is an antioxidant and is known to be associated with ovarian cancer; properdin plays a role in immune response and inflammation.

Note, as expression of SP-C is restricted to alveolar epithelium (AE), this gene construct enabled targeted expression of the transgene to this specific respiratory epithelium. There is growing evidence for AE to contribute to as much as 30% of all lung adenocarcinomas. Furthermore, AE functions in part as a stem cell with limited capacity for self renewal.

A number of disease regulated proteins were in common, such as A1AT6, A2M, transthyretin and properdin. Strikingly, regulation of several serum proteins was unique to bronchiolo-alveolar carcinomas (Tab. 3).

Taken collectively, this tumor model provides novel information on the serum proteome, some of which could already be translated to human malignant diseases.

### Figure captions

### Figure 1a:

Gene construct of the SP-C/c-myc transgenic mouse model as reported by Ehrhardt et al. [1].

### Figure 1b:

Histology of a lung of a healthy (A1) and a tumor bearing (B1) SP-C/c-myc transgenic mouse, aged 14 months. Hematoxylin and eosin staining is used for histopathology of tumors. A2 and B2: macroscopical views of lungs of healthy and tumor bearing mice, respectively.

### Figure 2a:

2-DE serum proteome map of lung tumor bearing SP-C/c-myc transgenic mice with a pH range from 3-10.

### Figure 2b:

2-DE serum proteome map of lung tumor bearing SP-C/c-myc transgenic mice with a pH range from 4-7.

### Figure 3a:

Examples of regulated serum proteins in tumor bearing SP-C/c-myc transgenic (T) and healthy (C) mice. Protein spots of interest are marked by circles.

### Figure 3b:

ppm values of 13 disease regulated proteins. A: control pH 4-7, B: tumor pH 4-7, C: control pH 3-10, D: tumor pH 3-10.

### Table captions

**Table 1: Protein** identification in 2-DE maps of mouse serum proteins including healthy and lung tumor bearing mice, identified by MALDI MS. A total of 46 proteins are identified. See Supplementary Table 1 for detailed information.

**Table 2:** Expression profiles of 13 significantly regulated proteins (p < 0.05) from 2-D gels with pH 4-7 and pH 3-10. Quantification of protein abundance is done using the PDQuest 2-D software (BioRad) by measurement of the normalized optical density (arbitrary units, AU) of each protein spot. The change in abundance of the proteins is expressed by the calculated ratio (T/C) between mean values from tumor (T) and healthy (C) samples. %RSD: percental relative standard deviation. Spot no. 41 (SAMP_MOUSE) is exclusively expressed in tumor bearing mice. Student's t-test is used for calculation of p-values.

**Table 3:** Common and specific regulated proteins in adenomatous hyperplasia (AAH) and bronchiolo-alveolar adenocarcinomas (BAC). Recently, AAH is studied in SP-C/c-raf transgenic mice [46], whereas BAC is the subject of our present work on SP-C/c-myc transgenic mice. Yes/No: protein regulation, Yes*: exclusive protein expression either in healthy or tumor bearing mice.

### Supplementary Table 1:

A summary of mouse serum proteins. The Mascot score (PMF), ions score (PFF), the number of identified peptides, their sequence, the protein coverage of the best hits and supporting information are shown for each identified protein. O@M: oxidation at the amino acid methionine.

### Supplementary Table 2:

Investigating the mouse serum proteome. 46 distinct proteins in healthy and tumor bearing mice are identified. From these, 3 proteins are novel and not reported so far in 2-D mouse serum proteome maps, for instance, by Duan et al. [44] (38 distinct proteins), Wait et al. [45] (30 distinct proteins) our recent serum proteomics study on c-raf transgenic mice [46] (45 distinct proteins) and Hood et al. [49].

### References

[1] Ehrhardt A, Bartels T, Geick A, Klocke R et al. Development of pulmonary bronchiolo-alveolar adenocarcinomas in transgenic mice overexpressing murine c-myc and epidermal growth factor in alveolar type II pneumocytes. Br J Cancer 2001, 84, 813-818.
[2] Duarte RL, Paschoal ME. Molecular markers in lung cancer: prognostic role and relationship to smoking. J Bras Pneumol 2006, 32, 56-65. Review.
[3] Bhattacharjee A, Richards WG, Staunton J, Li C et al. Classification of human lung carcinomas by mRNA expression profiling reveals distinct adenocarcinoma subclasses. Proc Natl Acad Sci USA 2001, 98, 13790-13795.
[4] Takezawa C, Takahashi H, Fujishima T, Shiratori M et al., Assessment of differentiation in adenocarcinoma cells from pleural effusion by peripheral airway cell markers and their diagnostic values. Lung Cancer 2002, 38, 273-281.
[5] Fields WR, Desiderio JG, Putnam KP, Bombick DW, Doolittle DJ. Quantification of changes in c-myc mRNA levels in normal human bronchial epithelial (NHBE) and lung adenocarcinoma (A549) cells following chemical treatment. Toxicol Sci 2001, 63, 107-114.
[6] Broers JL, Viallet J, Jensen SM, Pass H et al. Expression of c-myc in progenitor cells of the bronchopulmonary epithelium and in a large number of non-small cell lung cancers. Am J Respir Cell Mol Biol 1993, 9, 33-43.
[7] Facchini LM, Penn LZ. The molecular role of Myc in growth and transformation: recent discoveries lead to new insights. FASEB J 1998, 12, 633-651.
[8] Amati B, Frank SR, Donjerkovic D, Taubert S. Function of the c-Myc oncoprotein in chromatin remodeling and transcription. Biochim Biophys Acta 2001, 1471, 135-145. Review.
[9] Lee LA, Dang CV. Myc target transcriptomes. Curr Top Microbiol Immunol 2006, 302, 145-167. Review.
[10] Prochownik EV. c-Myc as a therapeutic target in cancer. Expert Rev Anticancer Ther 2004, 4, 289-302. Review.
[11] Dang CV. c-Myc target genes involved in cell growth, apoptosis, and metabolism. Mol Cell Biol 1999, 19, 1-11. Review.
[12] Ehrhardt A, Bartels T, Klocke R, Paul D, Halter R. Increased susceptibility to the tobacco carcinogen 4-(methylnitrosamino)-1-(3-pyridyl)-1-butanone in transgenic mice overexpressing c-myc and epidermal growth factor in alveolar type II cells. J Cancer Res Clin Oncol 2003, 129, 71-75.
[13] Görg A, Postel W, Weser J, Günther S et al. Elimination of point streaking on silver stained two-dimensional gels by addition of iodoacetamide to the equilibration buffer. Electrophoresis 1987, 8, 122-124.
[14] Rütters H, Zürbig P, Halter R, Borlak J. Towards a lung adenocarcinoma proteome map: studies with SP-C/c-raf transgenic mice. Proteomics 2006, 10, 3127-3137.
[15] Perkins DN, Pappin DJ, Creasy DM, Cottrell JS. Probability-based protein identification by searching sequence databases using mass spectrometry data. Electrophoresis 1999, 20, 3551-3567.
[16] Lehner I, Niehof M, Borlak J. An optimized method for the isolation and identification of membrane proteins. Electrophoresis 2003, 24, 1795-1808.
[17] Rabilloud T. Use of thiourea to increase the solubility of membrane proteins in two-dimensional electrophoresis. Electrophoresis 1998, 19, 758-760.
[18] Ceciliani F, Giordano A, Spagnolo V. The systemic reaction during inflammation: the acute-phase proteins. Protein Pept Lett 2002, 9, 211-223. Review.
[19] Kanoh Y, Ohtani N, Mashiko T, Ohtani S et al. Levels of alpha 2 macroglobulin can predict bone metastases in prostate cancer. Anticancer Res 2001, 21, 551-556.
[20] Misra UK, Pizzo SV. Regulation of cytosolic phospholipase A2 activity in macrophages stimulated with receptor-recognized forms of alpha 2-macroglobulin: role in mitogenesis and cell proliferation. J Biol Chem 2002, 277, 4069-4078.
[21] Pepys MB, Herbert J, Hutchinson WL, Tennent GA et al. Targeted pharmacological depletion of serum amyloid P component for treatment of human amyloidosis. Nature 2002, 417, 254-259.
[22] Van Bennekum AM, Wei S, Gamble MV et al. Biochemical basis for depressed serum retinol levels in transthyretin-deficient mice. J Biol Chem 2001, 276, 1107-1113.
[23] Roberts D, Williams SJ, Cvetkovic D et al. Decreased expression of retinol-binding proteins is associated with malignant transformation of the ovarian surface epithelium. DNA Cell Biol 2002, 21, 11-19.
[24] Zhang Z, Bast RC Jr, Yu Y, Li J et al. Three biomarkers identified from serum proteomic analysis for the detection of early stage ovarian cancer. Cancer Res 2004, 64, 5882-5890.
[25] Moore LE, Fung ET, McGuire M, Rabkin CC et al. Evaluation of apolipoprotein A1 and posttranslationally modified forms of transthyretin as biomarkers for ovarian cancer detection in an independent study population. Cancer Epidemiol Biomarkers Prev 2006, 15, 1641-1646.
[26] Maciel CM, Junqueira M, Paschoal ME, Kawamura MT et al. Differential proteomic serum pattern of low molecular weight proteins expressed by adenocarcinoma lung cancer patients. J Exp Ther Oncol 2005, 5, 31-38.
[27] Feng JT, Liu YK, Song HY, Dai Z et al. Heat-shock protein 27: a potential biomarker for hepatocellular carcinoma identified by serum proteome analysis. Proteomics 2005, 5, 4581-4588.
[28] Cham BE. Importance of apolipoproteins in lipid metabolism. Chem Biol Interact 1978, 20, 263-277. Review.
[29] Chen J, Anderson M, Misek DE, Simeone DM, Lubman DM. Characterization of apolipoprotein and apolipoprotein precursors in pancreatic cancer serum samples via two-dimensional liquid chromatography and mass spectrometry. J Chromatogr A 2007 Mar 30; [Epub ahead of print]
[30] Venanzoni MC, Giunta S, Muraro GB, Storari L et al. Apolipoprotein E expression in localized prostate cancers. Int J Oncol 2003, 22, 779-786.
[31] Wright LC, Sullivan DR, Muller M, Dyne M et al. Elevated apolipoprotein(a) levels in cancer patients. Int J Cancer 1989, 43, 241-244.
[32] Han C, Zhang HT, Du L, Liu X et al. Serum levels of leptin, insulin, and lipids in relation to breast cancer in china. Endocrine 2005, 26, 19-24.
[33] Chang SI, El-Bayoumy K, Sinha I, Trushin N et al. 4-(Methylnitrosamino)-I-(3-pyridyl)-1-butanone enhances the expression of apolipoprotein A-I and Clara cell 17-kDa protein in the lung proteomes of rats fed a corn oil diet but not a fish oil diet. Cancer Epidemiol Biomarkers Prev 2007, 16, 228-235.
[34] Ishikawa I, Hayama T, Yoshida S, Asaka M et al. Proteomic analysis of rat plasma by SELDI-TOF-MS under the condition of prevention of progressive adriamycin nephropathy using oral adsorbent AST-120. Nephron Physiol 2006, 103, 125-130.
[35] Hiukka A, Fruchart-Najib J, Leinonen E, Hilden H et al. Alterations of lipids and apolipoprotein CIII in very low density lipoprotein subspecies in type 2 diabetes. Diabetologia 2005, 48, 1207-1215.
[36] Yokoyama Y, Kuramitsu Y, Takashima M, lizuka N, et al. Protein level of apolipoprotein E increased in human hepatocellular carcinoma. Int J Oncol 2006, 28, 625-631.
[37] Li ZG, Zhao L, Liu L, Ding YQ. [Monitoring changes of serum protein markers in metastatic colorectal carcinoma model]. Zhonghua Bing Li Xue Za Zhi 2007, 36, 48-52. Chinese.
[38] Grainger DJ, Reckless J, McKilligin E. Apolipoprotein E modulates clearance of apoptotic bodies in vitro and in vivo, resulting in a systemic proinflammatory state in apolipoprotein E-deficient mice. J Immunol 2004, 173, 6366-6375.
[39] Beecken WD, Engl T, Ringel EM, Camphausen K et al. An endogenous inhibitor of angiogenesis derived from a transitional cell carcinoma: clipped beta2-glycoprotein-I. Ann Surg Oncol 2006, 13, 1241-1251.
[40] Hough CD, Cho KR, Zonderman AB, Schwartz DR, Morin PJ. Coordinately up-regulated genes in ovarian cancer. Cancer Res 2001, 61, 3869-3876.
[41] Iwata K, Nishinaka T, Matsuno K, Yabe-Nishimura C. Increased gene expression of glutathione peroxidase-3 in diabetic mouse heart. Biol Pharm Bull 2006, 29, 1042-1045.
[42] Smida J, Holubek V, Oravec C, Thurzo V. The properdin system in tumorous disease. Part VI. Influence of the properdin fraction on the cancerogenic activity of chicken tumour virus B77. Neoplasma 1960, 7, 26-30.
[43] Weaver TR, Whitsett JA. Function and regulation of expression of pulmonary surfactant-associated proteins. Biochem J 1991, 273, 249-264.
[44] Duan X, Yarmush DM, Berthiaume F, Jayaraman A, Yarmush ML. A mouse serum two-dimensional gel map: application to profiling burn injury and infection. Electrophoresis 2004, 25, 3055-3065.
[45] Wait R, Chiesa G, Parolini C, Miller I et al. Reference maps of mouse serum acute-phase proteins: changes with LPS-induced inflammation and apolipoprotein A-I and A-II transgenes. Proteomics 2005, 5, 4245-4253.
[46] Chatterji B, Borlak J. Serum proteomics of lung adenocarcinomas induced by targeted overexpression of c-raf in alveolar epithelium identifies candidate biomarkers. Proteomics 2007, 7, 3980-91.
[47] Zeller KI, Jegga AG, Aronow BJ, O'Donnell KA, Dang CV. An integrated database of genes responsive to the Myc oncogenic transcription factor: identification of direct genomic targets. Genome Biol. 2003, 4, R69.
[48] Korbelik M, Cecic I, Merchant S, Sun J. Acute phase response induction by cancer treatment with photodynamic therapy. Int J Cancer 2007, Nov 21
[49] Hood BL, Zhou M, Chan KC, Lucas DA et al. Investigation of the mouse serum proteome. J Proteome Res. 2005, 4, 1561-1568.

**Table 1**

| **No.** | **Protein ID** | **Accession** | **Protein name** | **c-myc target** | **APP** |
|---|---|---|---|---|---|
| 1 | A1AG8_MUSCR | P21352 | Orosomucoid-8 | No | Yes |
| 2 | A1AT1_MOUSE | P07758 | Alpha-1-antitrypsin 1-1 | No | Yes |
| 3 | A1AT6_MOUSE | P81105 | Alpha-1-antitrypsin 1-6 | No | Yes |
| 4 | A2MG_MOUSE | Q61838 | Alpha-2-macroglobulin | Yes | Yes |
| 5 | ACTG_MOUSE | P63260 | Gamma-actin | Yes | No |
| 6 | AFAM_MOUSE | 089020 | Afamin | No | No |
| 7 | ALBU_MOUSE | P07724 | Serum Albumin | Yes | Yes |
| 8 | ANT3_MOUSE | P32261 | Antithrombin-III | No | No |
| 9 | APOA1_MOUSE | Q00623 | Apolipoprotein A-I | No | No |
| 10 | APOA4_MOUSE | P06728 | Apolipoprotein A-IV | No | No |
| 11 | APOC3_MOUSE | P33622 | Apolipoprotein C-III | No | No |
| 12 | APOE_MOUSE | P08226 | Apolipoprotein E | No | No |
| 13 | APOH_MOUSE | Q01339 | Apolipoprotein H | No | No |
| 14 | CFAB_MOUSE | P04186 | Complement factor B | Yes | No |
| 15 | CFAH_MOUSE | P06909 | Complement factor H | No | No |
| 16 | CLUS_MOUSE | Q06890 | Clusterin (Apolipoprotein J) | No | No |
| 17 | CO3_MOUSE | P01027 | Complement C3 | No | Yes |
| 18 | CRP_MOUSE | P14847 | C-reactive protein | No | Yes |
| 19 | FETUA_MOUSE | P29699 | Fetuin-A | Yes | No |
| 20 | GELS_MOUSE | P13020 | Gelsolin | No | No |
| 21 | GPX3_MOUSE | P46412 | Glutathione peroxidase 3 | No | No |
| 22 | HA10_MOUSE | P01898 | H-2 class I histocompatibility antigen, Q10 alpha chain | No | No |
| 23 | HBA_MOUSE | P01942 | Hemoglobin subunit alpha | Yes | No |
| 24 | HEMO_MOUSE | Q91X72 | Hemopexin | No | No |
| 25 | IGJ_MOUSE | P01592 | Immunglobulin J chain | No | No |
| 26 | K2C8_MOUSE | P11679 | Cytokeratin-8 | No | No |
| 27 | KAC_MOUSE | P01837 | Immunglobulin kappa chain C region | No | No |
| 28 | KNG1_MOUSE | 008677 | Kininogen-1 | No | No |
| 29 | KV3B_MOUSE | P01655 | Immunglobulin kappa chain V-III region PC7132 | No | No |
| 30 | KV5L_MOUSE | P01645 | Immunglobulin kappa chain V-V region HP93G7 | No | No |
| 31 | KV6K_MOUSE | P04945 | Immunglobulin kappa chain V-VI region NQ2-6.1 | No | No |
| 32 | MBL2_MOUSE | P41317 | Mannose-binding protein C | No | No |
| 33 | MUC_MOUSE | P01872 | Immunglobulin mu chain C region secreted form | No | No |
| 34 | MUP2_MOUSE | P11589 | Major urinary protein 2 | No | No |
| 35 | MUP8_MOUSE | P04938 | Major urinary proteins 11 and 8 | No | No |
| 36 | MYH9_MOUSE | Q8VDD5 | Myosin-9 | No | No |
| 37 | PLF4_MOUSE | Q9Z126 | Platelet factor 4 | No | No |
| 38 | PLMN_MOUSE | P20918 | Plasminogen | No | No |
| 39 | PROP_MOUSE | P11680 | Properdin | No | No |
| 40 | RETBP_MOUSE | Q00724 | Plasma retinol-binding protein | No | Yes |
| 41 | SAMP_MOUSE | P12246 | Serum amyloid P-component | No | Yes |
| 42 | SPA3K_MOUSE | P07759 | Contrapsin | No | No |
| 43 | TRFE_MOUSE | Q92111 | Serotransferrin | No | Yes |
| 44 | TTHY_MOUSE | P07309 | Transthyretin | No | Yes |
| 45 | VTDB_MOUSE | P21614 | Vitamin D-binding protein | No | No |
| 46 | ZA2G_MOUSE | Q64726 | Zinc-alpha-2-glycoprotein | No | No |

**Table 2**

| No. | pH | Protein ID | Tumor | | | | | | | | Control | | | | | | | | Ratio (T/C) | p |
|---|---|---|---|---|---|---|---|---|---|---|---|---|---|---|---|---|---|---|---|---|
| | | | T1 | T2 | T3 | T4 | T5 | T6 | mean | %RSD | C1 | C2 | C3 | C4 | C5 | C6 | mean | %RSD | | |
| 1 | pH 4- 7 | A1AG8_MUSCR | 1139 | 1003 | 1123 | 998 | 1217 | 867 | 1058 | 11.88 | 425 | 398 | 401 | 414 | 502 | 387 | 421 | 9.91 | 2.5 | 3.50 x 10⁻⁰⁷ |
| 1 | pH 3- 10 | A1AG8_MUSCR | 834 | 745 | 769 | 895 | 744 | 867 | 809 | 8.07 | 780 | 637 | 545 | 715 | 679 | 552 | 651 | 14.20 | 1.2 | 6.64 x 10⁻⁰³ |
| 2 | pH 4- 7 | A1AT1_MOUSE | 474 | 456 | 494 | 485 | 541 | 391 | 473 | 10.39 | 400 | 384 | 482 | 314 | 391 | 327 | 383 | 15.65 | 1.2 | 1.73 x 10⁻⁰² |
| 2 | pH 3- 10 | A1AT1_MOUSE | 518 | 616 | 549 | 863 | 636 | 663 | 641 | 18.99 | 498 | 344 | 475 | 555 | 475 | 562 | 485 | 16.26 | 1.3 | 216 x 10⁻⁰² |
| 3 | pH 4- 7 | A1AT6_MOUSE | 781 | 848 | 891 | 899 | 884 | 916 | 870 | 5.61 | 630 | 686 | 752 | 618 | 812 | 711 | 702 | 10.51 | 1.2 | 8.90 x 10⁻⁰⁴ |
| 3 | ph 4- 7 | A1AT6_MOUSE | 450 | 546 | 502 | 446 | 680 | 486 | 518 | 16.82 | 349 | 354 | 306 | 359 | 410 | 484 | 377 | 16.46 | 1.4 | 8.85 x 10⁻⁰³ |
| 3 | pH 3- 10 | A1AT6_MOUSE | 0 | 0 | 0 | 0 | 0 | 0 | 0 | 0 | 0 | 0 | 0 | 0 | 0 | 0 | 0 | 0 | 0 | 0 |
| 4 | pH 4- 7 | A2MG_MOUSE | 5588 | 5789 | 4708 | 5609 | 5332 | 6008 | 5506 | 8.19 | 867 | 935 | 766 | 790 | 846 | 910 | 852 | 7.74 | 6.5 | 2.39 x 10⁻¹⁰ |
| 4 | pH 3- 10 | A2MG_MOUSE | 6773 | 6059 | 6128 | 6592 | 5845 | 6307 | 6284 | 5.52 | 4176 | 4044 | 4087 | 4153 | 4900 | 3793 | 4192 | 8.89 | 1.5 | 1.51 x 10⁻⁰⁶ |
| 9 | pH 4- 7 | APOA1_MOUSE | 5423 | 5219 | 4868 | 5172 | 4972 | 5267 | 5154 | 3.93 | 3490 | 4036 | 3362 | 3379 | 4004 | 3313 | 3597 | 9.25 | 1.4 | 1.93 x 10⁻⁶ |
| 9 | pH 3- 10 | APOA1_MOUSE | 5952 | 4324 | 6205 | 5549 | 5869 | 7721 | 5937 | 18.47 | 1603 | 1725 | 2017 | 2471 | 2260 | 2495 | 2095 | 18.04 | 2.8 | 1.04 x 10⁻⁰⁵ |
| 11 | pH 4- 7 | APOC3_MOUSE | 3433 | 3693 | 3472 | 3132 | 3217 | 3552 | 3417 | 6.02 | 1313 | 1342 | 1236 | 1511 | 1189 | 1096 | 1281 | 11.17 | 2.7 | 1.57 x 10⁻⁹ |
| 11 | pH 3- 10 | APOC3_MOUSE | 3682 | 3357 | 3318 | 3716 | 3217 | 3885 | 3529 | 7.57 | 2372 | 1630 | 1749 | 2522 | 2131 | 2018 | 2070 | 16.71 | 1.7 | 9.73 x 10⁻⁰⁶ |
| 12 | pH 4- 7 | APOE_MOUSE | 2502 | 2139 | 2619 | 2204 | 2553 | 2267 | 2381 | 8.48 | 2478 | 2312 | 2823 | 2908 | 3116 | 2935 | 2762 | 11.02 | 0.9 | 2.85 x 10⁻⁰² |
| 12 | pH 3- 10 | APOE_MOUSE | 1857 | 1727 | 1294 | 1657 | 1553 | 1834 | 1654 | 12.65 | 2993 | 2207 | 2688 | 2876 | 2598 | 2978 | 2723 | 10.95 | 0.6 | 2.95 x 10⁻⁰⁵ |
| 13 | pH 4- 7 | APOH_MOUSE | 2377 | 2773 | 2292 | 2783 | 2365 | 2131 | 2454 | 10.85 | 1525 | 1330 | 1774 | 2073 | 2020 | 1947 | 1778 | 16.66 | 1.4 | 1.97 x 10⁻⁰³ |
| 13 | pH 3- 10 | APOH_MOUSE | 1148 | 1281 | 1269 | 1724 | 1247 | 1410 | 1347 | 15.08 | 845 | 752 | 809 | 898 | 749 | 683 | 790 | 9.75 | 1.7 | 9.11 x 10⁻⁰⁵ |
| 21 | pH 4- 7 | GPX3_MOUSE | 520 | 339 | 547 | 506 | 358 | 541 | 469 | 20.13 | 165 | 124 | 179 | 232 | 229 | 147 | 179 | 24.35 | 2.6 | 4.66 x 10⁻⁰⁵ |
| 21 | ph 3- 10 | GPX3_MOUSE | 189 | 223 | 240 | 233 | 213 | 290 | 231 | 14.62 | 19 | 20 | 15 | 20 | 25 | 30 | 22 | 24.39 | 10.8 | 3.45 x 10⁻⁰⁸ |
| 39 | pH 4- 7 | PROP_MOUSE | 0 | 0 | 0 | 0 | 0 | 0 | 0 | 0 | 0 | 0 | 0 | 0 | 0 | 0 | 0 | 0 | 0 | 0 |
| 39 | pH 3- 10 | PROP_MOUSE | 284 | 311 | 304 | 248 | 240 | 240 | 271 | 12.02 | 228 | 156 | 182 | 139 | 217 | 187 | 185 | 18.42 | 1.5 | 1.16 x 10⁻⁰³ |
| 40 | pH 4- 7 | RETBP_MOUSE | 1244 | 1185 | 1534 | 1450 | 1225 | 1442 | 1347 | 10.83 | 429 | 466 | 555 | 716 | 521 | 546 | 539 | 18.44 | 2.5 | 5.52 x 10⁻⁰⁷ |
| 40 | pH 3- 10 | RETBP_MOUSE | 407 | 320 | 537 | 402 | 367 | 427 | 410 | 17.74 | 66 | 106 | 87 | 63 | 94 | 74 | 82 | 20.67 | 5.0 | 8.00 x 10⁻⁰⁷ |
| 41 | pH 4- 7 | SAMP_MOUSE | 638 | 1271 | 1350 | 724 | 523 | 1496 | 1000 | 41.86 | 0 | 0 | 0 | 0 | 0 | 0 | 0 | 0.00 | T | 1.61 x 10⁻⁰⁴ |
| 41 | pH 3- 10 | SAMP_MOUSE | 411 | 967 | 750 | 901 | 501 | 800 | 722 | 30.64 | 0 | 0 | 0 | 0 | 0 | 0 | 0 | 0.00 | T | 1.19 x 10⁻⁰⁵ |
| 44 | pH 4- 7 | TTHY_MOUSE | 9952 | 9049 | 9204 | 8992 | 9869 | 10721 | 9631 | 7.01 | 8045 | 7835 | 6190 | 9258 | 5998 | 5384 | 7118 | 20.91 | 1.4 | 3.68 x 10⁻⁰³ |
| 44 | pH 3- 10 | TTHY_MOUSE | 9569 | 10047 | 8934 | 8170 | 8330 | 9154 | 9034 | 7.95 | 6148 | 6432 | 6932 | 6738 | 6676 | 6095 | 6504 | 5.18 | 1.4 | 1.44 x 10⁻⁰⁵ |

**Table 3**

| | **Protein ID** | **Protein name** | **BAC** | **AAH** |
|---|---|---|---|---|
| **common** | A1AT6_MOUSE | Alpha-1-antitrypsin 1-6 | Yes | Yes |
| | A2MG_MOUSE | Alpha-2-macroglobulin (A2M) | Yes | Yes |
| | TTHY_MOUSE | Transthyretin | Yes | Yes |
| | PROP_MOUSE | Properdin | Yes | Yes * |
| **specific** | A1AG8_MOUSE | Orosomucoid-8 | Yes | No |
| | APOA1_MOUSE | Apolipoprotein A-I | Yes | No |
| | APOC3_MOUSE | Apolipoprotein C-III | Yes | No |
| | APOE_MOUSE | Apolipoprotein E | Yes | No |
| | APOH_MOUSE | Apolipoprotein H | Yes | No |
| | GPX3_MOUSE | Glutathione peroxidase 3 | Yes | No |
| | RETBD_MOUSE | Plasma retinol-binding protein | Yes | No |
| | SAMP_MOUSE | Serum amyloid P-component | Yes * | No |
| | MUP8_MOUSE | Major urinary proteins (MUP) | No | Yes |
| | VTBD_MOUSE | Vitamin D-binding protein | No | Yes |
| | S6A11_MOUSE | Sodium- and chloride-dependent GABA transporter 4 | No | Yes |
| | EGFR_MOUSE | Epidermal growth factor receptor (EGFR) | No | Yes * |

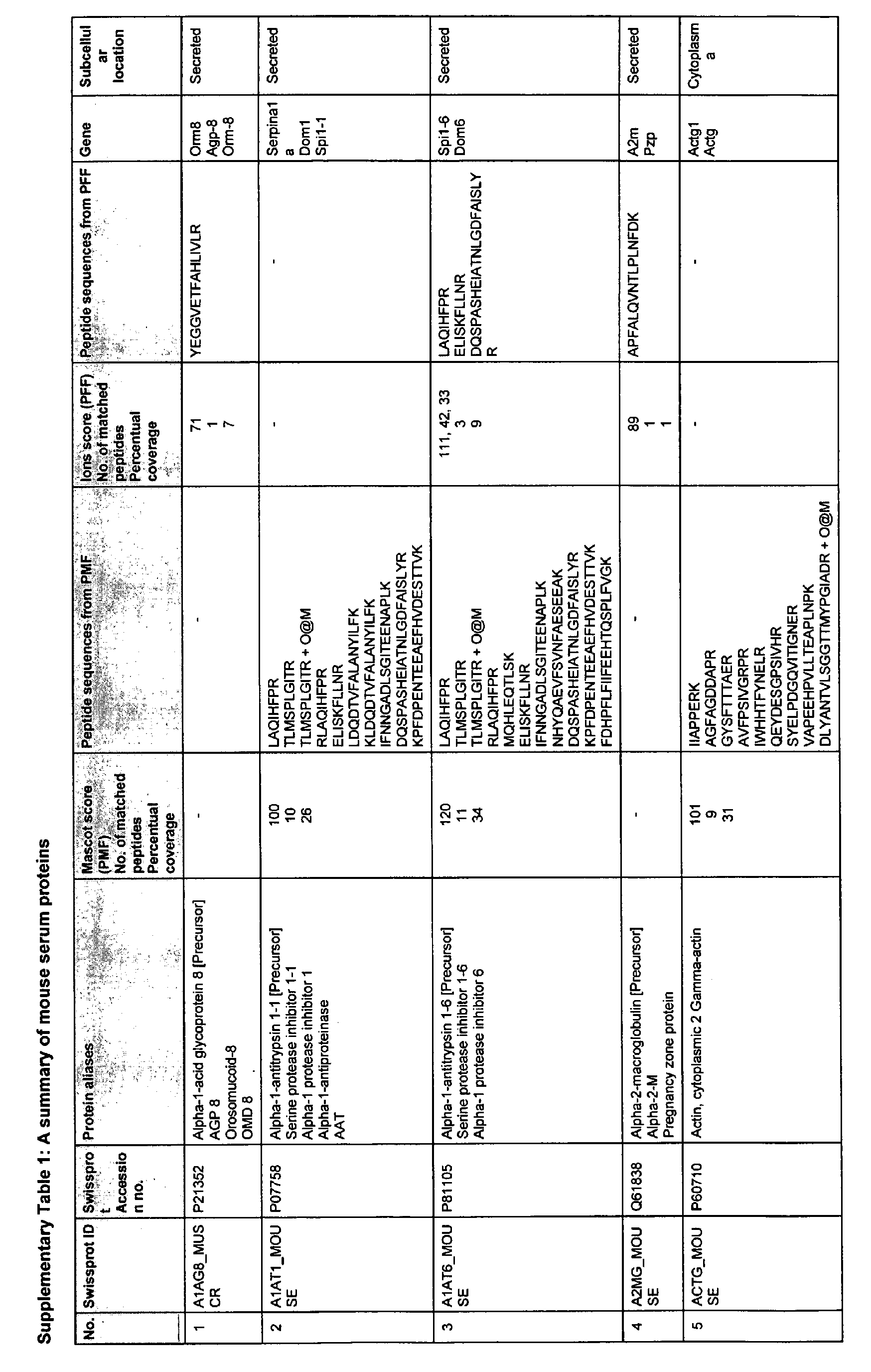

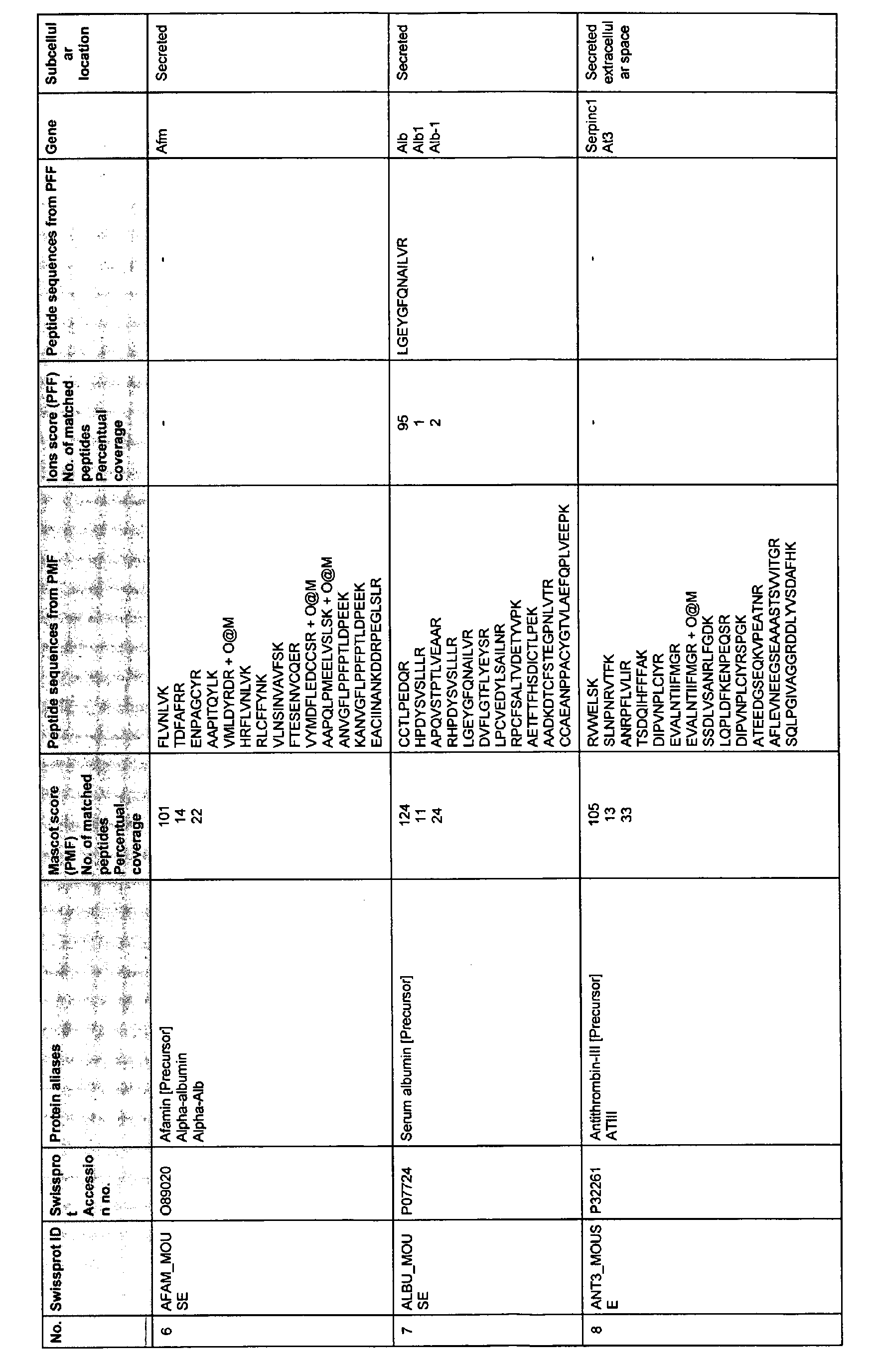

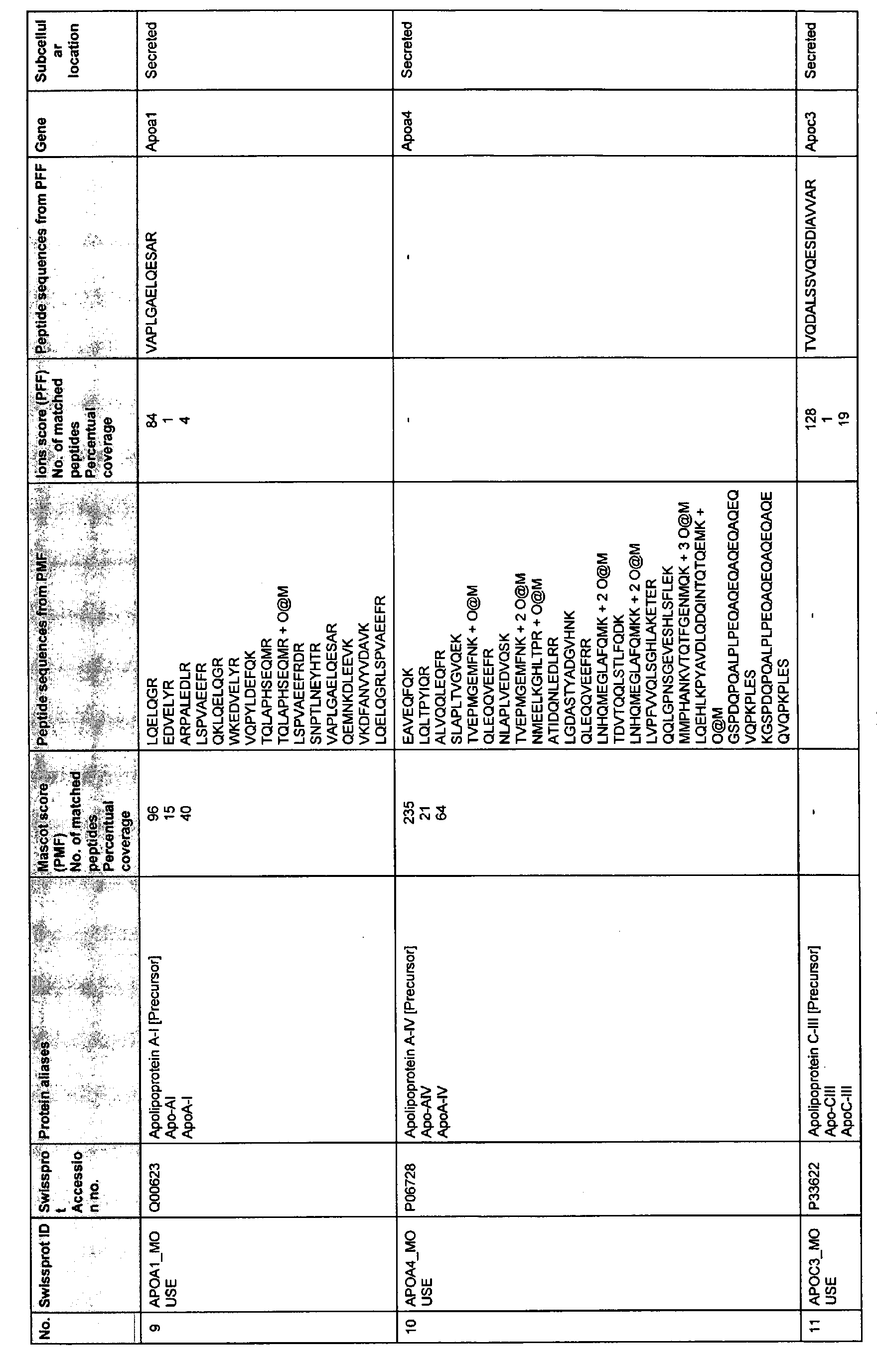

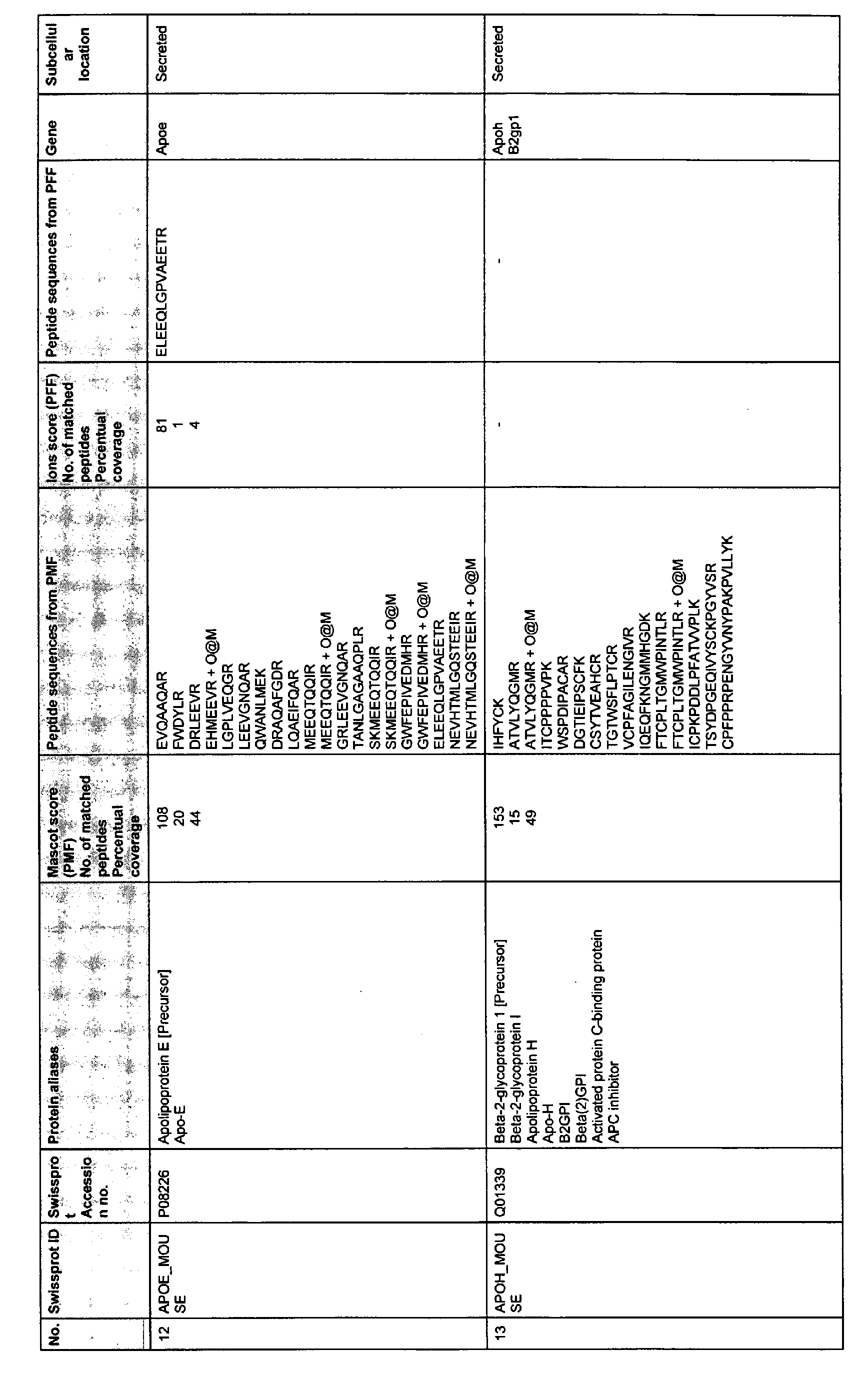

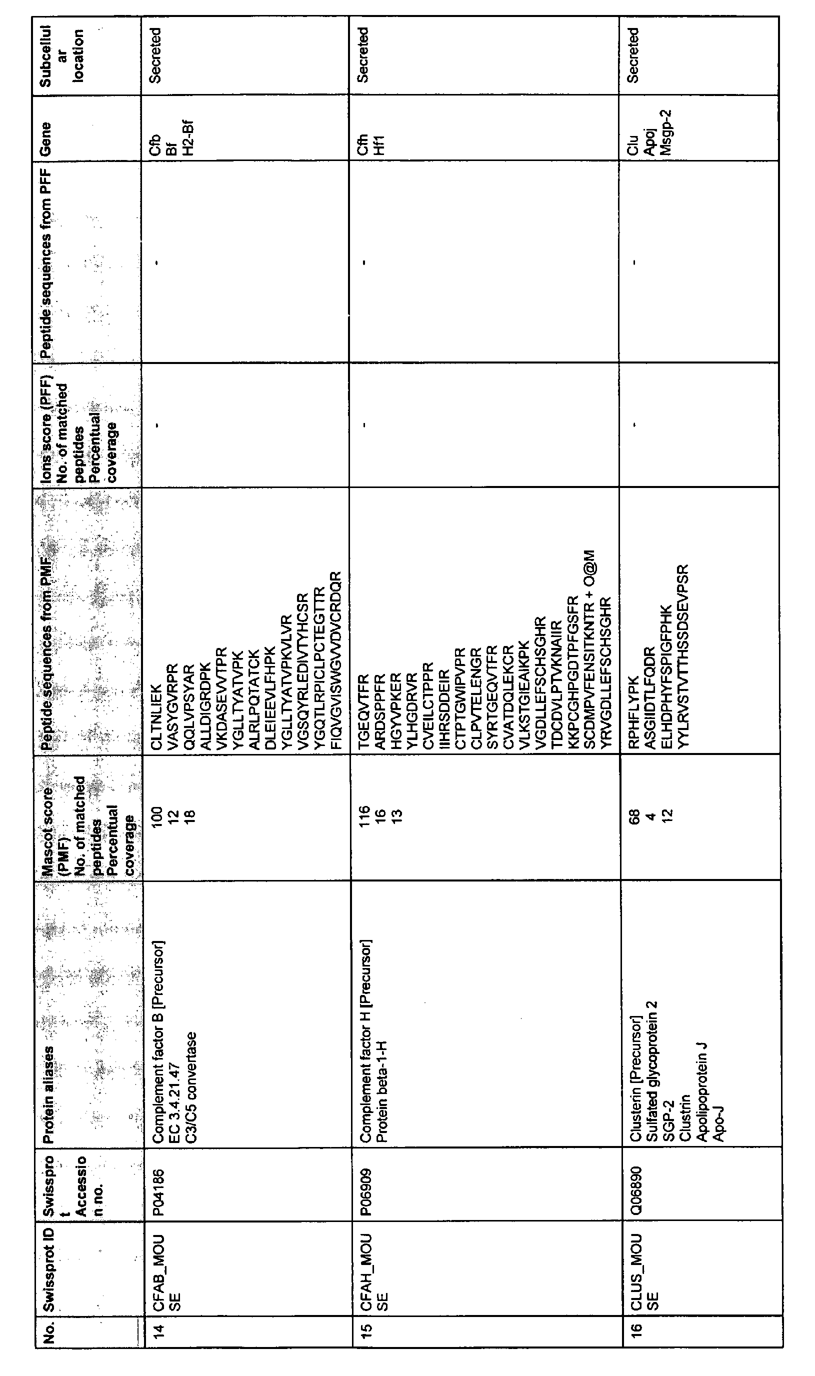

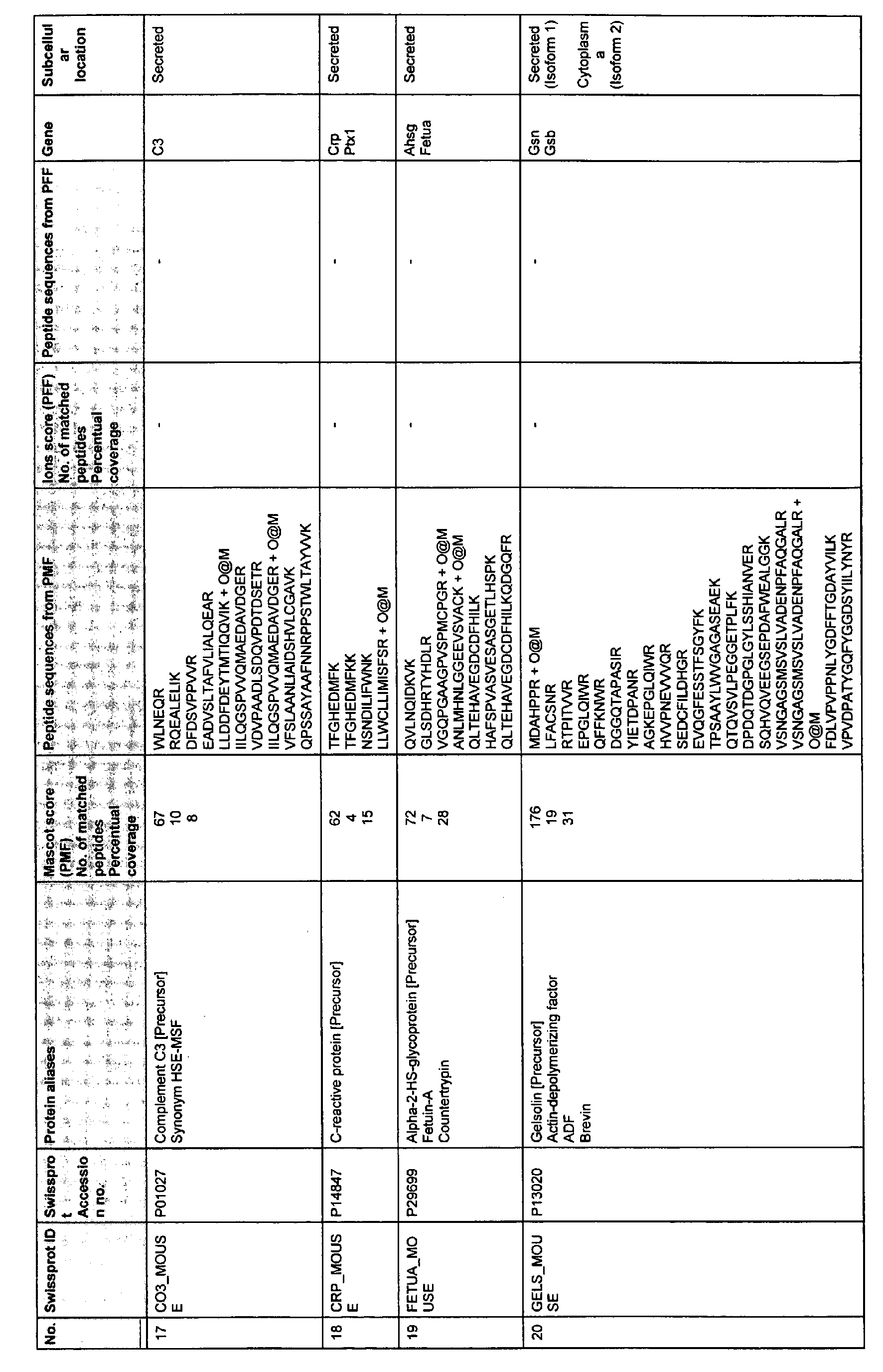

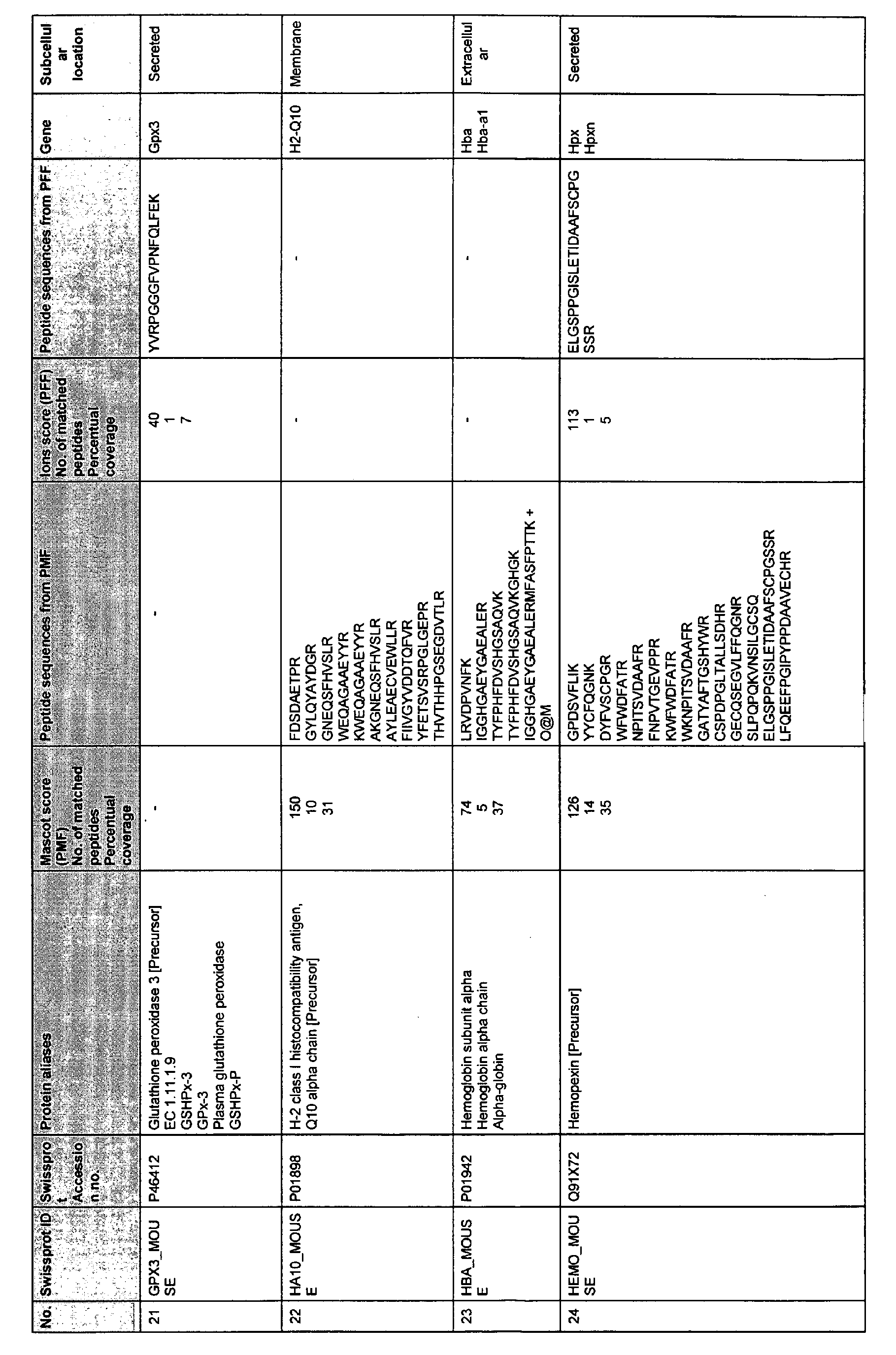

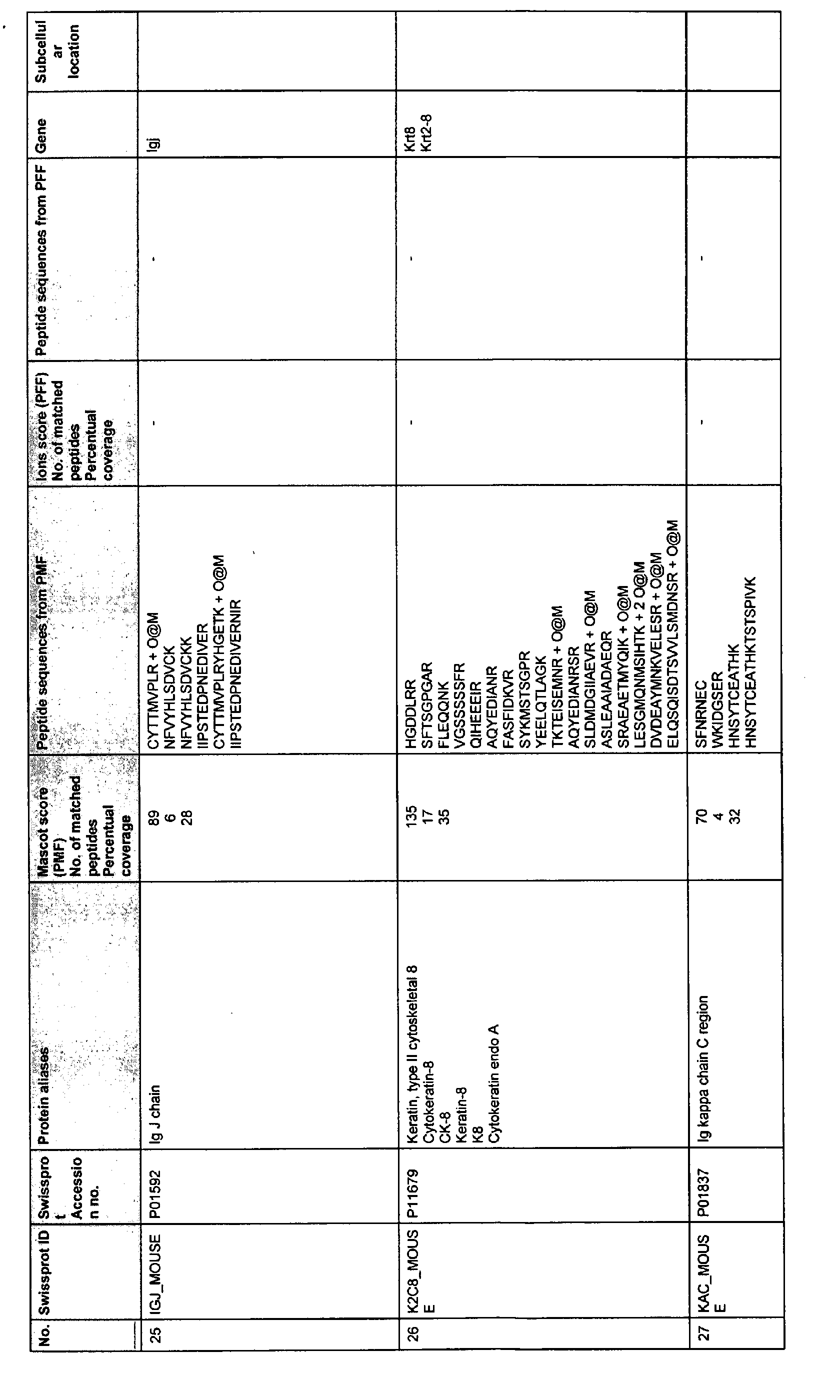

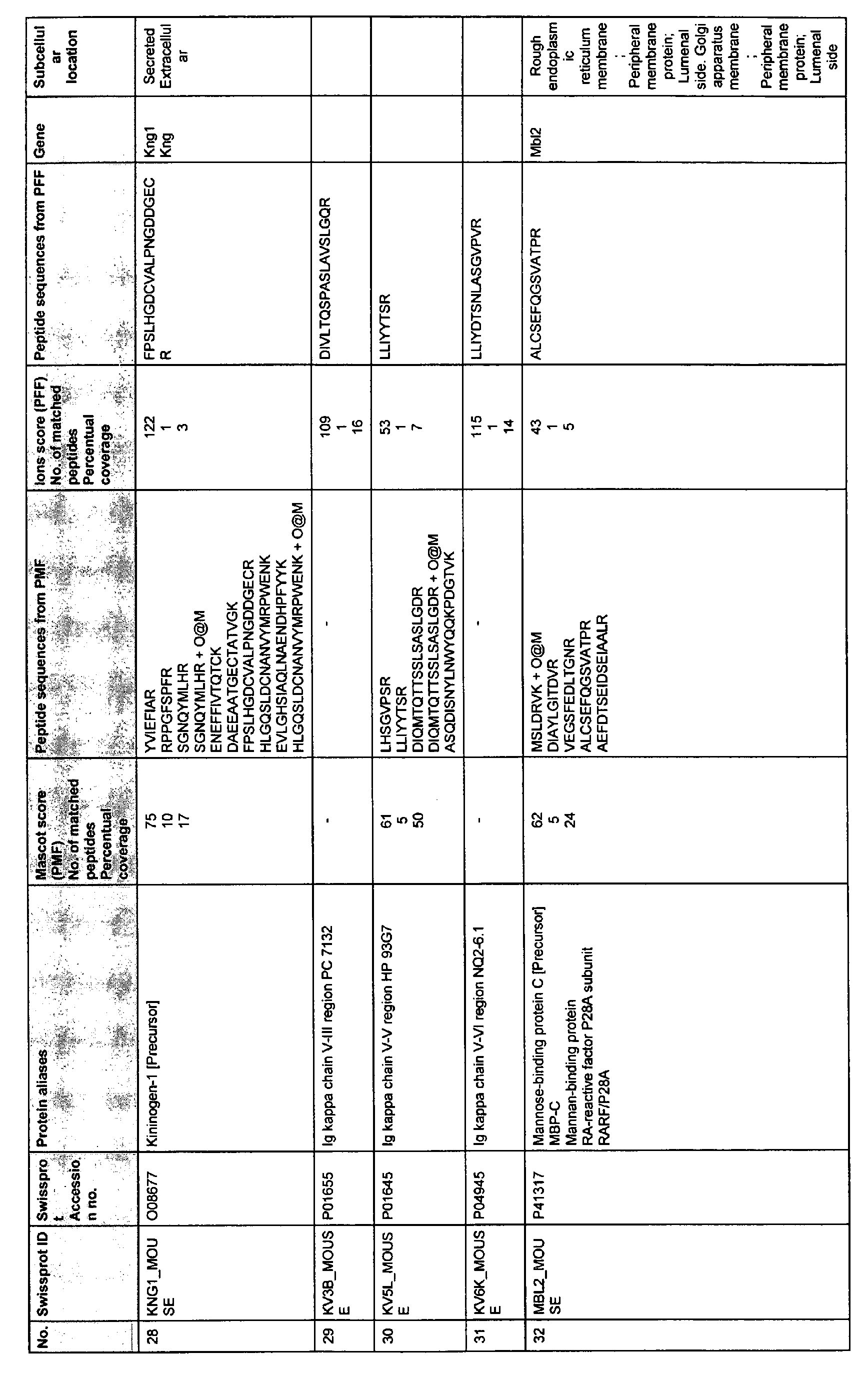

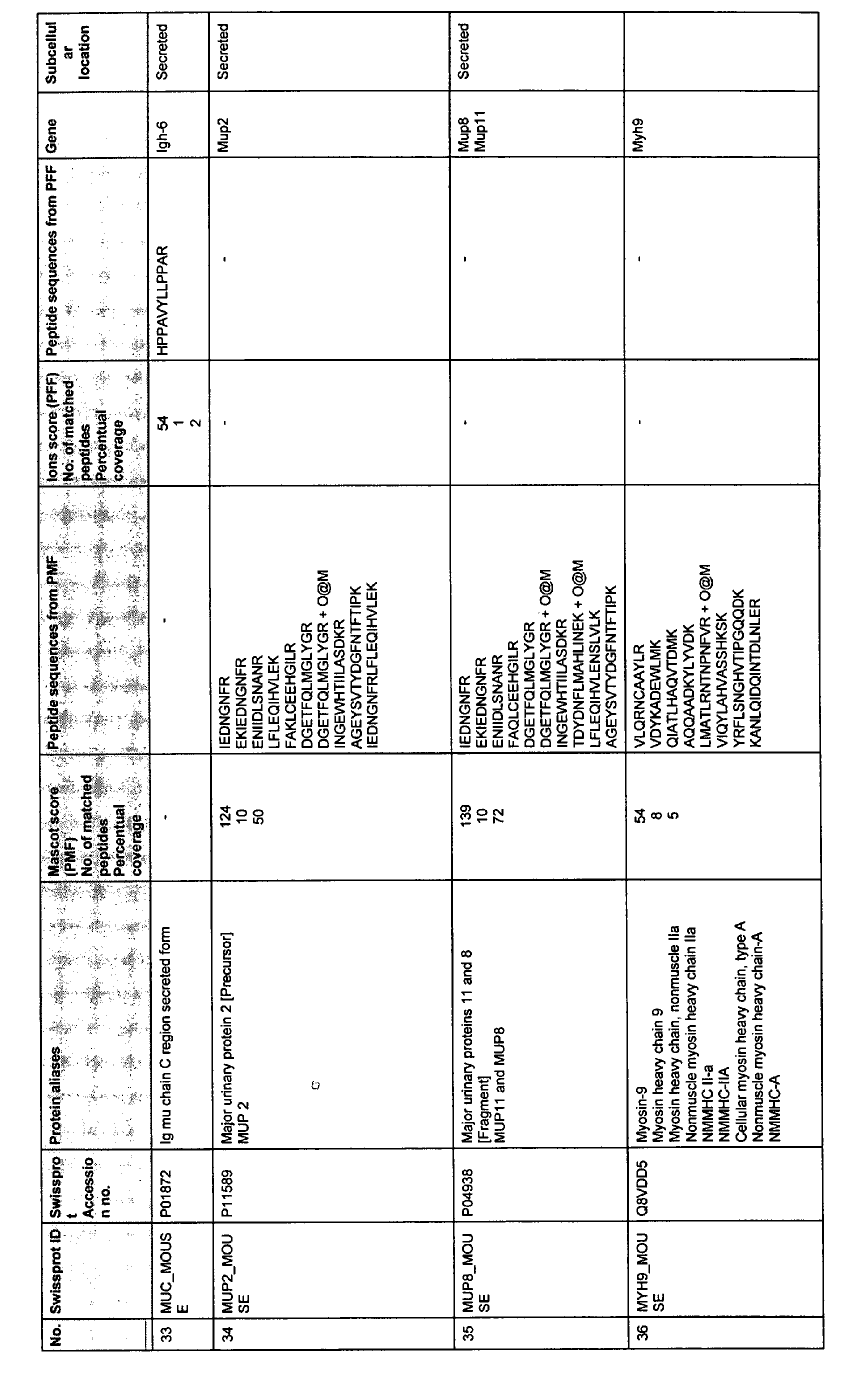

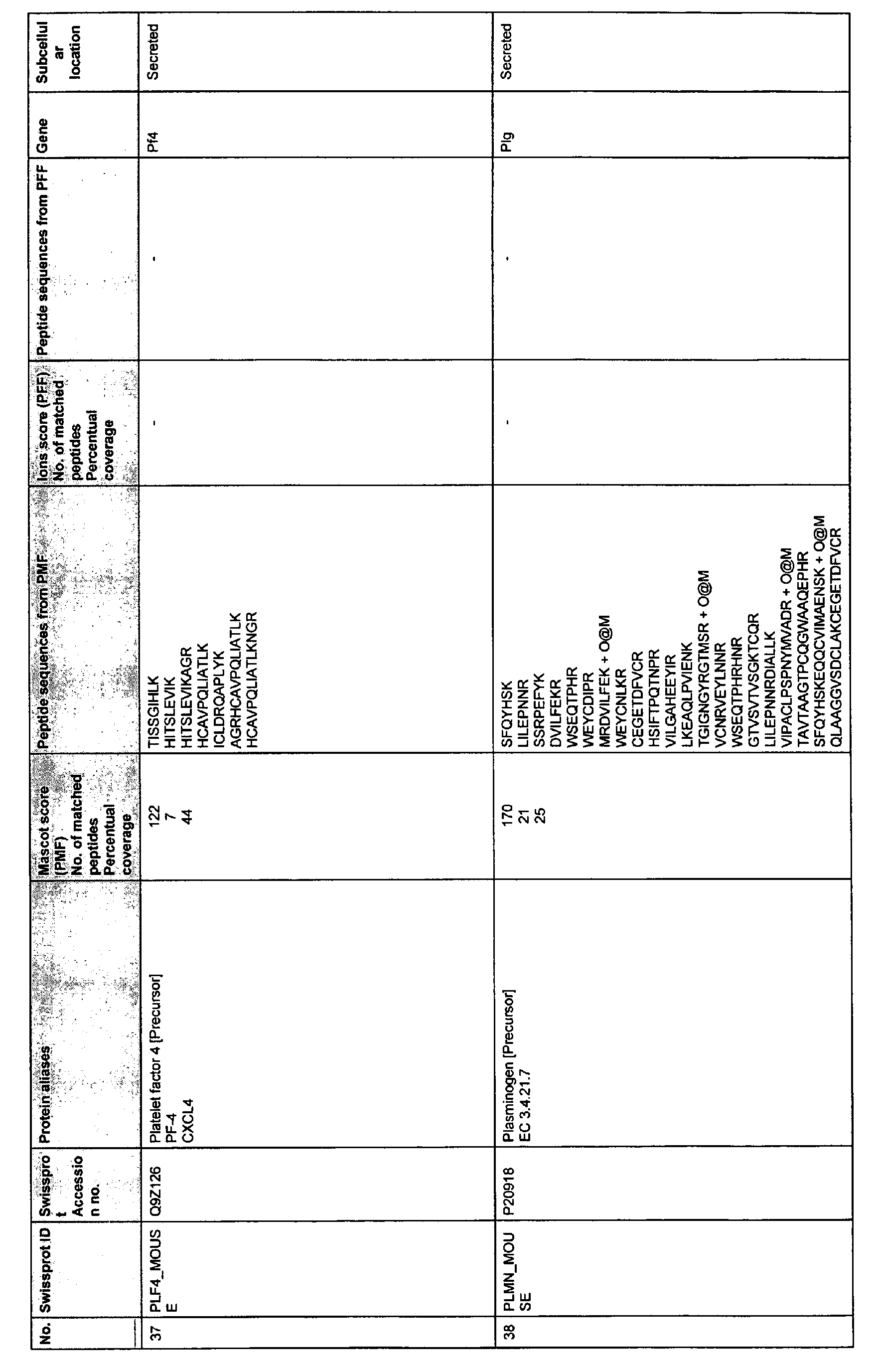

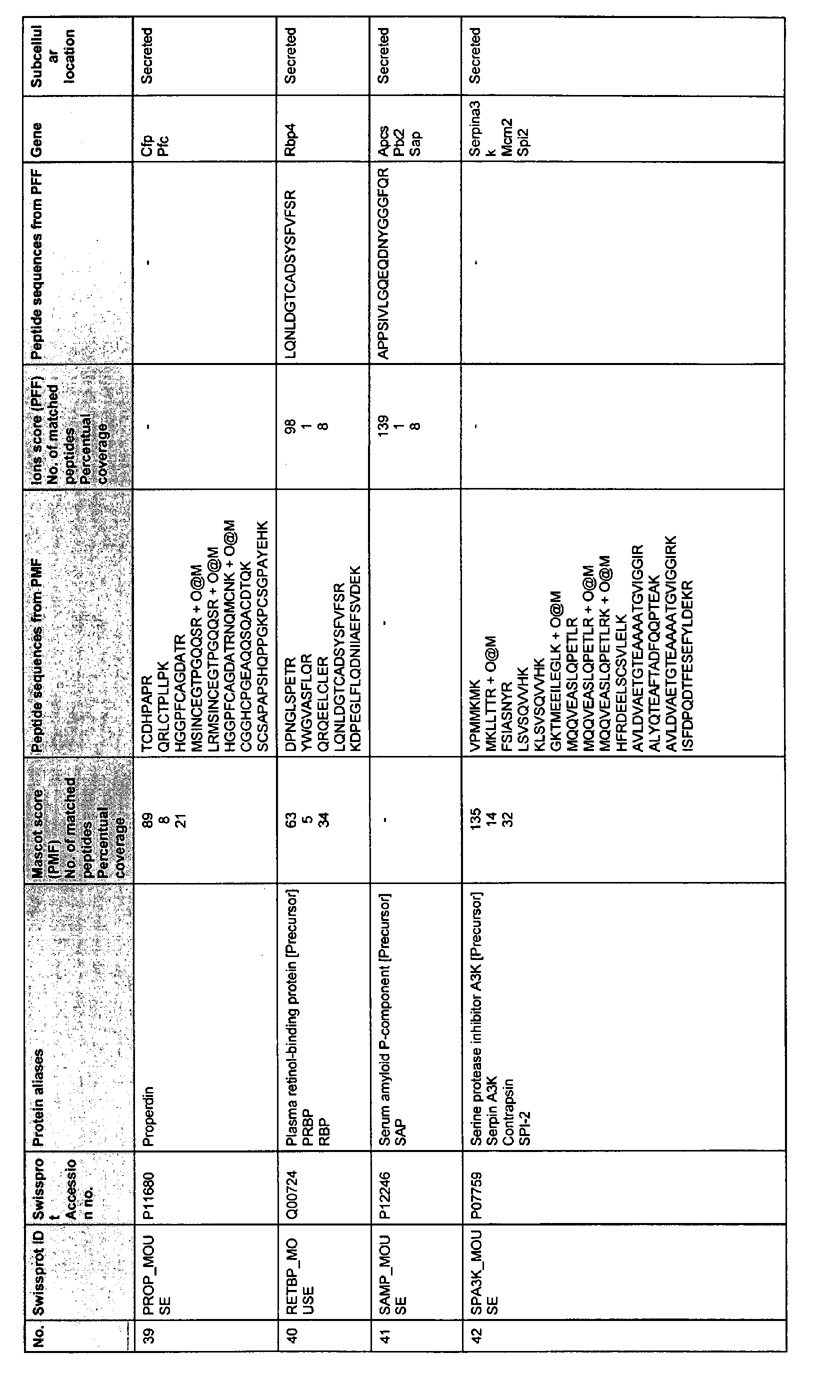

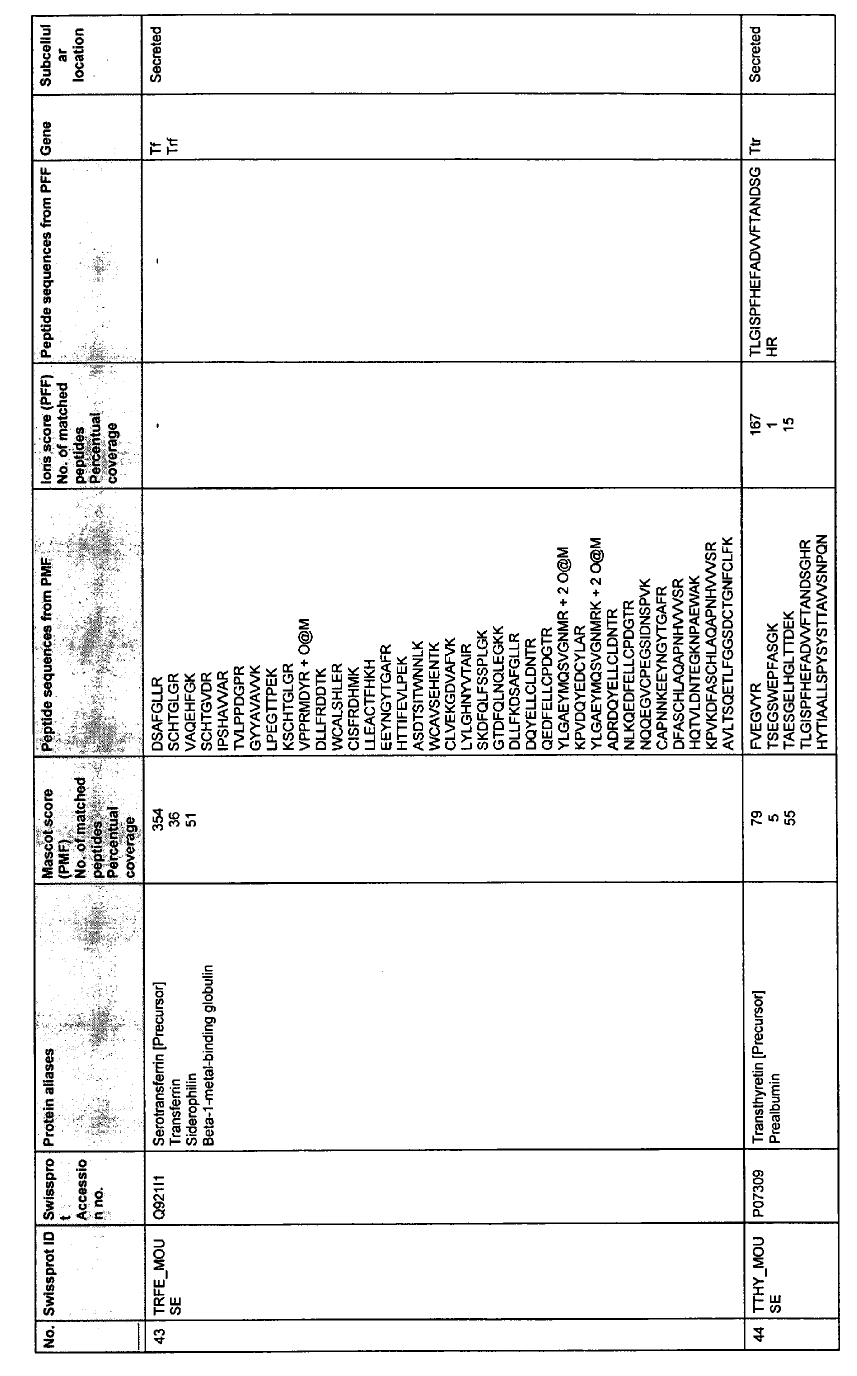

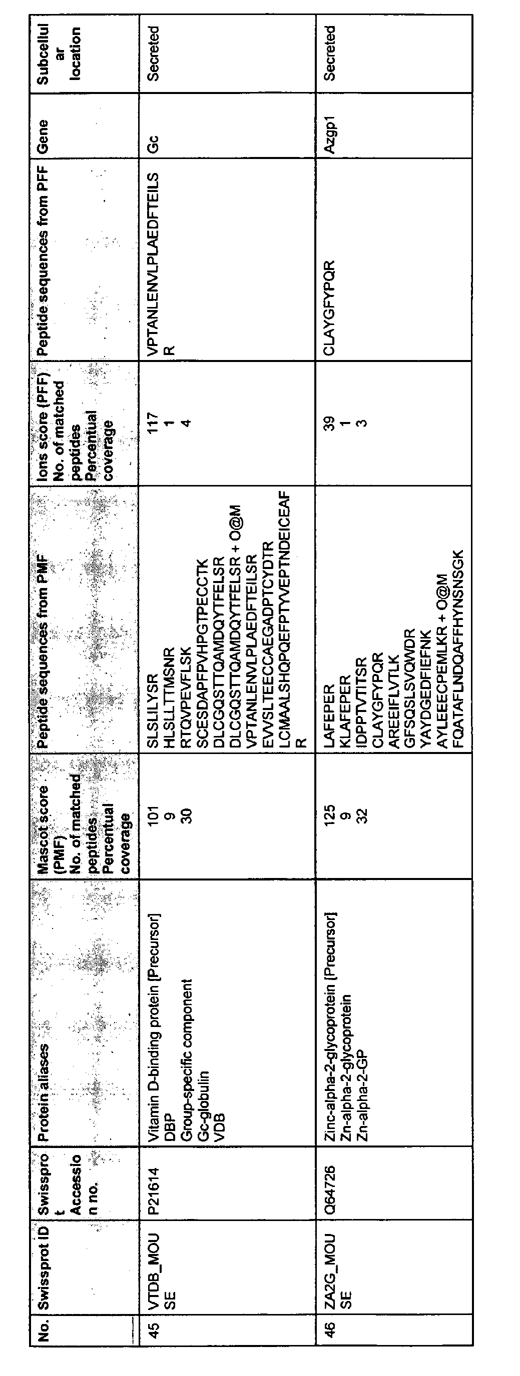

**Supplementary Table 2: Investigating the mouse serum proteome**

| **Swiss-Prot ID** | **Swiss-Prot Accession no.** | **Protein aliases** | **Chatterji B, Borlak J. A** 2-DE MALDI-TOF study to identify disease regulated serum proteins in lung cancer o c-myc transgenic mice. Submitted 2007, **Total: 46** | **Chatterji B, Borlak J. A** Serum proteomics of lung adenocarcinomas induced by targeted overexpression of c-raf in aleveolar epithelim identifies candidate biomakers. Proteomics. 2007 7,3960-91. **Total: 45** | **Wait R *et al*** Reference maps of mouse acute-phase proteins: changes with LPS-induced inflammation and A-I and A-II ransgenes Proteomics. 2005 **Total: 30** | **Duan X *et al*** mouse serum two-dimensional gel map; application to profiling burn injury and infection. Electrophoresis.2004, 25, 3055-65. **Total: 38** |
|---|---|---|---|---|---|---|
| A1AG1_MOUSE | Q60590 | Alpha-1-acid glycoprotein 1 [Precursor] AGP 1 Orosomucoid-1 OMD 1 | | X | X | X |
| A1AG8_MUSCR | P21352 | Alpha-1-acid glycoprotein 8 [Precursor] AGP 8 Orosomucoid-8 OMD 8 | X | | | |
| A1AT1_MOUSE | P07758 | Alpha-1-antitrypsin 1-1 [Precursor] Serine protease inhibitor 1-1 Alpha-1 protease inhibitor 1 Alpha-1-antiproteinase AAT | X | | X | X |
| A1AT2_MOUSE | P22599 | Alpha-1-antitrypsin 1-2 [Precursor] Serine protease inhibitor 1-2 Alpha-1 protease inhibitor 2 Alpha-1-antiproteinase AAT | | X | X | X |
| A1AT3_MOUSE | Q00896 | Alpha-1-antitrypsin 1-3 [Precursor] Serine protease inhibitor 1-3 Alpha-1 protease inhibitor 3 | | X | X | X |
| A1AT4_MOUSE | Q00897 | Alpha-1-antitrypsin 1-4 [Precursor] Serine protease inhibitor 1-4 Alpha-1 protease inhibitor 4 | | X | | |
| A1AT6_MOUSE | P81105 | Alpha-1-antitrypsin 1-6 [Precursor] Serine protease inhibitor 1-6 Alpha-1 protease inhibitor 6 | X | X | | X |
| A2AP_MOUSE | Q61247 | Alpha-2-antiplasmin [Precursor] Alpha-2-plasmin inhibitor Alpha-2-PI Alpha-2-AP | | X | | |
| A2MG_MOUSE | Q61838 | Alpha-2-macroglobulin [Precursor] Alpha-2-M Pregnancy zone protein | | X | | X |
| ACTG_MOUSE | P60710 | Actin, cytoplasmic 2 Gamma-actin | X | X | | |
| AFAM_MOUSE | 089020 | Afamin [Precursor] Alpha-albumin Alpha-Alb | X | X | X | |
| ALBU_MOUSE | P07724 | Serum albumin [Precursor] | X | X | X | X |
| ANT3_MOUSE | P32261 | Antithrombin-III [Precursor] ATIII | X | | | |
| APOA1_MOUSE | Q00623 | Apolipoprotein A-I [Precursor] Apo-AI ApoA-1 | X | X | X | X |
| APOA2_MOUSE | P09813 | Apolipoprotein A-II [Precursor] Apo-AII ApoA-II | | | X | X |
| APOA4_MOUSE | P06728 | Apolipoprotein A-IV [Precursor] Apo-AIV ApoA-IV | X | X | X | X |
| APOC3_MOUSE | P33622 | Apolipoprotein C-III [Precursor] Apo-CIII ApoC-III | X | | X | X |
| APOE_MOUSE | P08226 | Apolipoprotein E [Precursor] Apo-E | X | X | X | X |
| APOH_MOUSE | Q01339 | Beta-2-glycoprotein 1 [Precursor] Beta-2-glycoprotein I Apolipoprotein H Apo-H B2GPI Beta(2)GPI Activated protein C-binding protein APC inhibitor | X | X | | X |
| CFAB_MOUSE | P04186 | Complement factor B [Precursor] EC 3.4.21.47 C3/C5 convertase | X | X | | |
| CFAH_MOUSE | P06909 | Complement factor H [Precursor] Protein beta-1-H | X | | | X |
| CLUS_MOUSE | Q06890 | Clusterin [Precursor] Sulfated glycoprotein 2 SGP-2 Clustrin Apolipoprotein J Apo-J | X | X | X | X |
| CO3_MOUSE | P01027 | Complement C3 [Precursor] Synonym HSE-MSF | X | | | |
| CPN2_MOUSE | Q9DBB9 | Carboxypeptidase N subunit 2 [Precursor] Carboxypeptidase N polypeptide 2 Carboxypeptidase N 83 kDa chain Carboxypeptidase N regulatory subunit Carboxypeptidase N large subunit | | X | | |
| CRP_MOUSE | P14847 | C-reactive protein [Precursor] | X | | | |
| EGFR_MOUSE | Q01279 | Epidermal growth factor receptor | | X | | X |
| ESTN_MOUSE | P23953 | Liver carboxylesterase N [Precursor] EC 3.1.1.1 PES-N Lung surfactant convertase | | X | X | |
| FCN1_MOUSE | 070165 | Ficolin-1 [Precursor] Ficolin-A Ficolin-alpha M-ficolin Collagen/fibrinogen domain containing protein 1 | | | | X |
| FETUA_MOUSE | P29699 | Alpha-2-HS-glycoprotein [Precursor] Fetuin-A Countertrypin | X | X | X | X |
| FETUB_MOUSE | Q9QXC1 | Fetuin-B IRL685 | | X | X | X |
| GCB_MOUSE | P01866 | Ig gamma-2B chain C region secreted form | | X | | |
| GCBM_MOUSE | P01867 | Ig gamma-2B chain C region, membrane-bound form | | X | | |
| GELS_MOUSE | P13020 | Gelsolin [Precursor] Actin-depolymerizing factor ADF Brevin | X | X | | X |
| GPX3_MOUSE | P46412 | Glutathione peroxidase 3 [Precursor] EC 1.11.1.9 GSHPx-3 GPx-3 Plasma glutathione peroxidase GSHPx-P | X | X | | |
| HA10_MOUSE | P01898 | H-2 class I histocompatibility antigen, Q10 alpha chain [Precursor] | X | X | | |
| HBA_MOUSE | P01942 | Hemoglobin subunit alpha Hemoglobin alpha chain Alpha-globin | X | X | | |
| HEMO_MOUSE | Q91X72 | Hemopexin [Precursor] | X | X | X | X |
| HPT_MOUSE | Q62558 | Haptoglobin | | | X | X |
| HV51_MOUSE | P06330 | Ig heavy chain V region AC38 205.12 | | X | | |
| IGHG1_MOUSE | P01868 | Ig gamma-1 chain C region secreted form | | X | | |
| IGJ_MOUSE | P01592 | Ig J chain | X | X | | |
| K2C8_MOUSE | P11679 | Keratin, type II cytoskeletal 8 Cytokeratin-8 CK-8 Keratin-8 K8 Cytokeratin endo A | X | | | |
| KAC_MOUSE | P01837 | Ig kappa chain C region | X | X | | |
| KBP_MOUSE | P29621 | Serine protease inhibitor A3C [Precursor] Serpin A3C Kallikrein-binding protein KBP | | | X | |
| KNG1_MOUSE | 008677 | Kininogen-1 [Precursor] | X | X | X | X |
| KV3C_MOUSE | P01656 | Ig kappa chain V-III region MOPC 70 | | X | | |
| KV3D_MOUSE | P03977 | Ig kappa chain V-III region 50S10.1 | | X | | |
| KV5K_MOUSE | P01644 | Ig kappa chain V-V region HP R16.7 | | X | | |
| KV5L_MOUSE | P01645 | Ig kappa chain V-V region HP 93G7 | X | X | | |
| KV6K_MOUSE | P04945 | Ig kappa chain V-VI region NQ2-6.1 | X | | | |
| LIFR_MOUSE | P42703 | Leukemia inhibitory factor receptor [Precursor] LIF receptor LIF-R D-factor/LIF receptor CD118 antigen | | | | X |
| MBL1_MOUSE | P39039 | Mannose-binding protein A [Precursor] MBP-A Mannan-binding protein Ra-reactive factor polysaccharide-binding component p28B polypeptide RaRF p28B | | X | | |
| MBL2_MOUSE | P41317 | Mannose-binding protein C [Precursor] MBP-C Mannan-binding protein RA-reactive factor P28A subunit RARF/P28A | X | | | X |
| MUC_MOUSE | P01872 | Ig mu chain C region secreted form | X | X | | |
| MUP1_MOUSE | P11588 | Major urinary protein 1 [Precursor] MUP 1 | | | X | |
| MUP2_MOUSE | P11589 | Major urinary protein 2 [Precursor] MUP 2 | X | | X | X |
| MUP4_MOUSE | P11590 | Major urinary protein 4 [Precursor] MUP 4 | | | X | |
| MUP6_MOUSE | P02762 | Major urinary protein 6 [Precursor] MUP 6 Alpha-2U-globulin Group 1, BS6 Allergen Mus m 1 | | | X | |
| MUP8_MOUSE | P04938 | Major urinary proteins 11 and 8 [Fragment] MUP11 and MUP8 | X | X | X | |
| MYH9_MOUSE | Q8VDD5 | Myosin-9 Myosin heavy chain 9 Myosin heavy chain, nonmuscle IIa Nonmuscle myosin heavy chain IIa NMMHC II-a NMMHC-IIA Cellular myosin heavy chain, type A Nonmuscle myosin heavy chain-A NMMHC-A | X | | | |
| PLF4_MOUSE | Q9Z126 | Platelet factor 4 [Precursor] PF-4 CXCL4 | X | | | |
| PLMN_MOUSE | P20918 | Plasminogen [Precursor] EC 3.4.21.7 | X | X | | X |
| PROP_MOUSE | P11680 | Properdin | X | X | | |
| Q91XL1_MOUSE | Q91XL1 | Leucine-rich alpha-2-glycoprotein 1 Leucine-rich HEV glycoprotein | | | X | |
| RETBP_MOUSE | Q00724 | Plasma retinol-binding protein [Precursor] PRBP RBP | X | X | | X |
| S6A11_MOUSE | P31650 | Sodium- and chloride dependent GABA transporter 4 GAT4 | | X | | |
| SAA1_MOUSE | P05366 | Serum amyloid A-1 protein [Precursor] | | | | X |
| SAA2_MOUSE | P05367 | Serum amyloid A-2 protein [Precursor] | | | | X |
| SAA4_MOUSE | P31532 | Serum amyloid A-4 protein [Precursor] Amyloid A-5 protein | | X | | |
| SAMP_MOUSE | P12246 | Serum amyloid P component [Precursor] SAP | X | | X | X |
| SPA3K_MOUSE | P07759 | Serine protease inhibitor A3K [Precursor] Serpin A3K Contrapsin SPI-2 | X | | X | X |
| TRFE_MOUSE | Q921I1 | Serotransferrin [Precursor] Transferrin Siderophilin Beta-1-metal-binding globulin | X | X | X | X |
| TTHY_MOUSE | P07309 | Transthyretin [Precursor] Prealbumin | X | X | X | X |
| THRB_MOUSE | P19221 | Prothrombin [Precursor] EC 3.4.21.5 Coagulation factor II | | | | X |
| VTDB_MOUSE | P21614 | Vitamin D-binding protein [Precursor] DBP Group-specific component Gc-globulin VDB | X | X | X | X |
| VTNC_MOUSE | P29788 | Vitronectin [Precursor] Serum-spreading factor S-protein | | X | | |
| ZA2G_MOUSE | Q64726 | Zinc-alpha-2-glycoprotein [Precursor] Zn-alpha-2-glycoprotein Zn-alpha-2-GP | X | | | X |
| ZA2G_MOUSE | Q64726 | Zinc-alpha-2-glycoprotein [Precursor] Zn-alpha-2-glycoprotein Zn-alpha-2-GP | | X | | |

We previously reported targeted overexpression of c-myc to alveolar epithelium to cause lung cancer. We now extended our studies to the serum proteome of tumor bearing mice. Proteins were extracted with a thiourea-containing lysis buffer and separated by 2-DE at pH 4-7 and 3-10 followed by MALDI-TOF/TOF analysis. Fourty six proteins were identified in tumor bearing mice of which n=9 were statistically significant. This included disease regulated expression of orosomucoid-8, alpha-2-macroglobulin, apolipoprotein-A1, apolipoprotein-C3, glutathione peroxidase-3, plasma retinol-binding protein and transthyretin, while expression of apolipoprotein-E was decreased in late stages of disease. Moreover, serum amyloid P component was uniquely expressed at late stages of cancer. It is of considerable importance that most disease regulated proteins carried the E-Box sequence (CACGTG) in the promoter of the coding gene, therefore providing evidence for their regulation by c-myc. Notably, expression of alpha-2-macroglobulin, transthyretin, alpha-1-antitrypsin and properdin was in common in different lung tumor models, but regulation of orosomucoid-8, apolipoprotein-A1, apolipoprotein-C3, apolipoprotein-E, glutathione peroxidase-3, plasma retinol-binding protein and serum amyloid P component was unique when the serum proteome of c-myc and c-raf tumor bearing mice were compared. Therefore, candidate biomarkers for atypical adenomatous hyperplasias (AAH) and bronchiolo-alveolar (BAC)/papillary adenocarcinomas (PLAC) can be proposed.

### 1

Lung cancer remains the leading cause of cancer death worldwide. In 2007, approximately 160.000 people died from lung cancer in the United States alone (American Cancer Society, 2007). Smoking is considered the primary cause of lung cancer and accounts for > 80% of all diagnosed cases [1]. In general, lung tumors are classified as small cell (SCLC) or non-small cell lung carcinomas (NSCLC). NSCLC are further divided into adenocarcinoma, large and squamous cell carcinoma. Subclasses of adenocarcinomas may be divided further in Clara and alveolar epithelial cancers [2]. Indeed, a recent study suggests alveolar epithelial carcinomas to be on the rise and may account for up to one third of all adenocarcinomas [3].
Despite of the fact that many cancers are considered to be of sporadic origin, many tumors display altered c-myc activity. In fact, various studies demonstrated altered c-myc activity in small cell and non-small cell lung cancers [4-12]. Here we report our efforts to identify serum biomarkers of disease in a c-myc transgenic lung cancer mouse model as previously reported by us [13]. Specifically, c-myc encodes a 49 kDa nuclear phosphoprotein and is classified as a basic helix-loop-helix/leucine zipper-type (bHLH/LZ) transcription factor. C-myc progresses cell cycle regulation [14] and forms a heterodimeric protein complex with max, e.g. another 18 kDa bHLH/LZ protein. The c-myc/max complex then recognizes the consensus sequence 5'-CACGTG-3', also known as an E-box motif [15]. This motif is located in promoter sequences of many genes targeted by c-myc. Genome wide scanning by DNA microarray, SAGE and chromatin immunoprecipitation (CHIP) provide evidence for more than 1.000 c-myc target genes. Through its numerous direct and indirect targeted genes, the c-myc oncoprotein is linked to many cellular processes including signal transduction, DNA synthesis and repair, apoptosis, cell adhesion, cytoskeleton dynamics and regulation of ion channels [16]. As detailed above, expression and activity of c-myc is increased in many human malignancies due to undue amplification of the reference allel or hyperactivity of the mutated protein [17]. Nonetheless, the precise molecular mechanism by which c-myc influences cell growth and division in normal and tumorous cells is still elusive. In fact, in the United States alone more than 70.000 cancer deaths per year are ascribed to altered c-myc abnormalities [18].
In the past we reported targeted overexpression of c-myc to alveolar epithelium to result in bronchiolo-alveolar carcinomas (BAC) and papillary adenocarcinomas (PLAC) [13, 19]. As of today, disease regulated serum proteins of c-myc-induced lung adenocarcinomas are unknown. We therefore applied 2-DE and MALDI MS to identify candidate biomarkers at early and late stages of disease. We further compared our findings with results from a recently published serum proteome study with c-raf transgenic mice, which developed lung cancer as well [20]. Overall, this study aimed at identifying candidate disease biomarkers for the detection of lung cancer.

### 2 Materials and methods

### 2.1 Histopathology

Transgenic lung tumor bearing mice were bred and kept in the C57BU6 background by Dr. Roman Halter. SP-C/c-myc transgenic mice were kept in the C57BU6 background for at least five generations. No difference in phenotype and morphology of the lung was observed by the different genetic background of wild-type strains. A detailed description of the SP-C/c-myc transgenic line is given in [13]. Here we refer to C57BU6 non-transgenic littermates as controls. Fig. 1a depicts the SP-C/c-myc gene construct while Fig. 1b (B1, B2) displays typical features of the lung tumors. Tissues were fixed in 4% buffered formaldehyde in PBS for approximately 20 h, dehydrated and embedded in paraffin (Roti-Plast ^{™}, Roth). Tissue captions were stained with hematoxylin and eosin according to standard protocols. The mouse tumors were classified according to the IARC - WHO system of classification (2004) by the board certified pathologist Dr. Reinhard Spanel (Leipzig, Germany).

### 2.2 Sample preparation

Blood serum of healthy aged matched controls (n=12) and of lung tumor bearing SP-C/c-myc mice, aged 3 (n=6) and 14 months (n=6) were studied. Blood was withdrawn from the *vena cava*. After clotting for 2 h at room temperature, the blood was centrifuged at 3500 rpm for 15 min. The resultant supernatants were removed, frozen immediately in liquid nitrogen and stored at - 80°C until further analysis. The protein concentration in serum was determined by the Bradford protein assay.

### 2.3 Two-dimensional gel-electrophoresis

Each sample was analyzed in triplicate. Serum proteins were separated by isoelectric focusing (IEF) with precast IPG strips (pH 3-10, non-linear gradient and pH 4-7, linear gradient; both 170x3x0.5 mm, BioRad). 800 µg was diluted in a lysis buffer containing 2 mol/L thiourea, 5 mol/L urea, 40 mmol/L Tris, 4% CHAPS, 0.5% BioLyte 3-10 (BioRad), 100 mmol/L DTT [21, 22] resulting in a total volume of 350 µL per strip. Focused IPG strips were rehydrated at 50 V for 12 h. IEF was performed at 20°C with a maximum voltage of 10 kV and a maximum current of 50 µA per strip. After IEF, IPG strips were equilibrated in 10 mL reducing buffer (2% DTT in 10 mL equilibration buffer containing 6 mol/L urea, 30% glycerin, 2% SDS, 0.05 mol/L Tris-HCl, pH 8.8 and 0.5% bromphenol blue) for 15 min, followed by equilibration in 10 mL alkylation buffer (4% iodoacetamide and 0.5% bromphenol blue in 10 mL equilibration buffer) for 15 min [23]. SDS-PAGE was performed in a Protean-plus Dodeca ^{™} Cell (BioRad) using self-cast polyacrylamide gels (200 x 205 x 1.5 mm; 12% T). Gels were run in parallel in 0.025 mol/L Tris/ 0.192 mol/L glycine/ 0.1% SDS at 10°C with a constant voltage of 70 V overnight. Precision Plus Protein Unstained Standards ^{™} (BioRad) was used for calibration of Mᵣ and p/.

### 2.4 Staining and imaging

2-D Gels were fixed overnight in 500 mL 30% ethanol/ 2% phosphoric acid and washed three times for 20 min each in 500 mL 2% phosphoric acid. Equilibration was done with 500 mL 2% phosphoric acid/ 18% ethanol/ 15% ammonium sulfate thereafter. Colloidal Coomassie Brilliant Blue (CBB) staining of proteins was started by addition of 5 mL staining solution (2% CBB G250, Roth) to 500 mL of equilibration solution. Staining was carried out for 48 h and thereafter washed once with 500 mL water for 10 min.
Gels were scanned with the Perfection 4990 Photo ^{™} densitometer (Epson). Detection of spots, quantification and comparison of 2-D protein profiles was done with the PDQuest 8.0 software (BioRad). After removal of background and vertical streaks from each gel image, spots were digitized by Gaussian fit. To quantify protein spots, a matchset of all gels was made and the absorbance of individual protein spots from 2-D gels was measured. The raw quantity of each spot in a member gel was divided by the total intensity value of all the pixels in the image, for instance total density in gel image. This normalization procedure of the software assumed that the total density of an image, consisting of background and spot density will be relatively consistent from gel to gel [24].
The expression of serum proteins was analyzed by the Student's t-test. A probability of p<0.05 was considered statistically significant (Tab. 2a and Tab. 2b). Graphical evaluation was performed with the SigmaPlot software (SPSS) (Fig. 3b). Spots were excised and transferred to 96-well microtiter plates (ABgene) by the EXQuest ^{™} spot cutter (BioRad).

### 2.4 Mass spectrometry

### 2.4.1 In-gel digestion

Each of the CBB-stained gel plugs was washed twice with 15 µL ammonium hydrogencarbonate solution (100 mmol/L) and then dehydrated twice with 15 µL acetonitrile. Proteins were digested with a total of 160 µg trypsin (13 ng/µL, sequencing grade, Promega) per gel plug at 37°C for 4 h. Resulting peptides were extracted with 8 µL n-Octyl-β-D-glucopyranoside (5 mmol/L, Applichem)/1% trifluoroacetic acid in an ultrasonic bath (Sonorex, Super RK 514 BH, Bandelin) for 5 min.

### 2.4.2 MALDI-TOF/TOF

The HCCA matrix was prepared with the thin layer method. 1 µL of the peptide extracts were manually spotted onto a 600 µm/384 well AnchorChip ^{™} sample target (Bruker Daltonics) and dried at ambient temperature. Recristallization was performed with 1 µL of 60% ethanol/ 30% acetone/ 10% of 1% trifluoroacetic acid thereafter. MALDI mass spectra were recorded using an Ultraflex II TOF/TOF mass spectrometer (Bruker Daltonics) equipped with a 384-sample scout source. A peptide calibration standard (Bruker Daltonics) was used for external calibration. MS and MS/MS data were recorded automatically on the MALDI-TOF/TOF instrument using the three most abundant peptide signals of the corresponding peptide mass fingerprint (PMF) spectrum. Mass spectra were acquired in an automatic mode using the AutoXecute module of FlexControl 2.4 software (Bruker Daltonics). Spectra were analyzed using the FlexAnalysis 2.4 software (Bruker Daltonics). The Swiss-Prot database employing the Mascot 2.0 program (Matrix Science, in-house server) was used for the search of peptide masses to identify proteins [25]. Database searches were performed taking into account carbamidomethyl modification of cysteines and possible oxidation of methionine. One missed cleavage was allowed. A mass accuracy of ≤ 100 ppm was requested for PMF. For MS/MS searches, a mass accuracy of ≤ 70 ppm was allowed for peptide masses and their fragments, respectively. For further consideration, only those proteins were assumed to be identified that were annotated from corresponding spots in at least five gels. Identified proteins were sent to the Proteinscape ^{™} database (Protagen) [26] and checked individually for further consideration.

### 3 Results and Discussion

### 3.1 Separation of serum proteins by 2-DE

We used a thiourea-containing lysis buffer to extract proteins from serum.
Proteins were separated within pH ranges of 3-10 and 4-7, respectively. Proteins were visualized with the colloidal CBB (CCB) stain. Approximately 400 (pH 3-10) and 200 (pH 4-7) spots per gel were detected. Fig. 2a and Fig. 2b depict examples of serum proteome maps (pH 3-10 and 4-7) of c-myc tumor bearing mice at late stages of carcinogenesis (14 months).

### 3.2 Protein identification

One hundred and eight 2-D gels were stained with the CCB method and studied by image analysis. After excision of >12.500 protein spots tryptic digests were analyzed by MALDI-MS and -MS/MS. Identification was based on Swiss-Prot database entries with the Mascot search engine. In tumor bearing and healthy non-transgenic mice, 46 common serum proteins were identified (Tab. 1). In Supplementary Table 1 we list the Mascot score, sequence coverage and the most informative peptide sequences identified by MS and -MS/MS, respectively. To the best of our knowledge, three serum proteins are novel and have not been reported so far in serum or plasma proteome studies, respectively [27, 28]. These include orosomucoid-8 (spot no. 1), C-reactive protein (spot no. 18) and cytokeratin-8 (spot no. 26).

### 3.3 Regulation of serum proteins in lung cancer

Tab. 2a and 2b provide expression profiles of statistically significantly regulated proteins (p<0.05) in lung tumor serum proteomes. N=13 proteins were regulated at late stages of tumorigenesis (14 months), of which n=9 were regulated at early stages (3 months) as well. Fig. 3a depicts prominent examples of disease regulated proteins in tumor bearing mice, while Fig. 3b compares the fold-changes between early and late stages of tumorigenesis. Differentially expressed proteins will be discussed later for their proven or infered association with human diease. Notably, their regulation by c-myc was determined by comparison of database entries from http://www.myccancergene.org [29] and by searching for the DNA consensus sequence for the E-Box motif 5'-CACGTG-3' in the promoter regions of the coding gene. Overall, six regulated proteins emerged as direct c-myc targets (Tab. 3). These include orosomucoid-8 (spot no. 1), alpha-1-antitrypsin-1 (spot no. 2), alpha-1-antitrypsin-2 (spot no. 3), alpha-2-macroglobulin (spot no. 4), apolipoprotein E (spot no. 12) and glutathione peroxidase 3 (spot no. 21). Furthermore, we compared our results with our recently published study on the serum proteome of c-raf transgenic mice which developed lung tumors as well [20]. In the following the biological relevance of the regulated proteins is being described.

### 3.3.1 Regulation of acute phase proteins in lung cancer

In general, concentrations of various positive and negative regulators of acute phase proteins (APP) increase or decrease in response to inflammation [30]. As observed in serum of healthy and tumor bearing mice, eight acute phase proteins were regulated. We found the positive acute phase regulators, e.g. alpha-1 acid glycoprotein-8, also named orosomucoid-8 (spot no. 1), alpha-1 antitrypsin (spot no. 2 and spot no. 3), alpha-2 macroglobulin (spot no. 4-B) and serum amyloid P component (spot no. 41) to be increased or exclusively expressed in tumor bearing mice. In contrast, the negative acute phase proteins (n-APP) plasma retinol-binding protein (spot no. 40), transthyretin (spot no. 44) and serum albumin (spot no. 7) were either up- or downregulated (see Figures 3a, 3b and Table 2). In particular, alpha-2 macroglobulin (A2M, spot no. 4, 4-B, 4-C, 4-D) is mainly produced in the liver, but in the lung as well. With a molecular weight of 165 kDa, A2M represents a large plasma protein that consists of four identical subunits that are linked together by disulfide bonds. Specifically, A2M acts as a proteinase inhibitor and targets serine-, cysteine-, aspartic- and metalloproteinases. The A2M structure includes a "decoy" region where such proteinases are bound and cleaved. Macrophage receptors recognize and eliminate the A2M-proteinase complex. It is known that A2M is regulated in patients with nephrotic syndrome and Alzheimer's disease. Serum A2M levels may be useful for diagnosis and therapeutic monitoring in lung cancer and in bone metastases of prostate cancer as reported elsewhere [31]. Note, the A2M gene is a well known target of c-myc. Its responsiveness to c-myc was shown by Misra and coworkers [32]. We observed a significant (p<0.05), > 6-fold (pH 4-7) and 1.5-fold (pH 3-10) increase of alpha-2 macroglobulin levels in serum of tumor bearing mice, aged 14 months and a 2.7-fold change in tumor bearing mice, aged 3 months (spot no. 4-B). Furthermore, a serum amyloid P component (SAMP, spot no. 41), a member of the pentraxins that is produced in the liver, was marginally increased (p>0.05) at early stages, but exclusively expressed at late stages of tumorigenesis in transgenic mice. SAMP has a sequence homology of 51% with the C-reactive protein (spot no. 18), a well known plasma APP. The physiological role of SAMP is not always clear, but may play a role in amyloidosis [33]. Recently, Korbelik and co-workers demonstrated significant increase of SAMP levels in liver tumors [34]. Transthyretin, also named prealbumin (spot no. 44) is a common blood protein. It is a carrier for thyroid hormones from bloodstream to tissues. Transthyretin interacts with the retinol binding protein (RBP, spot no. 40), thus enabling retinol transportation. If transthyretin is not expressed, lower levels of retinol and RBP are observed, as shown in ovarian cancer [35, 36]. In particular, a truncated form of transthyretin was repressed in women with ovarian cancer [37]; this protein may serve as a potential biomarker [38]. Since a decrease of transthyretin levels in serum is linked to a negative acute phase regulation during inflammation as, its increased expression might serve as a biomarker for cancer. The transthyretin monomer may also be a blood marker to cerebrospinal fluid barrier disruption, as shown in cerebral metastasis [39], even though it is differentially expressed in tumors [40]. Notably, a recent proteomic study reported induction of transthyretin in human lung adenocarcinoma patients. We observed upregulation of transthyretin by >1.4-fold in serum of lung tumor bearing mice, alongside an overexpression of RBP at early and late stages of tumorigenesis by 2.5-fold (pH 4-7) and 5-fold (pH 3-10), respectively.

### 3.3.2 Regulation of apolipoproteins in lung cancer

Several apolipoproteins were regulated in tumor bearing mice. Notably, apolipoproteins function primarily as lipid-binding proteins [41] to transport lipids from the intestine to the liver and from the liver to tissues, including adipocytes, lung, heart, muscle and breast tissues. As apolipoproteins are detergent-like, they solubilize the hydrophobic parts of lipoproteins. Additionally, they are widely known as receptor ligands, enzyme co-factors and lipid carriers that are involved in regulation of the intravascular metabolism of lipoproteins and their ultimate tissue uptake. The synthesis of apolipoproteins is controlled by hormones such as insulin or glucagon and environmental factors including alcohol and intake of drugs, such as fibric acids, statins or niacins. Notably, regulation of subclasses of apolipoproteins are reported for a large number of malignancies [42-44]. Here we report differential expression of apolipoproteins A, C, E and H at different stages of tumor development in c-myc transgenic mice. Their disease association is discussed below. Apolipoprotein A-I (ApoA1, spot no. 9) belongs to the ApoA1/A4/E protein family and is primarily produced in the liver and the intestine. ApoA1 can be found in the extracellular space and, being a structural component of high density lipid proteins (HDL), takes part in cholesterol absorption. ApoA1 upregulation is associated with breast and lung cancer as suggested elsewhere [45, 46]. We found ApoA1 expression to be significantly increased by 1.8-fold in tumor bearing mice, aged 3 months and by 1.4-fold (pH 4-7) and 2.8-fold (pH 3-10) in tumor bearing mice, aged 14 months. Spot no. 11 was identified as apolipoprotein C3 (ApoC3) which is produced in the liver. This protein inhibits the lipoprotein and hepatic lipase and represses the uptake of lymph chylomicrons by hepatic cells. Thus, ApoC3 may repress the catabolism of triglyceride-rich particles. Upregulation of ApoC3 was demonstrated in a chronic renal failure model [47] and in diabetes [48], but regulation of ApoC3 in lung cancer was not reported so far. Serum levels of ApoC3 were increased already at early stages of cancerogenesis by 2.3-fold and still increased by 2.7-fold (pH 4-7) and 1.7-fold (pH 3-10) in lung tumor bearing mice, aged 14 months. Apolipoprotein E (ApoE, spot no. 12) is a mediator for binding, internalizing and metabolism of lipoprotein particles. It serves as a ligand for the low density lipoprotein (LDL) receptor and for the ApoE receptor (chylomicron remnant) of hepatic tissues. ApoE expression was marginally induced (p<0.05) in serum of tumor bearing mice, aged 3 months, but reduced in tumor bearing mice, aged 14 months, by 1.6-fold (pH 4-7) and 1.2-fold (pH 3-10), respectively. Regulation of ApoE was observed in human hepatocellular [49], colorectal [50] and pancreatic carcinoma [51]. Its differential expression in lung cancer is novel and was not reported so far. Notably, apolipoprotein E is reported to modulate clearance of apoptotic bodies *in vitro* and *in vivo* [52] and therefore may have a protective role in various pathologies. We further detected an E-Box motif in the promoter region of the ApoE gene therefore suggesting ApoE as a direct target of c-myc. Apolipoprotein H, also named as beta-2 glycoprotein-1 (ApoH, spot no. 13) binds to various kinds of negatively charged substances such as heparin, phospholipids and dextran sulfate. Through binding to phospholipids on the surface of damaged cells, ApoH may inhibit activation of the intrinsic blood coagulation cascade. ApoH is synthesized in the liver and secreted into plasma. As reported by other investigators, ApoH induction may be involved in blocking angiogenic processes in bladder cancer [53]. ApoH expression was induced in sera of lung tumor bearing mice by 1.4-fold (pH 4-7) and 1.7-fold (pH 3-10) at late stages of lung cancer only while expression was unchanged at early stages. To the best of our knowledge, ApoH is a novel finding for its regulation in lung cancer.

### 3.3.3 Oxidative defense and complement activation in lung cancer

Glutathione peroxidase 3 (Gpx3, spot no. 21) functions in response to oxidative damage by catalyzing the reduction of hydrogen peroxide, lipid peroxides and organic hydroperoxide. Induction of Gpx3 expression was observed in ovarian cancer [54] and diabetes [55]. We observed an initial upregulation of Gpx3 in serum of lung tumor bearing mice, aged 3 months, by 1.7-fold, followed by an increase in transgenic mice, aged 14 months, by > 10-fold (pH 3-10). Note, the E-Box motif "CACGTG" in the promoter region of the Gpx3 gene makes this gene a likely target for c-myc. Thus, upregulation of Gpx3 alongwith an overexpression of c-myc in this transgenic mouse model has been observed.
Properdin (spot no. 39), also known as factor P, is a serum glycoprotein and positive regulator of the alternate pathway for complement activation. It binds to and stabilizes the C3- and C5-convertase enzyme complexes. Complement C3 (spot no. 17) was not regulated in tumor bearing mice whereas properdin expression was increased by 1.5-fold at late stages of tumorigenesis only. Properdin plays a role in some specific immune responses and in tissue inflammation. It is known that properdin is involved in engulfing of pathogenes by phagocytes and in helping to neutralize some viruses, such as the influenza virus. [56, 57].

### 3.4 Disease associated regulation of serum proteins in c-myc and c-raf transgenic mouse models of lung cancer

We recently reported the serum proteome of c-raf transgenic mice which developed lung cancer through targeted overexpression of this kinase to alveolar epithelium [20]. Regulation of various serum proteins in c-myc transgenic mice of our present study was compared with those of c-raf transgenic mice (Suppl. Tab. 2). Strikingly, our data of regulated proteins are highly suggestive for candidate serum biomarkers that enable differentiation between AAH diagnosed in c-raf transgenic mice with that of BAC observed in c-myc transgenic mice. Table 3 gives an overview of our findings. Although alpha-1 antitrypsin, alpha-2 macroglobulin, transthyretin and properdin were commonly regulated in serum of tumor bearing mice, several proteins described in section 3.3, such as apolipoproteins, plasma retinol binding protein or serum amyloid P component were exclusively associated with BAC. Similarily, regulation of major urinary proteins, vitamin D-binding protein or a soluble form of the epidermal growth factor receptor (EGFR) was uniquely associated with AAH.

### 4 Conclusions

Based on our previous work on the SP-C/c-myc transgenic mouse model [13] we report disease regulated serum proteins at early and advanced stages of lung cancer. Serum protein expression of tumor bearing mice was compared with those of healthy animals. Separation of serum proteins with 2-DE at two different pH ranges yielded a total of 46 distinct proteins identified by MALDI-MS. Some of all identified proteins are acute phase reactants (APP) and were either regulated or exclusively expressed in lung cancer bearing mice, notably orosomucoid-8, alpha-1 antitrypsin (A1AT), A2M, serum amyloid P component (SAMP), plasma retinol binding protein (RBP), transthyretin and serum albumin. Likewise, A2M and apolipoprotein A-I have been shown to be upregulated in lung cancer [46], while regulation of apolipoprotein E is novel and may play a role in apoptosis [52]. Additionally, disease associated regulation of apolipoprotein H may also play a role in angiogenesis [53]. We found glutathione peroxidase 3 (Gpx3), properdin and transthyretin to be upregulated in tumor bearing mice. Notably, Gpx3 was identified as a direct target of c-myc and is an antioxidant and was shown to be associated with ovarian cancer [54]; properdin plays a role in immune response and inflammation. Finally, increased transthyretin serum levels observed in tumor bearing mice was also reported for human lung cancer patients [39]. A number of differentially expressed proteins were identified as direct target genes of the c-myc transcription factor and include amongst others orosomucoid-8, alpha-2 macroglobulin (A2M), apolipoprotein E and glutathione peroxidase 3. Thus, regulation of these proteins caused by an exaggerated expression of c-myc may be a cause for their regulation. Nevertheless, some of the regulated proteins are no direct targets of the c-myc transcription factor, i.e. several apolipoproteins, properdin, plasma retinol binding protein, serum amyloid P component and transthyretin. As the effect of indirect c-myc targeting on gene regulation is known, further investigation of these proteins needs to be accomplished. Our data are highly suggestive for serum biomarkers to differentiate between atypical adenomatous hyperplasia (AAH) and bronchiolo-alveolar (BAC)/papillary adenocarcinomas (PLAC) at early stages of disease. Taken collectively, our present study validates this tumor model further and provides novel information on its serum proteome. Some of the regulated proteins could already be translated to human malignancies, as reported elsewhere.

### Figure captions

### Figure 1a:

Gene construct of the SP-C/c-myc transgenic mouse model as reported by Ehrhardt *et al.* [13].

### Figure 1b:

Histology of a lung of a healthy (A1) and a tumor bearing (B1) SP-C/c-myc transgenic mouse, aged 14 months. Hematoxylin and eosin staining was used for histopathology of tumors. A2 and B2: macroscopical views of lungs of healthy and tumor bearing mice, respectively.

### Figure 2a:

2-DE serum proteome map of lung tumor bearing SP-C/c-myc transgenic mice with a pH range from 3-10.

### Figure 2b:

2-DE serum proteome map of lung tumor bearing SP-C/c-myc transgenic mice with a pH range from 4-7.

### Figure 3a:

Examples of regulated serum proteins at different stages of tumorigenesis (3 months and 14 months) in SP-C/c-myc transgenic (T) and healthy (C) mice. Protein spots of interest are marked by circles.

### Figure 3b:

Comparison of fold-changes of disease regulated proteins between two stages of lung tumorigenesis, i.e. 3 and 14 months (p<0.05).

### Table captions

### Table 1:

Protein identification in 2-DE maps of mouse serum proteins including healthy and lung tumor bearing mice, identified by MALDI MS/MS. A total of 46 proteins were identified. P/ and M*ᵣ* are based on the theoretical values of the precursor. Sequence coverage and no. of matched peptides: The value from one typical spot was given if many spots were assigned as one number (protein). Protein identification was based on PMF and PFF. See Supplementary Table 1 for detailed information.

### Table 2a and 2b:

Expression profiles of significantly regulated proteins (p<0.05) from 2-D gels of tumor bearing mice, aged 3 months (pH 3-10) and tumor bearing mice, aged 14 months (pH 4-7 and pH 3-10). Quantification of protein abundance was done using the PDQuest 2-D software (BioRad) by measurement of the normalized optical density (arbitrary units, AU) of each protein spot. The change in abundance of the proteins is expressed by the calculated ratio (T/C) between mean values from tumor (T) and healthy (C) samples. %RSD: percental relative standard deviation. Spot no. 41 (SAMP_MOUSE) is exclusively expressed in tumor bearing mice. Student's t-test was used for calculation of p-values.

### Table 3:

Commonly and specifically regulated proteins in adenomatous hyperplasia (AAH) and bronchiolo-alveolar adenocarcinomas (BAC). Recently, AAH was studied in SP-C/c-raf transgenic mice [20], whereas BAC is the subject of our present work on SP-C/c-myc transgenic mice. 1M: tumor bearing mice, aged 1 month; 12M: 12 months; 3M: 3 months; 14M: 14 months; C: exclusively expressed in control mice; T: exclusively expressed in tumor bearing mice; -: protein not detected; ↔: no regulation/no fold-change; ↑: ≤ 1.5 fold-change; ↑↑: ≤ 3.0 fold-change; ↑↑↑: > 3.0 fold-change; ↓: ≥ 0.7 fold-change; ↓↓: ≥ 0.3 fold-change ; ↓↓↓: < 0.3 fold-change. APP: acute phase protein; E-Box: containing 5'-CACGTG-3' consensus sequence in promoter region.

### Supplementary Table 1:

A summary of serum proteins from SP-C/c-myc transgenic mice. The Mascot score (PMF), ions score (PFF), the number of identified peptides, their sequence, the protein coverage of the best hits and supporting information are shown for each identified protein. O@M: oxidation at the amino acid methionine.

**Table 1**

| **No.** | **UniProt entry name** | **UniProt accession** **no.** | **Protein name** | **p/** | **M*ᵣ* (kDa)** | **Sequence coverage** | **No. of matched peptides** | **Identification** |
|---|---|---|---|---|---|---|---|---|
| 1 | A1AG8_MUSCR | P21352 | Orosomucoid-8 | 5.6 | 23.9 | 7% | 1 | PFF |
| 2 | A1AT1_MOUSE | P07758 | Alpha-1-antitrypsin 1-1 | 5.4 | 46.0 | 26% | 10 | PMF |
| 3-A, 3-B | A1AT6_MOUSE | P81105 | Alpha-1-antitrypsin 1-6 | 5.3 | 45.8 | 34% | 11 | PMF |
| | | | | | | 9% | 3 | PFF |
| 4, 4-B, 4-C, 4-D | A2MG_MOUSE | 061838 | Alpha-2-maaoglobulin | 6.2 | 165.9 | 1% | 1 | PFF |
| 5 | ACTG_MOUSE | P63260 | Gamma-actin | 5.3 | 41.8 | 31% | 9 | PMF |
| 6 | AFAM_MOUSE | 089020 | Afamin | 5.5 | 69.4 | 22% | 14 | PMF |
| 7 | ALBU_MOUSE | P07724 | Serum Albumin | 5.8 | 68.7 | 24% | 11 | PMF |
| | | | | | | 2% | 1 | PFF |
| 8 | ANT3_MOUSE | P32261 | Antithrombin-III | 6.1 | 52.0 | 33% | 13 | PMF |
| 9 | APOA1_MOUSE | Q00623 | Apolipoprotein A-I | 5.6 | 30.6 | 40% | 15 | PMF |
| | | | | | | 4% | 1 | PFF |
| 10 | APOA4_MOUSE | P06728 | ApolipoproteinA-IV | 5.4 | 45.0 | 64% | 21 | PMF |
| 11 | APOC3_MOUSE | P33622 | ApolipoproteinC-III | 4.6 | 11.0 | 19% | 1 | PFF |
| 12 | APOE_MOUSE | P08226 | Apolipoprotein E | 5.6 | 35.9 | 44% | 20 | PMF |
| | | | | | | 4% | 1 | PFF |
| 13. 13-B | APOH_MOUSE | Q01339 | Apolipoprotein H | 8.6 | 38.6 | 49% | 15 | PMF |
| 14 | CFAB_MOUSE | P04186 | Complement factor B | 7.2 | 85.0 | 18% | 12 | PMF |
| 15 | CFAH_MOUSE | P06909 | Complement factor H | 6.6 | 139.1 | 13% | 16 | PFF |
| 16 | CLUS_MOUSE | 006890 | Clusterin (Apolipoprotein J) | 5.5 | 51.7 | 12% | 4 | PMF |
| 17 | CO3_MOUSE | P01027 | Complement C3 | 6.4 | 186.5 | 8% | 10 | PMF |
| 18 | CRP_MOUSE | P14847 | C-reactive protein | 5.8 | 25.4 | 15% | 4 | PMF |
| 19 | FETUA_MOUSE | P29699 | Fetuin-A | 6.0 | 37.3 | 28% | 7 | PMF |
| 20 | GELS_MOUSE | P13020 | Gelsolin | 5.8 | 85.9 | 31% | 19 | PMF |
| 21 | GPX3_MOUSE | P46412 | Glutathione peroxidase 3 | 8.3 | 25.3 | 7% | 1 | PFF |
| 22 | HA10_MOUSE | P01898 | H-2 class I histocompatibility antigen, Q10 alpha chain | 5.1 | 37.3 | 31% | 10 | PMF |
| 23 | HBA_MOUSE | P01942 | Hemoglobin subunit alpha | 8.0 | 15.1 | 37% | 5 | PMF |
| 24 | HEMO_MOUSE | Q91X72 | Hemopexin | 7.9 | 51.3 | 35% | 14 | PMF |
| | | | | | | 5% | 1 | PFF |
| 25 | IGJ_MOUSE | P01592 | Immunglobulin J chain | 4.8 | 18.0 | 28% | 6 | PMF |
| 26 | K2C8_MOUSE | P11679 | Cytokeratin-8 | 5.7 | 54.6 | 35% | 17 | PMF |
| 27 | KAC_MOUSE | P01837 | lmmunglobulin kappa chain C region | 5.2 | 11.8 | 32% | 4 | PMF |
| 28 | KNG1_MOUSE | 008677 | Kininogen-1 | 6.1 | 73.1 | 17% | 10 | PMF |
| | | | | | | 3% | 1 | PFF |
| 29 | KV3B_MOUSE | P01655 | Immunglobulin kappa chain V-III region PC 7132 | 5.2 | 12.1 | 16% | 1 | PFF |
| 30 | KV5L_MOUSE | P01645 | Immunglobulin kappa chain V-V region HP 93G7 | 8.0 | 12.0 | 50% | 5 | PMF |
| | | | | | | 7% | 1 | PFF |
| 31 | KV6K_MOUSE | P04945 | Immunglobulin kappa chain V-VI region NQ2-6.1 | 9.0 | 11.7 | 14% | 1 | PFF |
| 32 | MBL2_MOUSE | P41317 | Mannose-binding protein C | 5.0 | 26.0 | 24% | 5 | PMF |
| | | | | | | 5% | 1 | PFF |
| 33 | MUC_MOUSE | P01872 | Immunglobulin mu chain C region secreted form | 6.6 | 50.0 | 2% | 1 | PFF |
| 34 | MUP2_MOUSE | P11589 | Major urinary protein 2 | 5.0 | 20.7 | 50% | 10 | PMF |
| 35 | MUP8_MOUSE | P04938 | Major urinary proteins 11 and 8 | 4.9 | 17.6 | 72% | 10 | PMF |
| 36 | MYH9_MOUSE | Q8VDD5 | Myosin-9 | 5.5 | 226.4 | 5% | 8 | PMF |
| 37 | PLF4_MOUSE | Q9Z126 | Platelet factor 4 | 9.4 | 11.2 | 44% | 7 | PMF |
| 38 | PLMN_MOUSE | P20918 | Plasminogen | 6.2 | 90.8 | 25% | 21 | PMF |
| 39 | PROP_MOUSE | P11680 | Properdin | 8.3 | 50.3 | 21% | 8 | PMF |
| 40 | RETBP_MOUSE | Q00724 | Plasma retinol-binding protein | 5.7 | 23.2 | 24% | 5 | PMF |
| | | | | | | 8% | 1 | PFF |
| 41 | SAMP_MOUSE | P12246 | Serum amyloid P-component | 6.0 | 26.2 | 8% | 1 | PFF |
| 42 | SPA3K_MOUSE | P07759 | Contrapsin | 5.1 | 46.9 | 32% | 14 | PMF |
| 43 | TRFE_MOUSE | Q92111 | Serotransferrin | 6.9 | 76.7 | 51% | 36 | PMF |
| 44 | TTHY_MOUSE | P07309 | Transthyretin | 5.8 | 15.8 | 55% | 5 | PMF |
| | | | | | | 15% | 1 | PFF |
| 45 | VTDB_MOUSE | P21614 | Vitamin D-binding protein | 5.4 | 53.6 | 30% | 9 | PMF |
| | | | | | | 4% | 1 | PFF |
| 46 | ZA2G_MOUSE | Q64726 | Zinc-alpha-2-glycoprotein | 5.8 | 35.3 | 32% | 9 | PMF |
| | | | | | | 3% | 1 | PFF |

**Table 2a**

| No. | pH | Protein ID | Tumor (3 months) | | | | | | | | Control (3 months) | | | | | | | | Ratio (T/C) | p |
|---|---|---|---|---|---|---|---|---|---|---|---|---|---|---|---|---|---|---|---|---|
| | | | T1 | T2 | T3 | T4 | T5 | T6 | mean | %RSD | C1 | C2 | C3 | C4 | C5 | C6 | mean | %RSD | | |
| 1 | pH 3-10 | A1AG8_MUSCR | 3856 | 4255 | 4040 | 4232 | 3453 | 4196 | 4005 | 7.72 | 2221 | 1994 | 1914 | 2772 | 2273 | 1948 | 2187 | 14.73 | 1.8 | 1.63 x 10⁻⁰⁶ |
| 4-B | pH 3-10 | A2MG_MOUSE | 2064 | 2195 | 2205 | 2278 | 2128 | 2146 | 2169 | 3.4 | 794 | 627 | 837 | 990 | 784 | 846 | 813 | 14.43 | 2.7 | 3.62 x 10⁻¹⁰ |
| 9 | pH 3-10 | APOA1_MOUSE | 8228 | 7526 | 7911 | 7777 | 8011 | 7826 | 7880 | 2.99 | 4729 | 4474 | 4085 | 4775 | 4105 | 4264 | 4405 | 6.88 | 1.8 | 7.80 x 10⁻¹⁰ |
| 11 | pH 3-10 | APOC3_MOUSE | 1765 | 1697 | 1841 | 1763 | 1780 | 795 | 1606 | 24.93 | 815 | 898 | 809 | 758 | 845 | 97 | 704 | 42.76 | 2.3 | 0.00130 |
| 12 | pH 3-10 | APOE_MOUSE | 2144 | 2075 | 2021 | 2264 | 1886 | 2221 | 2102 | 6.6 | 1714 | 1782 | 1730 | 1916 | 1765 | 1827 | 1789 | 4.12 | 1.2 | 0.00064 |
| 21 | pH 3-10 | GPX3_MOUSE | 439 | 421 | 403 | 474 | 435 | 418 | 432 | 5.67 | 242 | 253 | 247 | 269 | 255 | 251 | 253 | 3.61 | 1.7 | 1.19 x 10⁻⁰⁶ |
| 40 | pH 3-10 | RETBP_MOUSE | 993 | 995 | 997 | 1107 | 1067 | 967 | 1021 | 5.27 | 407 | 417 | 426 | 408 | 494 | 418 | 428 | 7.65 | 2.4 | 5.33 x 10⁻¹⁰ |
| 41 | pH 3-10 | SAMP_MOUSE | 178 | 103 | 12 | 4 | 4 | 3 | 50 | 145.76 | 9 | 18 | 9 | 14 | 5 | 3 | 10 | 57.44 | 5.2 | 0.20532 |
| 44 | pH 3-10 | TTHY_MOUSE | 3568 | 3654 | 3629 | 3693 | 3690 | 3542 | 3629 | 1.73 | 2352 | 2533 | 2400 | 2329 | 2343 | 2305 | 2377 | 3.47 | 1.5 | 4.59 x 10⁻¹¹ |

**Table 2b**

| No. | pH | Protein ID | Tumor (14 months) | | | | | | | | Control (14 months) | | | | | | | | Ratio (T/C) | p |
|---|---|---|---|---|---|---|---|---|---|---|---|---|---|---|---|---|---|---|---|---|
| | | | T1 | T2 | T3 | T4 | T5 | T6 | mean | %RSD | C1 | C2 | C3 | C4 | C5 | C6 | mean | %RSD | | |
| 1 | pH 4-7 | AIAG8_MUSCR | 1139 | 1003 | 1123 | 998 | 1217 | 867 | 1056 | 11.88 | 425 | 398 | 401 | 414 | 502 | 387 | 421 | 9.91 | 2.5 | 3.50 x 10⁻⁰⁷ |
| 1 | pH 3-10 | A1AG8_MUSCR | 834 | 745 | 769 | 895 | 744 | 867 | 809 | 8.07 | 780 | 637 | 545 | 715 | 679 | 552 | 651 | 14.20 | 1.2 | 6.64 x 10⁻⁰³ |
| | | | | | | | | | | | | | | | | | | | | |
| 2 | pH 4-7 | A1AT1_MOUSE | 474 | 456 | 494 | 485 | 541 | 391 | 473 | 10.39 | 400 | 384 | 482 | 314 | 391 | 327 | 383 | 15.65 | 1.2 | 1.73 x 10⁻⁰² |
| 2 | pH 3-10 | A1AT1_MOUSE | 518 | 616 | 549 | 863 | 636 | 663 | 641 | 18.99 | 498 | 344 | 475 | 555 | 475 | 562 | 485 | 16.26 | 1.3 | 2.49 x 10⁻⁰² |
| | | | | | | | | | | | | | | | | | | | | |
| 3-A | pH 4-7 | A1AT6_MOUSE | 781 | 848 | 891 | 899 | 884 | 916 | 870 | 5.61 | 630 | 686 | 752 | 618 | 812 | 711 | 702 | 10.51 | 1.2 | 8.90 x 10⁻⁰⁴ |
| 3-B | pH 4-7 | A1AT6_MOUSE | 450 | 546 | 502 | 446 | 680 | 486 | 518 | 16.82 | 349 | 354 | 306 | 359 | 410 | 484 | 377 | 16.46 | 1.4 | 8.85 x 10⁻⁰³ |
| 3-A/B | pH 3-10 | A1AT6_MOUSE | 0 | 0 | 0 | 0 | 0 | 0 | 0 | 0 | 0 | 0 | 0 | 0 | 0 | 0 | 0 | 0 | 0 | 0 |
| | | | | | | | | | | | | | | | | | | | | |
| 4-B | pH4-7 | A2MG_MOUSE | 5588 | 5789 | 4708 | 5609 | 5332 | 6008 | 5506 | 8.19 | 867 | 935 | 766 | 790 | 846 | 910 | 852 | 7.74 | 6.5 | 2.39 x 10⁻¹⁰ |
| 4-B | pH 3-10 | A2MG_MOUSE | 6773 | 6059 | 6128 | 6592 | 5845 | 6307 | 6284 | 5.52 | 4176 | 4044 | 4087 | 4153 | 4900 | 3793 | 4192 | 8.89 | 1.5 | 1.51 x 10⁻⁰⁶ |
| | | | | | | | | | | | | | | | | | | | | |
| 9 | pH4-7 | APOA1_MOUSE | 5423 | 5219 | 4868 | 5172 | 4972 | 5267 | 5154 | 3.93 | 3490 | 4036 | 3362 | 3379 | 4004 | 3313 | 3597 | 9.25 | 1.4 | 1.93 x 10⁻⁰⁶ |
| 9 | pH 3-10 | APOA1_MOUSE | 5952 | 4324 | 6205 | 5549 | 5869 | 7721 | 5937 | 18.47 | 1603 | 1725 | 2017 | 2471 | 2260 | 2495 | 2095 | 18.04 | 2.8 | 1.04 x 10⁻⁰⁵ |
| | | | | | | | | | | | | | | | | | | | | |
| 11 | pH 4-7 | APOC3_MOUSE | 3433 | 3693 | 3472 | 3132 | 3217 | 3552 | 3417 | 6.02 | 1313 | 1342 | 1236 | 1511 | 1189 | 1096 | 1281 | 11.17 | 2.7 | 1.57 x 10⁻⁰⁹ |
| 11 | pH3-10 | APOC3_MOUSE | 3682 | 3357 | 3318 | 3716 | 3217 | 3885 | 3529 | 7.57 | 2372 | 1630 | 1749 | 2522 | 2131 | 2018 | 2070 | 16.71 | 1.7 | 9.73x10⁻⁰⁶ |
| | | | | | | | | | | | | | | | | | | | | |
| 12 | pH 4-7 | APOE_MOUSE | 2502 | 2139 | 2619 | 2204 | 2553 | 2267 | 2381 | 8.48 | 2478 | 2312 | 2823 | 2908 | 3116 | 2935 | 2762 | 11.02 | 0.9 | 2.85 x 10⁻⁰² |
| 12 | pH 3-10 | APOE_MOUSE | 1857 | 1727 | 1294 | 1657 | 1553 | 1834 | 1654 | 12.65 | 2993 | 2207 | 2688 | 2876 | 2598 | 2978 | 2723 | 10.95 | 0.6 | 2.95 x 10⁻⁰⁵ |
| | | | | | | | | | | | | | | | | | | | | |
| 13 | pH 4-7 | APOH_MOUSE | 2377 | 2773 | 2292 | 2783 | 2365 | 2131 | 2454 | 10.85 | 1525 | 1330 | 1774 | 2073 | 2020 | 1947 | 1778 | 16.66 | 1.4 | 1.97 x 10⁻⁰³ |
| 13 | pH 3-10 | APOH_MOUSE | 1148 | 1281 | 1269 | 1724 | 1247 | 1410 | 1347 | 15.08 | 845 | 752 | 809 | 898 | 749 | 683 | 790 | 9.75 | 1.7 | 9.11 x 10⁻⁰⁵ |
| | | | | | | | | | | | | | | | | | | | | |
| 21 | pH4-7 | GPX3_MOUSE | 520 | 339 | 547 | 506 | 358 | 541 | 469 | 20.13 | 165 | 124 | 179 | 232 | 229 | 147 | 179 | 24.35 | 2.6 | 4.66 x 10⁻⁰⁵ |
| 21 | pH 3-10 | GPX3_MOUSE | 189 | 223 | 240 | 233 | 213 | 290 | 231 | 14.62 | 19 | 20 | 15 | 20 | 25 | 30 | 22 | 24.39 | 10.8 | 3.45 x 10⁻⁰⁸ |
| | | | | | | | | | | | | | | | | | | | | |
| 39 | pH 4-7 | PROP_MOUSE | 0 | 0 | 0 | 0 | 0 | 0 | 0 | 0 | 0 | 0 | 0 | 0 | 0 | 0 | 0 | 0 | 0 | 0 |
| 39 | pH 3-10 | PROP_MOUSE | 284 | 311 | 304 | 248 | 240 | 240 | 271 | 12.02 | 228 | 156 | 182 | 139 | 217 | 187 | 185 | 18.42 | 1.5 | 1.16 x 10⁻⁰³ |
| | | | | | | | | | | | | | | | | | | | | |
| 40 | pH 4-7 | RETBP_MOUSE | 1244 | 1185 | 1534 | 1450 | 1225 | 1442 | 1347 | 10.83 | 429 | 466 | 555 | 716 | 521 | 546 | 539 | 18.44 | 2.5 | 5.52 x 10⁻⁰⁷ |
| 40 | pH 3-10 | RETBP_MOUSE | 407 | 320 | 537 | 402 | 367 | 427 | 410 | 17.74 | 66 | 106 | 87 | 63 | 94 | 74 | 82 | 20.67 | 5.0 | 8.00 x 10⁻⁰⁷ |
| | | | | | | | | | | | | | | | | | | | | |
| 41 | pH 4-7 | SAMP_MOUSE | 638 | 1271 | 1350 | 724 | 523 | 1496 | 1000 | 41.86 | 0 | 0 | 0 | 0 | 0 | 0 | 0 | 0 | T | 1.61 x 10⁻⁰⁴ |
| 41 | pH 3-10 | SAMP_MOUSE | 411 | 967 | 750 | 901 | 501 | 800 | 722 | 30.64 | 0 | 0 | 0 | 0 | 0 | 0 | 0 | 0 | T | 1.19 x 10⁻⁰⁵ |
| | | | | | | | | | | | | | | | | | | | | |
| 44 | pH 4-7 | TTHY_MOUSE | 9952 | 9049 | 9204 | 8992 | 9869 | 10721 | 9631 | 7.01 | 8045 | 7835 | 6190 | 9258 | 5998 | 5384 | 7118 | 20.91 | 1.4 | 3.68 x 10⁻⁰³ |
| 44 | pH 3-10 | TTHY_MOUSE | 9569 | 10047 | 8934 | 8170 | 8330 | 9154 | 9034 | 7.95 | 6148 | 6432 | 6932 | 6738 | 6676 | 6095 | 6504 | 5.18 | 1.4 | 1.44 x 10⁻⁰⁵ |

**Table 3**

| | **Protein ID** | **Protein name** | **AAH** | | **BAC** | | **APP** | **E-Box** |
|---|---|---|---|---|---|---|---|---|
| | | | 1M | 12M | 3M | 14M | | |
| **common** | A1AT6_MOUSE | Alpha-1-antitrypsin 1-6 | ↑ | ↑↑↑ | ↔ | ↑ | yes | yes |
| | A2MG_MOUSE | Alpha-2-macroglobulin (A2M) | ↓ | ↑↑ | ↑↑ | ↑↑↑ | yes | yes |
| | TTHY_MOUSE | Transthyretin | ↑ | ↓↓↓ | ↑ | ↑ | yes | no |
| | PROP_MOUSE | Properdin | - | C | ↔ | ↑ | no | no |
| **specific** | A1AG8_MUSCR | Orosomucoid-8 | ↔ | ↔ | ↑↑ | ↑↑ | yes | yes |
| | APOA1_MOUSE | Apolipoprotein A-I | ↔ | ↔ | ↑↑ | ↑↑ | no | no |
| | APOC3_MOUSE | Apolipoprotein C-III | ↔ | ↔ | ↑↑ | ↑↑ | no | no |
| | APOE_MOUSE | Apolipoprotein E | ↔ | ↔ | ↑ | ↓↓ | no | yes |
| | APOH_MOUSE | Apolipoprotein H | ↔ | ↔ | ↔ | ↑↑ | no | no |
| | GPX3_MOUSE | Glutathione peroxidase 3 | ↔ | ↔ | ↑↑ | ↑↑↑ | no | yes |
| | RETBP_MOUSE | Plasma retinol-binding protein | ↔ | ↔ | ↑↑ | ↑↑↑ | yes | no |
| | SAMP_MOUSE | Serum amyloid P-component | - | - | ↑↑↑ | T | yes | no |
| | MUP8_MOUSE | Major urinary proteins (MUP) | ↑↑↑ | ↑↑↑ | ↔ | ↔ | no | no |
| | VTDB_MOUSE | Vitamin D-binding protein | ↓ | ↑↑ | ↔ | ↔ | no | no |
| | S6A11_MOUSE | Sodium- and chloride- | - | ↑↑↑ | ↔ | - | no | no |
| | | dependent GABA transporter 4 | | | | | | |
| | EGFR_MOUSE | Epidermal growth factor | - | C | ↔ | - | no | no |
| | | receptor (EGFR) | | | | | | |

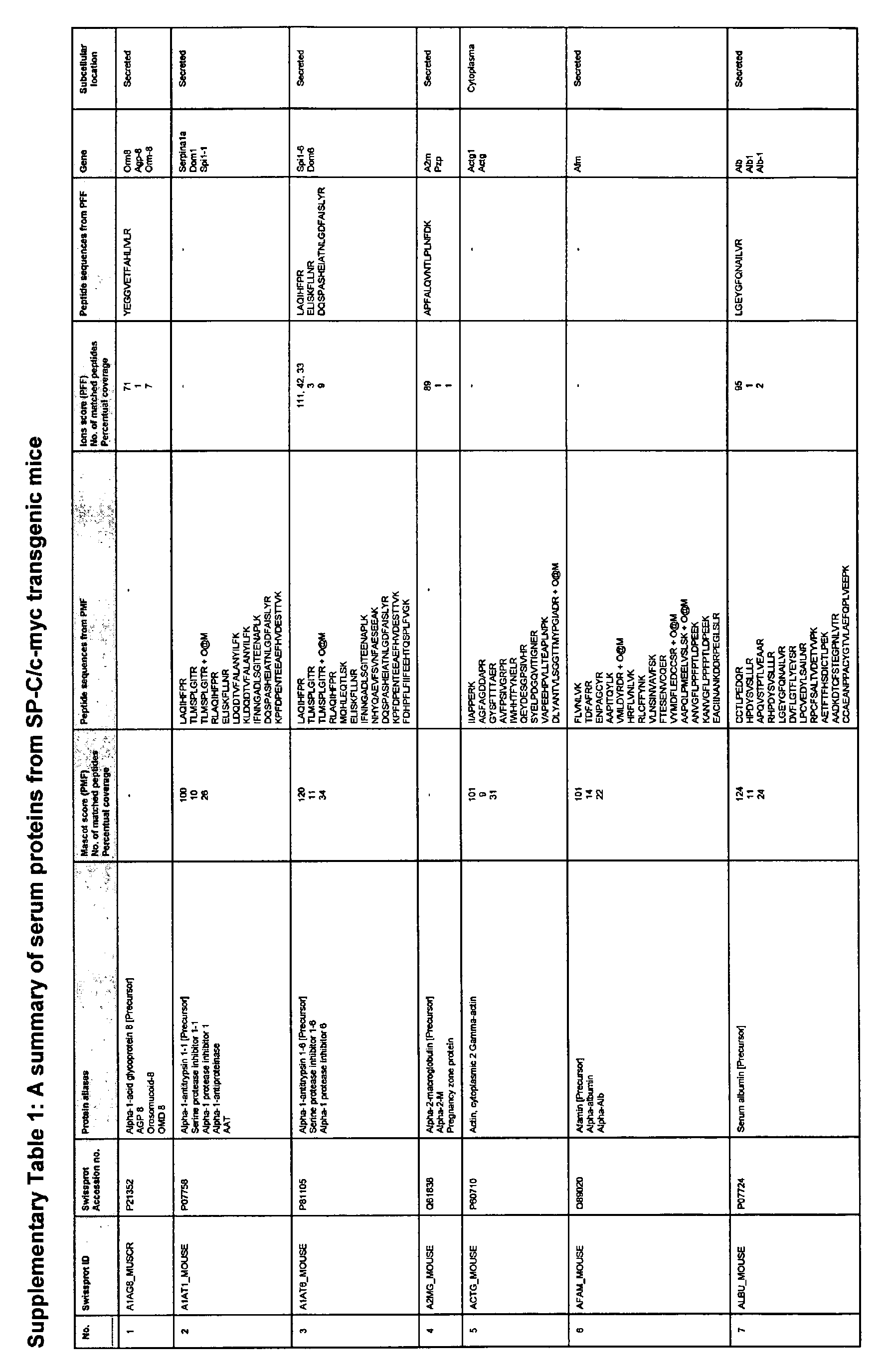

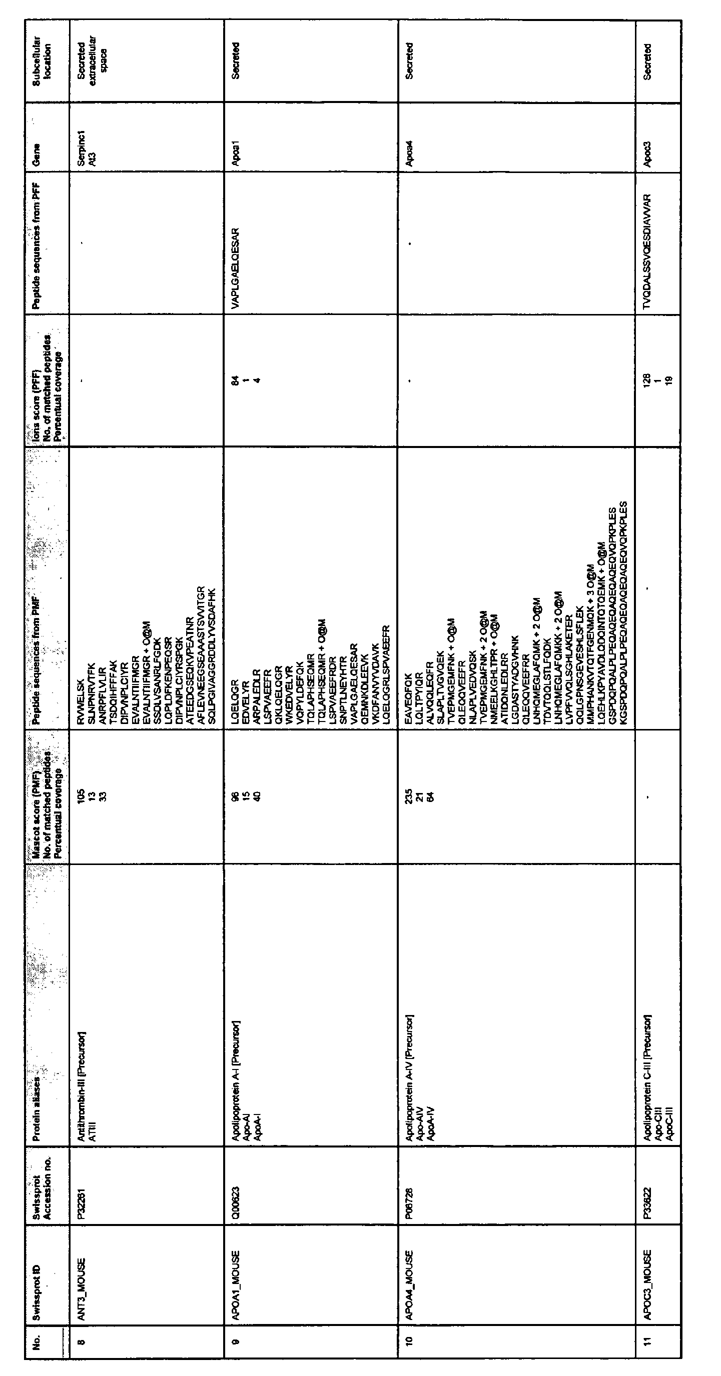

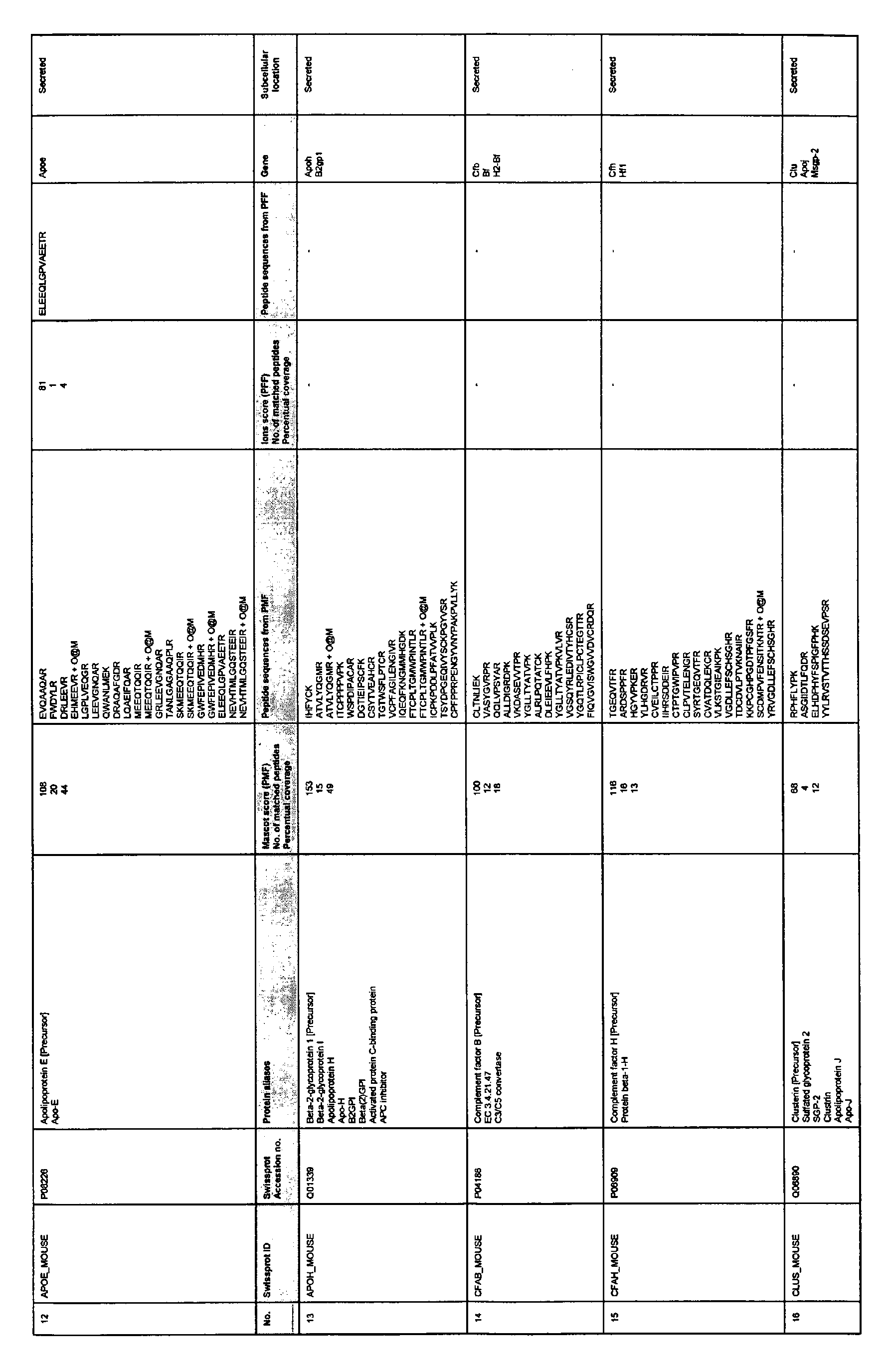

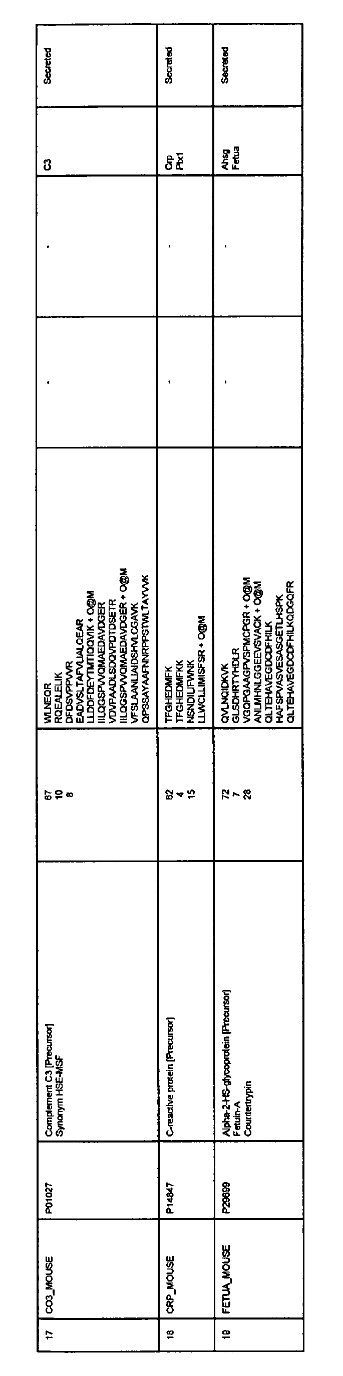

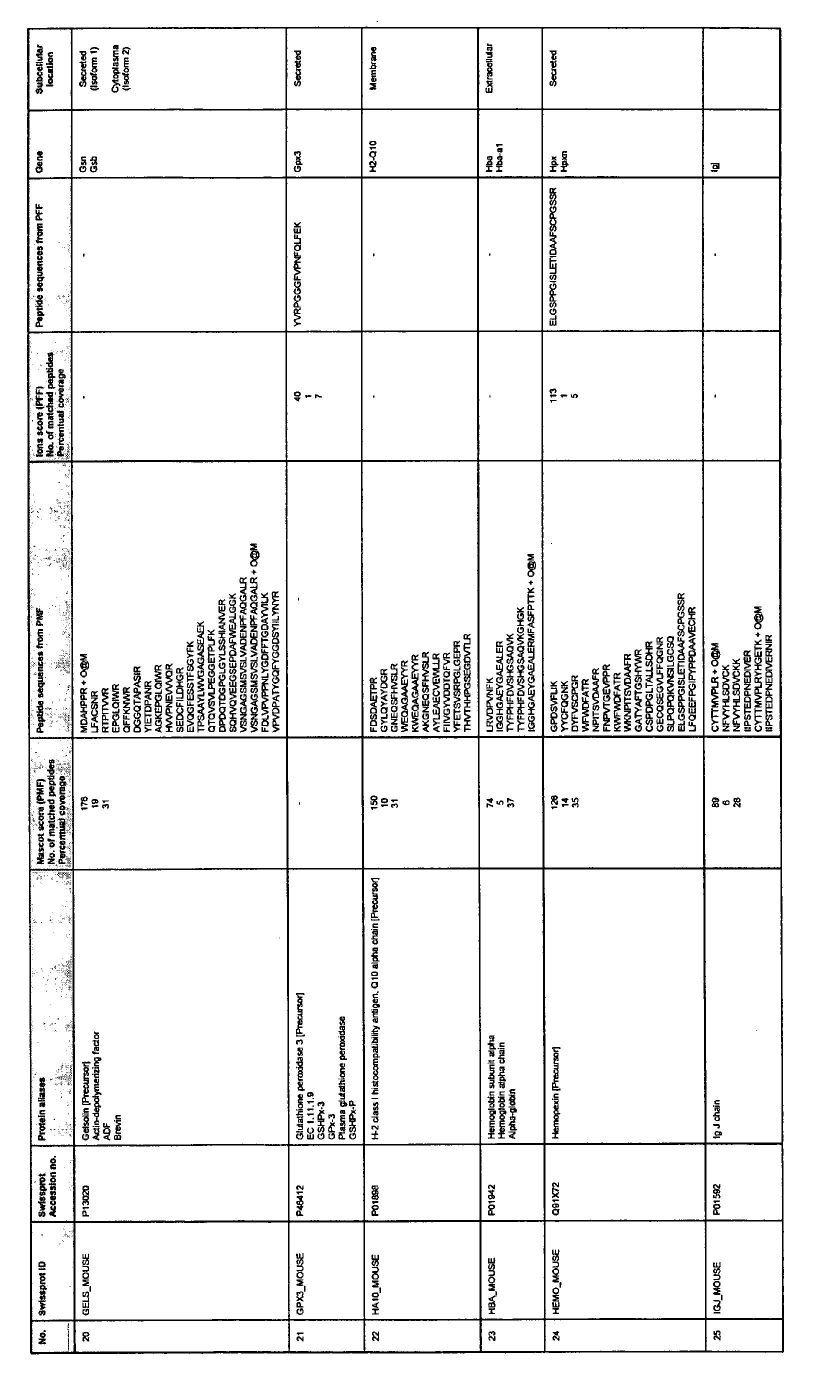

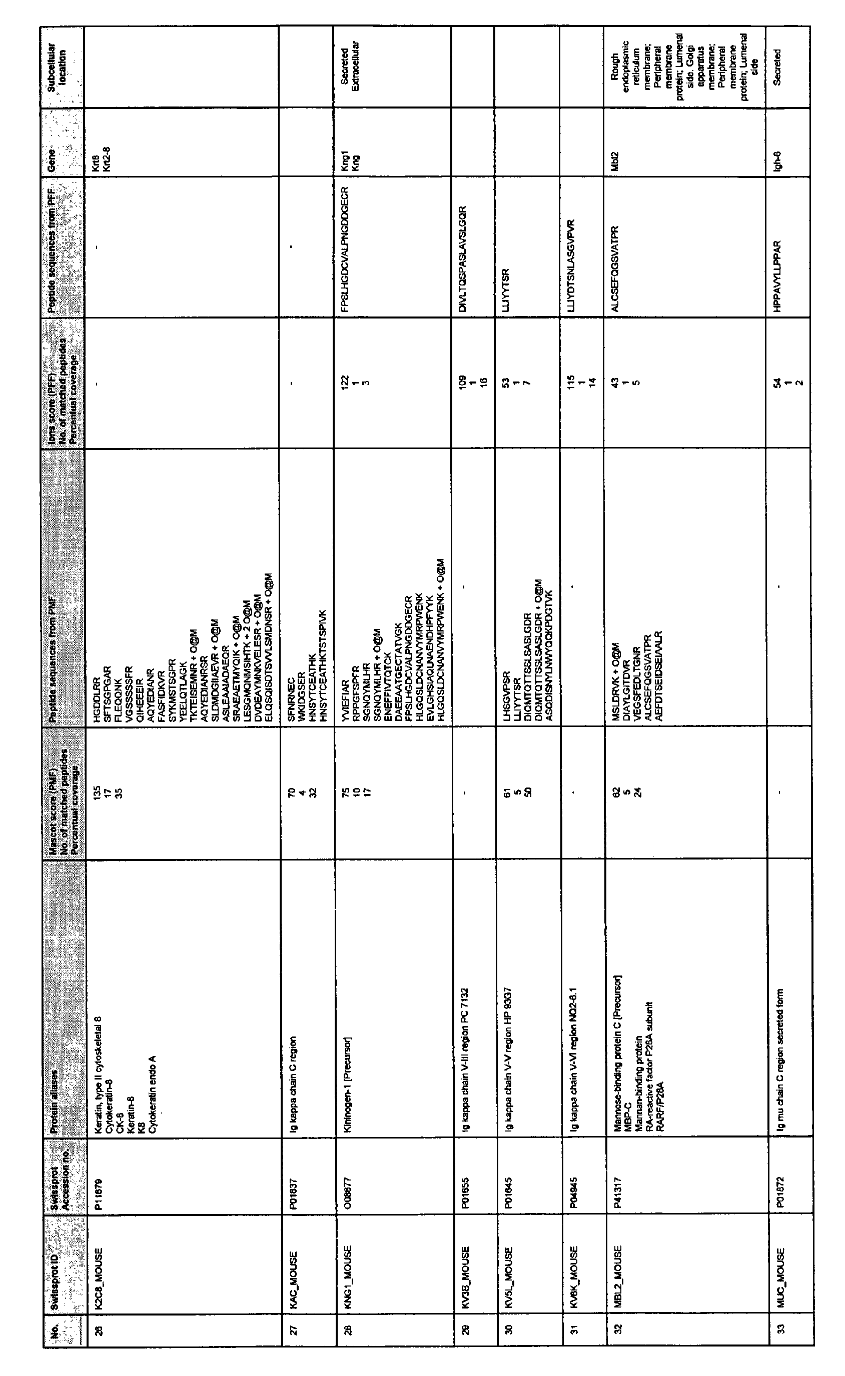

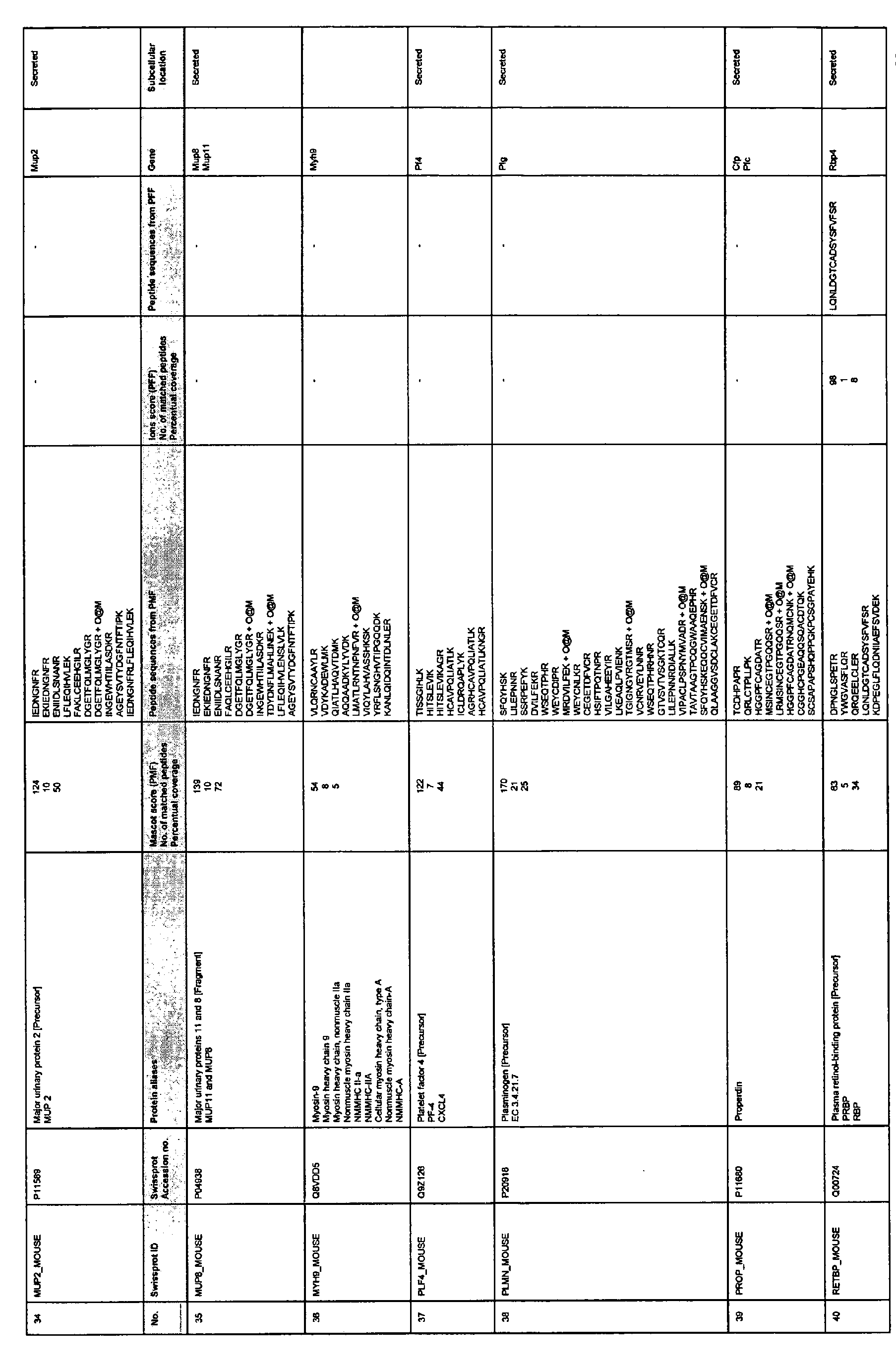

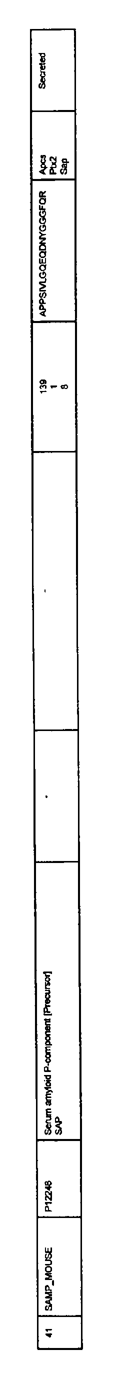

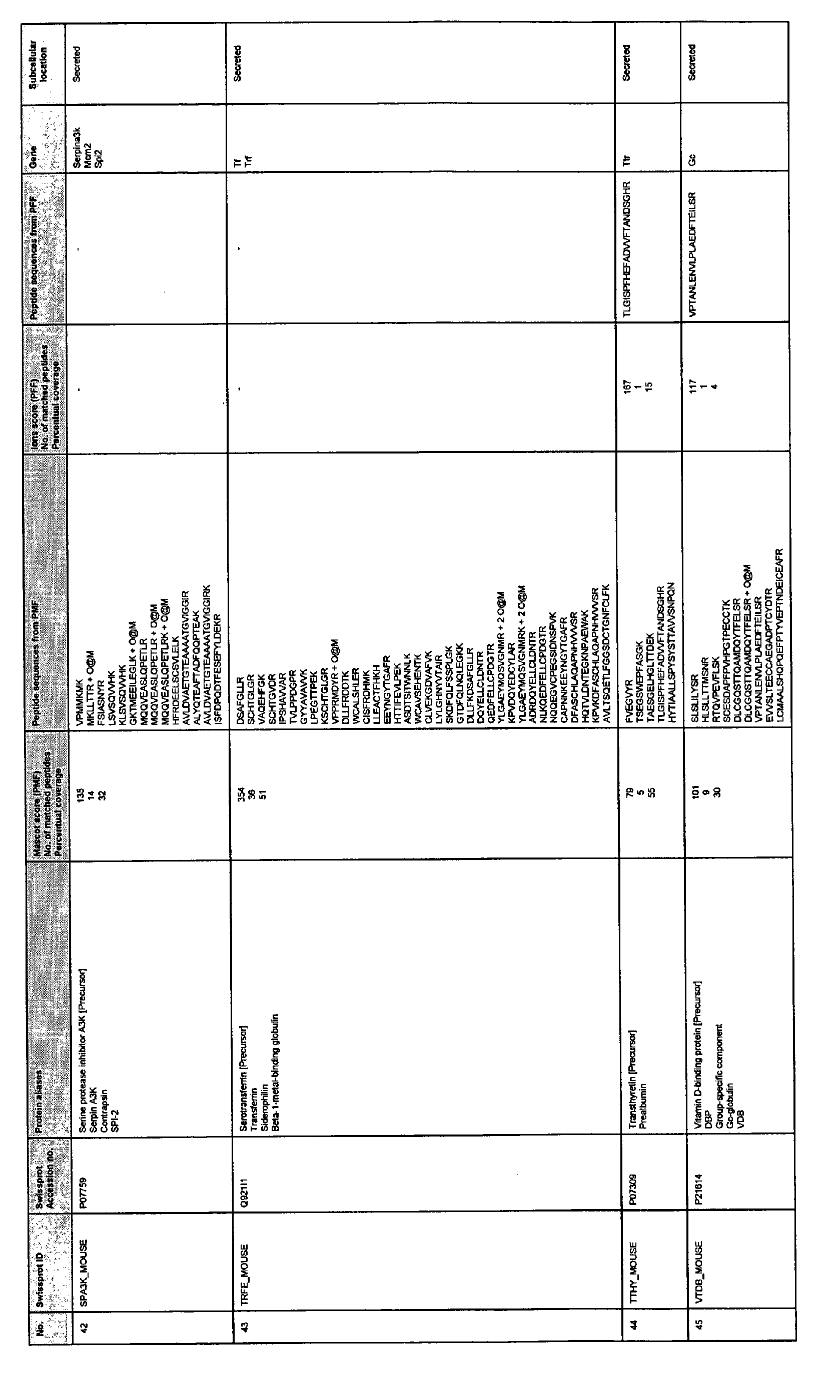

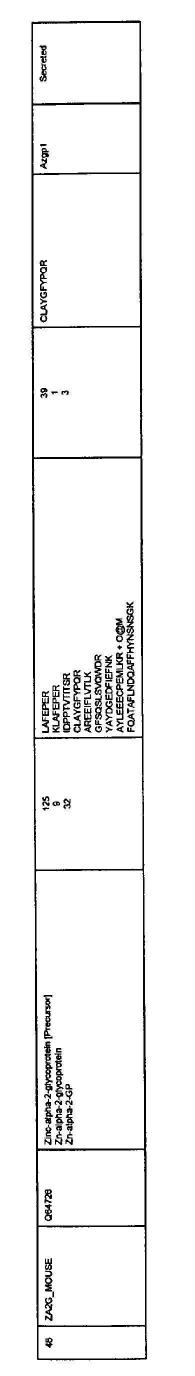

### 5 References

[1] Duarte RL, Paschoal ME. Molecular markers in lung cancer: prognostic role and relationship to smoking. J Bras Pneumol 2006, 32, 56-65. *Review.*
[2] Bhattacharjee A, Richards WG, Staunton J, Li C et al. Classification of human lung carcinomas by mRNA expression profiling reveals distinct adenocarcinoma subclasses. Proc Natl Acad Sci USA 2001, 98, 13790-13795.
[3] Takezawa C, Takahashi H, Fujishima T, Shiratori M et al., Assessment of differentiation in adenocarcinoma cells from pleural effusion by peripheral airway cell markers and their diagnostic values. Lung Cancer 2002, 38, 273-281.
[4] Fields WR, Desiderio JG, Putnam KP, Bombick DW, Doolittle DJ. Quantification of changes in c-myc mRNA levels in normal human bronchial epithelial (NHBE) and lung adenocarcinoma (A549) cells following chemical treatment. Toxicol Sci 2001, 63, 107-114.
[5] Broers JL, Viallet J, Jensen SM, Pass H et al. Expression of c-myc in progenitor cells of the bronchopulmonary epithelium and in a large number of non-small cell lung cancers. Am J Respir Cell Mol Biol 1993, 9, 33-43.
[6] Little CD, Nau MM, Carney DN, Gazdar AF, Minna JD. Amplification and expression of the c-myc oncogene in human lung cancer cell lines. Nature 1983, 306, 194-196.
[7] Zajac-Kaye M. Myc oncogene: a key component in cell cycle regulation and its implication for lung cancer. Lung Cancer 2001, 34, S43-S46.
[8] Bernasconi NL, Wormhoudt TA, Laird-Offringa IA. Post-transcriptional deregulation of myc genes in lung cancer cell lines. Am J Respir Cell Mol Biol 2000, 23, 560-565.
[9] Barr LF, Campbell SE, Bochner BS, Dang CV. Association of the decreased expression of alpha3beta1 integrin with the altered cell: environmental interactions and enhanced soft agar cloning ability of c-myc-overexpressing small cell lung cancer cells. Cancer Res 1998, 58, 5537-5545.
[10] Lorenz J, Friedberg T, Paulus R, Oesch F, Ferlinz R. Oncogene overexpression in non-small-cell lung cancer tissue: prevalence and clinicopathological significance. Clin Investig 1994, 72, 156-163.
[11] Volm M, Drings P, Wodrich W, van Kaick G. Expression of oncoproteins in primary human non-small cell lung cancer and incidence of metastases. Clin Exp Metastasis 1993, 11, 325-329.
[12] Pfeifer AM, Mark GE 3rd, Malan-Shibley L, Graziano S et al. Cooperation of c-raf-1 and c-myc protooncogenes in the neoplastic transformation of simian virus 40 large tumor antigen-immortalized human bronchial epithelial cells. Proc Natl Acad Sci U S A 1989, 86, 10075-10079.
[13] Ehrhardt A, Bartels T, Geick A, Klocke R et al. Development of pulmonary bronchiolo-alveolar adenocarcinomas in transgenic mice overexpressing murine c-myc and epidermal growth factor in alveolar type II pneumocytes. Br J Cancer 2001, 84, 813-818.
[14] Facchini LM, Penn LZ. The molecular role of Myc in growth and transformation: recent discoveries lead to new insights. FASEB J 1998, 12, 633-651.
[15] Amati B, Frank SR, Donjerkovic D, Taubert S. Function of the c-Myc oncoprotein in chromatin remodeling and transcription. Biochim Biophys Acta 2001, 1471, 135-145. *Review.*
[16] Lee LA, Dang CV. Myc target transcriptomes. Curr Top Microbiol Immunol 2006, 302, 145-167. *Review.*
[17] Prochownik EV. c-Myc as a therapeutic target in cancer. Expert Rev Anticancer Ther 2004, 4, 289-302. *Review.*
[18] Dang CV. c-Myc target genes involved in cell growth, apoptosis, and metabolism. Mol Cell Biol 1999, 19, 1-11. *Review.*
[19] Ehrhardt A, Bartels T, Klocke R, Paul D, Halter R. Increased susceptibility to the tobacco carcinogen 4-(methylnitrosamino)-1-(3-pyridyl)-1-butanone in transgenic mice overexpressing c-myc and epidermal growth factor in alveolar type II cells. J Cancer Res Clin Oncol 2003, 129, 71-75.
[20] Chatterji B, Borlak J. Serum proteomics of lung adenocarcinomas induced by targeted overexpression of c-raf in alveolar epithelium identifies candidate biomarkers. Proteomics 2007, 7, 3980-3991.
[21] Lehner I, Niehof M, Borlak J. An optimized method for the isolation and identification of membrane proteins. Electrophoresis 2003, 24, 1795-1808.
[22] Rabilloud T. Use of thiourea to increase the solubility of membrane proteins in two-dimensional electrophoresis. Electrophoresis 1998, 19, 758-760.
[23] Görg A, Postel W, Weser J, Günther S et al. Elimination of point streaking on silver stained two-dimensional gels by addition of iodoacetamide to the equilibration buffer. Electrophoresis 1987, 8, 122-124.
[24] Rutters H, Zürbig P, Halter R, Borlak J. Towards a lung adenocarcinoma proteome map: studies with SP-C/c-raf transgenic mice. Proteomics 2006, 10, 3127-3137.
[25] Perkins DN, Pappin DJ, Creasy DM, Cottrell JS. Probability-based protein identification by searching sequence databases using mass spectrometry data. Electrophoresis 1999, 20, 3551-3567.
[26] Chamrad DC, Koerting G, Gobom J, Thiele H et al. Interpretation of mass spectrometry data for high-throughput proteomics. Anal Bioanal Chem 2003, 376, 1014-1022.
[27] Hood BL, Zhou M, Chan KC, Lucas DA et al. Investigation of the mouse serum proteome. J Proteome Res 2005, 4, 1561-1568.
[28] Anderson NL, Anderson NG. The human plasma proteome: history, character, and diagnostic prospects. Mol Cell Proteomics 2002, 1, 845-867. *Review.* Erratum in: Mol Cell Proteomics 2003, 2, 50.
[29] Zeller KI, Jegga AG, Aronow BJ, O'Donnell KA, Dang CV. An integrated database of genes responsive to the Myc oncogenic transcription factor: identification of direct genomic targets. Genome Biol 2003, 4, R69.
[30] Ceciliani F, Giordano A, Spagnolo V. The systemic reaction during inflammation: the acute-phase proteins. Protein Pept Lett 2002, 9, 211-223. *Review.*
[31] Kanoh Y, Ohtani N, Mashiko T, Ohtani S et al. Levels of alpha 2 macroglobulin can predict bone metastases in prostate cancer. Anticancer Res 2001, 21, 551-556.
[32] Misra UK, Pizzo SV. Regulation of cytosolic phospholipase A2 activity in macrophages stimulated with receptor-recognized forms of alpha 2-macroglobulin: role in mitogenesis and cell proliferation. J Biol Chem 2002, 277, 4069-4078.
[33] Pepys MB, Herbert J, Hutchinson WL, Tennent GA et al. Targeted pharmacological depletion of serum amyloid P component for treatment of human amyloidosis. Nature 2002, 417, 254-259.
[34] Korbelik M, Cecic I, Merchant S, Sun J. Acute phase response induction by cancer treatment with photodynamic therapy. Int J Cancer 2007, Nov 21
[35] Van Bennekum AM, Wei S, Gamble MV et al. Biochemical basis for depressed serum retinol levels in transthyretin-deficient mice. J Biol Chem 2001, 276, 1107-1113.
[36] Roberts D, Williams SJ, Cvetkovic D et al. Decreased expression of retinol-binding proteins is associated with malignant transformation of the ovarian surface epithelium. DNA Cell Biol 2002, 21, 11-19.
[37] Zhang Z, Bast RC Jr, Yu Y, Li J et al. Three biomarkers identified from serum proteomic analysis for the detection of early stage ovarian cancer. Cancer Res 2004, 64, 5882-5890.
[38] Moore LE, Fung ET, McGuire M, Rabkin CC et al. Evaluation of apolipoprotein A1 and posttranslationally modified forms of transthyretin as biomarkers for ovarian cancer detection in an independent study population. Cancer Epidemiol Biomarkers Prev 2006, 15, 1641-1646.
[39] Maciel CM, Junqueira M, Paschoal ME, Kawamura MT et al. Differential proteomic serum pattern of low molecular weight proteins expressed by adenocarcinoma lung cancer patients. J Exp Ther Oncol 2005, 5, 31-38.
[40] Feng JT, Liu YK, Song HY, Dai Z et al. Heat-shock protein 27: a potential biomarker for hepatocellular carcinoma identified by serum proteome analysis. Proteomics 2005, 5, 4581-4588.
[41] Cham BE. Importance of apolipoproteins in lipid metabolism. Chem Biol Interact 1978, 20, 263-277. *Review.*
[42] Chen J, Anderson M, Misek DE, Simeone DM, Lubman DM. Characterization of apolipoprotein and apolipoprotein precursors in pancreatic cancer serum samples via two-dimensional liquid chromatography and mass spectrometry. J Chromatogr A 2007, 1162, 117-25.
[43] Venanzoni MC, Giunta S, Muraro GB, Storari L et al. Apolipoprotein E expression in localized prostate cancers. Int J Oncol 2003, 22, 779-786.
[44] Wright LC, Sullivan DR, Muller M, Dyne M et al. Elevated apolipoprotein(a) levels in cancer patients. Int J Cancer 1989, 43, 241-244.
[45] Han C, Zhang HT, Du L, Liu X et al. Serum levels of leptin, insulin, and lipids in relation to breast cancer in china. Endocrine 2005, 26, 19-24.
[46] Chang SI, EI-Bayoumy K, Sinha I, Trushin N et al. 4-(Methylnitrosamino)-I-(3-pyridyl)-1-butanone enhances the expression of apolipoprotein A-I and Clara cell 17-kDa protein in the lung proteomes of rats fed a corn oil diet but not a fish oil diet. Cancer Epidemiol Biomarkers Prev 2007, 16, 228-235.
[47] Ishikawa I, Hayama T, Yoshida S, Asaka M et al. Proteomic analysis of rat plasma by SELDI-TOF-MS under the condition of prevention of progressive adriamycin nephropathy using oral adsorbent AST-120. Nephron Physiol 2006, 103, 125-130.
[48] Hiukka A, Fruchart-Najib J, Leinonen E, Hilden H et al. Alterations of lipids and apolipoprotein CIII in very low density lipoprotein subspecies in type 2 diabetes. Diabetologia 2005, 48, 1207-1215.
[49] Yokoyama Y, Kuramitsu Y, Takashima M, lizuka N, et al. Protein level of apolipoprotein E increased in human hepatocellular carcinoma. Int J Oncol 2006, 28, 625-631.
[50] Li ZG, Zhao L, Liu L, Ding YQ. Monitoring changes of serum protein markers in metastatic colorectal carcinoma model. Zhonghua Bing Li Xue Za Zhi 2007, 36, 48-52.
[51] Grønborg M, Kristiansen TZ, Iwahori A, Chang R et al. Biomarker discovery from pancreatic cancer secretome using a differential proteomic approach. Mol Cell Proteomics 2006, 5, 157-171.
[52] Grainger DJ, Reckless J, McKilligin E. Apolipoprotein E modulates clearance of apoptotic bodies in vitro and in vivo, resulting in a systemic proinflammatory state in apolipoprotein E-deficient mice. J Immunol 2004, 173, 6366-6375.
[53] Beecken WD, Engl T, Ringel EM, Camphausen K et al. An endogenous inhibitor of angiogenesis derived from a transitional cell carcinoma: clipped beta2-glycoprotein-I. Ann Surg Oncol 2006, 13, 1241-1251.
[54] Hough CD, Cho KR, Zonderman AB, Schwartz DR, Morin PJ. Coordinately up-regulated genes in ovarian cancer. Cancer Res 2001, 61, 3869-3876.
[55] Iwata K, Nishinaka T, Matsuno K, Yabe-Nishimura C. Increased gene expression of glutathione peroxidase-3 in diabetic mouse heart. Biol Pharm Bull 2006, 29, 1042-1045.
[56] Smida J, Holubek V, Oravec C, Thurzo V. The properdin system in tumorous disease. Part VI. Influence of the properdin fraction on the cancerogenic activity of chicken tumour virus B77. Neoplasma 1960, 7, 26-30.
[57] Beebe DP, Schreiber RD, Cooper NR. Neutralization of influenza virus by normal human sera: mechanisms involving antibody and complement. J Immunol 1983, 130, 1317-1322.

## Claims

1. Use of at least one biomarker selected from the group consisting of APOE, APOC3, A1AT6, A2MG, PROP, TTHY, A1AG8, APOA1, APOH, GPX3, MUP8, RETBP, SAMP, VTDB, S6A11, EGFR in the diagnosis, prognosis and/or treatment monitoring of cancer, in particular of lung cancer.

2. Use as claimed in claim 1 for monitoring the therapeutic treatment of a patient suffering from lung cancer, in particular the treatment with irinotecan, paclitaxel and/or 5-fluorouracil.

3. Use as claimed in one of the claims 1-2, wherein the at least one biomarker is selected from the group consisting of APOE, APOC3, A1AG8, APOA1, APOH, GPX3, RETBP, SAMP.

4. Use as claimed in claim 3 for the diagnosis, prognosis and/or treatment monitoring of BAC.

5. Use as claimed in one of the claims 1-2, wherein the at least one biomarker is selected from the group consisting of MUP8, VTDB, S6A11, EGFR.

6. Use as claimed in claim 5 for the diagnosis, prognosis and/or treatment monitoring of AAH.

7. Use as claimed in one of the claims 1-6, wherein at least one biomarker selected from the group consisting of APOE, APOC3, A1AG8, APOA1, APOH, GPX3, RETBP, SAMP and at least one biomarker selected from the group consisiting of MUP8, VTDB, S6A11, EGFR are used.

8. A method for diagnosing, prognosing and/or staging cancer and/or monitoring the treatment of cancer, in particular lung cancer, comprising
(a) measuring the level of at least one biomarker selected from the group consisting of APOE, APOC3, A1AT6, A2MG, PROP, TTHY, A1AG8, APOA1, APOH, GPX3, MUP8 RETBP, SAMP, VTDB, S6A11, EGFR in a body fluid sample, in particular in a serum sample, of a patient suffering from or being susceptible to cancer, and
(b) comparing the level of said at least one biomarker in said sample to a reference level of said at least one biomarker, in particular by the use according to one of the claims 1-8.

9. Method as claimed in claim 8 for monitoring the therapeutic treatment of a patient suffering from lung cancer, in particular the treatment with irinotecan, paclitaxel and/or 5-fluorouracil.

10. Method as claimed in one of the claims 8-9, wherein the at least one biomarker is selected from the group consisting of APOE, APOC3, A1AG8, APOA1, APOH, GPX3, RETBP, SAMP.

11. Method as claimed in claim 10 for the diagnosis, prognosis, staging and/or treatment monitoring of BAC.

12. Method as claimed in one of the claims 8-9, wherein the at least one biomarker is selected from the group consisting of MUP8, VTDB, S6A11, EGFR.

13. Method as claimed in claim 11 for the diagnosis, prognosis, staging and/or treatment monitoring of AAH.

14. Method as claimed in one of the claims 8-13 to distinguish between different subtypes of lung cancer, such as (but not limited to) lung adenocarcinomas as defined by AAH or BAC, wherein at least one biomarker selected from the group consiting of APOE, APOC3, A1AG8, APOA1, APOH, GPX3, RETBP, SAMP and at least one biomarker selected from the group consisiting of MUP8, VTDB, S6A11, EGFR are measured.

15. Method as claimed in claim 14, wherein a significantly altered level of APOE, APOC3, A1AG8, APOA1, APOH, GPX3, RETBP, SAMP in comparison with the level of a normal individual is indicative of BAC and wherein a significantly altered level of MUP8, VTDB, S6A11, EGFR in comparison with the level of a normal individual is indicative of AAH.

16. A biomarker selected from a first group consisting of APOC3, A1AT6, A2MG, PROP, TTHY, A1AG8, APOA1, APOH, GPX3, MUP8 RETBP, SAMP, VTDB, S6A11, EGFR or from a second group consisting of APOE and sequence fragments thereof, in particular sequence fragments of APOE being 6-24 amino acid residues in length,
wherein the biomarker is regulated by c-myc overexpression in a subject.

17. Biomarker as claimed in claim 16 selected from a first group consisting of APOC3, A1AG8, APOA1, APOH, GPX3, MUP8, RETBP, SAMP or from a second group consisting of APOEand sequence fragments thereof, in particular sequence fragments of APOE being 6-15 amino acid residues in length.

18. A biomarker for qualifying the c-myc activity in a patient suffering or being susceptible to cancer or for classifying a patient suffering from or being susceptible to lung cancer selected from a first group consisting of sequence fragments of the group of biomarkers according to one of the claims 16-17 or from a second group consisiting of sequence fragments of APOE, wherein the sequence fragments are 6-24 amino acid residues in length and are preferably synthetic peptides.

19. Biomarker as claimed in claim 18 selected from a first group consisting of
LAQIHFPR,
TLMSPLGITR,
RLAQIHFPR,
MQHLEQTLSK,
ELISKFLLNR,
IFNNGADLSGITEENAPLK,
NHYQAEVFSVNFAESEEAK,
DQSPASHEIATNLGDFAISLYR,
KPFDPENTEEAEFHVDESTTVK,
FDHPFLFIIFEEHTQSPLFVGK,
APFALQVNTLPLNFDK,
TCDHPAPR,
QRLCTPLLPK,
HGGPFCAGDATR,
MSINCEGTPGQQSR,
LRMSINCEGTPGQQSR,
HGGPFCAGDATRNQMCNK,
CGGHCPGEAQQSQACDTQK,
SCSAPAPSHQPPGKPCSGPAYEHK,
FVEGVYR,
TSEGSWEPFASGK,
TAESGELHGLTTDEK,
TLGISPFHEFADVVFTANDSGHR,
HYTIAALLSPYSYSTTAVVSNPQN,
YEGGVETFAHLIVLR,
LQELQGR,
EDVELYR,
ARPALEDLR,
LSPVAEEFR,
QKLQELQGR,
WKEDVELYR,
VQPYLDEFQK,
TQLAPHSEQMR,
LSPVAEEFRDR,
SNPTLNEYHTR,
VAPLGAELQESAR,
QEMNKDLEEVK,
VKDFANVYVDAVK,
LQELQGRLSPVAEEFR,
TVQDALSSVQESDIAVVAR,
IHFYCK,
ATVLYQGMR,
ITCPPPPVPK,
WSPDIPACAR,
DGTIEIPSCFK,
CSYTVEAHCR,
TGTWSFLPTCR,
VCPFAGILENGIVR,
IQEQFKNGMMHGDK,
FTCPLTGMWPINTLR,
ICPKPDDLPFATVVPLK,
TSYDPGEQIVYSCKPGYVSR,
CPFPPRPENGYVNYPAKPVLLYK,
YVRPGGGFVPNFQLFEK,
IEDNGNFR,
EKIEDNGNFR,
ENIIDLSNANR,
FAQLCEEHGILR,
DGETFQLMGLYGR,
INGEWHTIILASDKR,
TDYDNFLMAHLINEK,
LFLEQIHVLENSLVLK,
AGEYSVTYDGFNTFTIPK,
DPNGLSPETR,
YWGVASFLQR,
QRQEELCLER,
LQNLDGTCADSYSFVFSR,
KDPEGLFLQDNIIAEFSVDEK,
APPSIVLGQEQDNYGGGFQR,
or from a second group consisting of
EVQAAQAR,
FWDYLR,
DRLEEVR,
EHMEEVR,
LGPLVEQGR,
LEEVGNQAR,
QWANLMEK,
DRAQAFGDR,
LQAEIFQAR,
MEEQTQQIR,
GRLEEVGNQAR,
TANLGAGAAQPLR,
SKMEEQTQQIR,
GWFEPIVEDMHR,
ELEEQLGPVAEETR,
NEVHTMLGQSTEEIR.

20. Biomarker as claimed in claim 19 selected from a first group consisting of
YEGGVETFAHLIVLR,
LQELQGR,
EDVELYR,
ARPALEDLR,
LSPVAEEFR,
QKLQELQGR,
WKEDVELYR,
VQPYLDEFQK,
TQLAPHSEQMR,
LSPVAEEFRDR,
SNPTLNEYHTR,
VAPLGAELQESAR,
QEMNKDLEEVK,
VKDFANVYVDAVK,
LQELQGRLSPVAEEFR,
TVQDALSSVQESDIAVVAR,
IHFYCK,
ATVLYQGMR,
ITCPPPPVPK,
WSPDIPACAR,
DGTIEIPSCFK,
CSYTVEAHCR,
TGTWSFLPTCR,
VCPFAGILENGIVR,
IQEQFKNGMMHGDK,
FTCPLTGMWPINTLR,
ICPKPDDLPFATVVPLK,
TSYDPGEQIVYSCKPGYVSR,
CPFPPRPENGYVNYPAKPVLLYK,
YVRPGGGFVPNFQLFEK,
IEDNGNFR,
EKIEDNGNFR,
ENIIDLSNANR,
FAQLCEEHGILR,
DGETFQLMGLYGR,
INGEWHTIILASDKR,
TDYDNFLMAHLINEK,
LFLEQIHVLENSLVLK,
AGEYSVTYDGFNTFTIPK,
DPNGLSPETR,
YWGVASFLQR,
QRQEELCLER,
LQNLDGTCADSYSFVFSR,
KDPEGLFLQDNIIAEFSVDEK,
APPSIVLGQEQDNYGGGFQR,
or from a second group consisting of
EVQAAQAR,
FWDYLR,
DRLEEVR,
EHMEEVR,
LGPLVEQGR,
LEEVGNQAR,
QWANLMEK,
DRAQAFGDR,
LQAEIFQAR,
MEEQTQQIR,
GRLEEVGNQAR,
TANLGAGAAQPLR,
SKMEEQTQQIR,
GWFEPIVEDMHR,
ELEEQLGPVAEETR,
NEVHTMLGQSTEEIR.

21. A composition for qualifying the c-myc activity in a patient suffering or being susceptible to cancer or for classifying a patient suffering from or being susceptible to lung cancer, in particular by an *in vitro* body fluid analysis, comprising an effective amount of at least one biomarker selected from the first group according to one of the claims 16-20 or an effective amount of at least one biomarker selected from the second group according to one of the claims 16-20

22. Composition as claimed in claim 21 comprising an effective amount of at least one biomarker selected from the first group according to one of the claims 16-20 and an effective amount of at least one biomarker selected from the second group according to one of the claims 16-20.

23. Use of a composition as claimed in one of the claims 21-22 for the production of a diagnostic agent, in particular of a diagnostic standard for body fluid analysis.

24. Use as claimed in claim 23 for the production of a diagnostic agent for qualifying the c-myc activity in a patient suffering or being susceptible to cancer or for classifying a patient suffering from or being susceptible to lung cancer.

25. Use as claimed in one of the claims 23-24 for the production of a diagnostic agent for predicting or monitoring the response of a cancer patient to a method of treating cancer comprising administering a c-myc activity modulator, e.g. an inhibitor of c-myc/max dimerization.

26. A kit for qualifying the the c-myc activity in a patient suffering or being susceptible to cancer or for classifying a patient suffering from or being susceptible to lung cancer, in particular for predicting or monitoring the response of a cancer patient to a method of treating cancer comprising administering an c-myc activity modulator, comprising at least one standard (1) indicative of the body fluid level of a biomarker selected from the first group according to one of the claims 16-20 in normal individuals or individuals having cancer associated with increased c-myc activity and/or at least one standard (2) indicative of the body fluid level of a biomarker selected from the second group according to one of the claims 16-20 in normal individuals or individuals having cancer associated with increased c-myc activity, and instructions for the use of the kit.

27. The kit as claimed in claim 26, wherein the at least one standard (1) comprises an indicative amount of at least one biomarker selected from the first group according to one of the claims 16-20 and/or wherein the at least one standard (2) comprises an indicative amount of at least one biomarker selected from the second group according to one of the claims 16-20.

28. The kit as claimed in one of the claims 26-27, comprising a mixture of the at least one standard (1) and the at least one standard (2), in particular the composition according to claim 22.

29. The kit as claimed in one of the claims 26-28, further comprising a lysis buffer according to one of the claims 41-43 and/or a digesting buffer according to one of the claims 41-43.

30. The kit as claimed in one of the claims 26-29, further comprising at least one antibody specific for a biomarker selected from the first group according to one of the claims 16-21 and/or at least one antibody specific for a biomarker selected from the second group according to one of the claims 16-21, and reagents effective to detect said biomarker(s) in a serum sample.

31. The kit as claimed in claim 30, wherein the at least one antibody is polyclonal.

32. The kit as claimed in one of the claims 30-31 comprising at least one labelled secondary antibody specific for the at least one antibody of claim 30-31.

33. A method of qualifying the c-myc activity in a patient suffering or being susceptible to cancer or for classifying a patient suffering from or being susceptible to lung cancer, comprising determining in a body fluid sample of a subject suffering from or being susceptible to cancer at least one biomarker selected from the first group according to one of the claims 16-20 and/or at least one biomarker selected from the second group according to one of the claims 16-20, wherein the body fluid level of the at least one biomarker of said first group being significantly higher and/or the body fluid level of the at least one biomarker of said second group being significantly lower than the level of said biomarker(s) in the body fluid of subjects without cancer associated with increased activity of c-myc is indicative of induced c-myc kinase activity in the subject.

34. Method as claimed in claim 33 for classifying a patient suffering from or being susceptible to lung cancer, comprising determining in a body fluid sample of the patient at least one biomarker selected from the first group according to one of the claims 17 or 20 and/or at least one biomarker selected from the second group according to one of the claims 17 or 20, wherein the body fluid level of the at least one biomarker of said first group being significantly higher and/or the body fluid level of the at least one biomarker of said second group being significantly lower than the level of said biomarker(s) in the body fluid of subjects without bronchiolo-alveolar carcinoma (BAC) associated with increased activity of c-myc is indicative of BAC in the subject.

35. Method as claimed in one of the claims 33-34 for predicting the response of a cancer patient to a method of treating cancer comprising administering a c-myc activity modulator,
wherein the body fluid level of the at least one biomarker of said first group being significantly higher and/or the body fluid level of the at least one biomarker of said second group being significantly lower than the level of said biomarker(s) in the body fluid of subjects without cancer associated with increased activity of c-myc is indicative that the subject will respond therapeutically to a method of treating cancer comprising administering a c-myc activity modulator.

36. Method as claimed in one of the claims 33-35 for monitoring the therapeutically response of a cancer patient to a method of treating cancer comprising administering an c-myc activity modulator, wherein the body fluid level of the at least one biomarker of said first group before and after the treatment and/or the body fluid level of the at least one biomarker of said second group before and after the treatment is determined, and a significant decrease of said body fluid level(s) of the at least one biomarker of said first group and/or a significant increase of said body fluid level(s) of the at least one biomarker of said second group after the treatment is indicative that the subject therapeutically responds to the administration of the c-myc activity modulator.

37. The method as claimed in one of the claims 33-36, wherein an immunoassay is performed, in particular by using the kit as claimed in one of the claims 26-32.

38. The method as claimed in claim 37, wherein at least one antibody specific for a biomarker selected from the first group according to one of the claims 16-20, preferably to one of the claims 17 or 20, and/or at least one antibody specific for a biomarker selected from the second group according to one of the claims 16-20, preferably to one of the claims 17 or 20, and reagents effective to detect said biomarker(s) in a serum sample is used for the immunoassay.

39. The method as claimed in one of the claims 33-38, wherein a peptide mass fingerprinting is performed, in particular by using the kit as claimed in one of the claims 26-29.

40. The method as claimed in claim 39, comprising the steps of
- isolating a serum sample from a blood sample of a subject suffering from or being susceptible to cancer;
- adding lysis buffer to the serum sample;
- separating the proteins of the lysed serum sample by 2-D gel electrophoresis;
- excising from the gel at least one sample containing a protein of interest;
- adding digesting buffer to the at least one excised sample;
- determining the amount of the at least one protein of interest by analyzing the at least one digest mixture by mass spectrometry.

41. The method as claimed in claim 40, wherein
- the subject is a human patient or non-human transgenic animal; and/or
- the serum sample is isolated by centrifuging the blood sample; and/or
- the 2-DE is performed by using two different pH gradients; and/or
- the lysis buffer comprises (a) at least one buffer component, (b) at least one chaotrope, (c) at least one detergens, (d) at least one reducing agent (e) at least one carrier ampholyte, (f) at least one ribonuclease; and/or
- the protein of interest is a biomarker selected from the first group according to one of the claims 16-18 or a biomarker selected from the second group according to one of the claims 16-18; and/or
- the digesting buffer comprises a bicarbonate compound and a protease; and/or
- wherein the mass spectrometry is selected from the group consisting of MALDI-TOF and ESI-TOF.

42. The method as claimed in one of the claims 40-41, wherein
- the subject is suffering from or is susceptible to cancer of the lung; and/or
- the 2-DE is performed by using the pH gradients 3-10 and 4-7; and/or
- the lysis buffer is an aqueous solution of (a) at least one buffer compound selected from the group consisting of Tris and HEPES, (b) at least one chaotrope selected from the group consisting of urea and thiourea, (c) at least one detergens selected from the group consisting of CHAPS and SDS, (d) at least one reducing agent selected from the group consisting of DTT and TCEP, (e) at least one carrier ampholyte selected from the group consisting of biolyte 5-7 and biolyte 3-10, (f) at least one ribonuclease selected from the group consisting of endonuclease and exonuclease; and/or
- the protein of interest is a biomarker selected from the first group according to one of the claims 17-18 or a biomarker selected from the second group according to one of the claims 17-18; and/or
- the digesting buffer is an aqueous solution of at least one bicarbonate compound selected from the group consisting of ammonium bicarbonate and sodium bicarbonate and of at least one serine protease, in particular selected from the group consisting of trypsin, chymotrypsin and elastase; and/or
- the mass spectrometry is performed by MALDI-TOF; and/or
- a tandem mass spectrometer is used; and/or
- a matrix is used for the mass spectrometry selected from the group consisting of 3,5-dimethoxy-4-hydroxycinnamic acid, α-cyano-4-hydroxycinnamic acid and 2,5-dihydroxybenzoic acid.

43. The method as claimed in one of the claims 40-42, wherein
- the subject is a transgenic mouse, in particular a mouse whose genome comprises a non natural c-myc sequence; and/or
- the lysis buffer is an aqueous solution of (a) Tris; (b) urea and thiourea, (c) CHAPS,
(d) DTT, (e) biolyte 3-10, (f) endonuclease; and/or
- the serum sample is calibrated or the serum samples are equilibrated to a predefined protein concentration by adding the lysis buffer; and/or
- the protein of interest is a biomarker selected from the first group according to claim 18 or a biomarker selected from the second group according to claim 18; and/or
- the digesting buffer is an aqueous solution of ammonium bicarbonate and trypsin; and/or
- a MALDI-TOF/TOF spectrometry is performed; and/or
- a matrix is used for the mass spectrometry selected from the group consisting of α-cyano-4-hydroxycinnamic acid.

44. The method as claimed in one of the claims 40-43, further comprising the steps of
- determining the protein concentration of the serum sample, in particular by the Bradford method; and/or
- freezing and thawing the serum sample before the lysis buffer is added; and/or
- staining the gel after the 2-DE, in particular by using coomassie blue; and/or
- destaining the excised sample; and/or
- shrinking, in particular by adding acetonitrile, and drying of the excised sample before the digesting buffer is added; and/or
- using a peptide calibration standard for the mass spectrometry.

45. A procedure to screen for and to identify drugs against cancer associated with an increased c-myc activity comprising determining in a body fluid sample of a transgenic cancer mouse being treated with a compound to be tested, in particular of a mouse whose genome comprises a non natural c-myc sequence, at least one biomarker selected from the first group according to one of the claims 16-20 and/or at least one biomarker selected from the second group according to one of the claims 16-20, wherein the body fluid level of the at least one biomarker of said first group being significantly lower and/or the body fluid level of the at least one biomarker of said second group being significantly higher than the level of said biomarker(s) in the body fluid of an untreated transgenic cancer mouse is indicative of the therapeutic effect of said compound as a c-myc activity modulator.

46. The procedure as claimed in claim 45, wherein the method as claimed in claim 36, in particular according to one of the claims 37-44, is used.

47. A procedure for identifying diagnostic cancer biomarkers comprising the steps of
- isolating serum samples from blood samples of a plurality of c-myc cancer mice bearing the same type of tumor;
- adding lysis buffer to said serum samples;
- separating the proteins of said lysed serum samples by 2-DE gel electrophoreses;
- excising from the gels each one sample containing a protein of interest,
- adding digesting buffer to the excised samples;
- analyzing the digest mixtures by mass spectrometry, in particular by peptide mass fingerprinting, and determining the protein of interest as biomarker, wherein a serum level of the protein being significantly higher and/or a serum level of the protein being significantly lower than the level of said protein in the serum of normal subjects is indicating the biomarker.

48. The procedure as claimed in claim 47, wherein the steps are implemented according to one of the claims 37-43.

49. The procedure as claimed in one of the claims 47-48, further comprising the steps according to claim 44.
